# EUROPEAN PATENT SPECIFICATION

(11) **EP 1 863 902 B1**
(45) Date of publication and mention of the grant of the patent: **18.05.2011**
(21) Application number: 06706121.8
(22) Date of filing: 15.03.2006
(51) Int. Cl.: C12N 1/21, A23L 3/3571

(54) **LACTOSE-POSITIVE RECOMBINANT LEUCONOSTOC STRA**
LACTOSEPOSITIVER REKOMBINANTER LEUCONOSTOC-STAMM
SOUCHE DE LEUCONOSTOC DE RECOMBINAISON LACTOSE POSITIVE

(30) Priority: 15.03.2005 DK 200500372; 15.03.2005 US 662111 P
(43) Date of publication of application: 12.12.2007
(73) Proprietor: The Technical University of Denmark, 2800 Lyngby (DK)
(72) Inventor: JENSEN, Peter, Ruhdal, DK-2820 Gentofte (DK); HELMARK, Søren, DK-2800 Kgs. Lyngby (DK)
(74) Representative: Benthin, Stig
(86) International application number: PCT/DK2006/000150
(87) International publication number: WO 2006/097107

(56) References cited:
- YANG R ET AL: "Enhanced bacteriocin production by lactic acid bacteria in a dairy-based medium, supplemented with beta-galactosidase;" INDIAN J.MICROBIOL.; 39, 4, 235-40 CODEN: IJMBAC ISSN: 0046-8891, 1999, XP009058629
- RUBIO-TEXEIRA MARTA ET AL: "Highly efficient assimilation of lactose by metabolically engineered strain of Saccharomyces cerevisiae" YEAST, vol. 14, no. 9, 30 June 1998 (1998-06-30), pages 827-837, XP002358177 ISSN: 0749-503X
- DATABASE UniProt [Online] 1 October 2002 (2002-10-01), "Lactose permease." XP002358190 retrieved from EBI accession no. UNIPROT:Q8L2I0 Database accession no. Q8L2I0
- DATABASE UniProt [Online] 1 October 2002 (2002-10-01), "Beta-galactosidase." XP002358191 retrieved from EBI accession no. UNIPROT:Q8L2H9 Database accession no. Q8L2H9
- DATABASE UniProt 1 July 1993 (1993-07-01), "Beta-galactosidase large subunit (LacL)" XP002389930 Database accession no. Q02603
- DATABASE UniProt 1 July 1993 (1993-07-01), "Beta-galactosidase small subunit (LacM)" XP002389931 Database accession no. Q02604
- DATABASE UniProt 15 December 1998 (1998-12-15), "Lactose permease (LacS)" XP002389932 Database accession no. Q48624
- DATABASE WPI Section Ch, Week 199918 Derwent Publications Ltd., London, GB; Class D13, AN 1999-206535 XP002361142 & DK 9 700 835 A (SLAGTERIERNES FORSKNINGSINSTITUT) 11 January 1999 (1999-01-11)
- HELMARK SOREN ET AL: "Transformation of Leuconostoc carnosum 4010 and evidence for natural competence of the organism" APPLIED AND ENVIRONMENTAL MICROBIOLOGY, vol. 70, no. 6, June 2004 (2004-06), pages 3695-3699, XP002358178 ISSN: 0099-2240

## Description

### Background of the invention

Improved methods and tools for protecting food products against spoilage by bacteria, moulds and other undesirable organisms and that can minimize or replace the use of chemical preservatives are of great importance to the food industry. Among the most serious causes of food spoilage is *Listeria,* a gram positive facultative anaerobe that is widespread in the environment. In particular the species *Listeria monocytogenes* is now recognized to be one of the most important food-borne pathogens and is often isolated from raw meat, vegetables, raw milk, and milk products, particularly cheese which may also be contaminated during processing. *L. monocytogenes* is known to be a human pathogen causing the potentially fatal disease listeriosis, while other species such as *L. inanovii* may also be human pathogens. *L*. *monocytogenes* is acid-, salt- and psychro-tolerant, and thus the proliferation of *L*. *monocytogenes* in food cannot be prevented by refrigerated storage or the addition of acid or salt.

Lactic acid bacteria are widely used in the food industry due to their ability to preserve food and add desirable flavour and texture to products. *Leuconostoc* species are lactic acid bacteria which have particular commercial importance in the dairy and meat industries. One attribute of some *Leuconostoc* species of value to industry is the ability to produce bacteriocins, which are peptides that exhibit antagonistic activities against a range of closely related bacteria. The growth of pathogenic micro-organisms in food can be prevented by the addition of bacteria that produce bacteriocins with antagonistic activity against the pathogenic micro-organisms.

The bacteriocin producing bacterium *Leuconostoc carnosum* is used in industry for bio-preservation of meat products because of its ability to prevent the growth of the food borne pathogen *L. monocytogenes,* while not imparting any undesirable flavour or odour to the product. When *Leu. carnosum* is added to sliced meat products (WO02/056694), it effectively prevents the growth of *L. monocytogenes* as its bacteriocins are active against all tested serotypes of *L. monocytogenes* (Budde, B.B., et a/. (2003) International Journal of Food Microbiology. 83: 171- 184; Keppler, K., et al., (1994) Food Microbiology. 11: 39-45; van Laack, R.L.J.M., et a/., (1992) International Journal of Food Microbiology. 16: 183-195).

*L. monocytogenes* also thrives in dairy products, in particular in soft cheeses. Contamination by *L. monocytogenes* may originate from the raw milk, particularly from un-pasteurised milk products, but may also be introduced post-pasteurisation, during the many handling steps involved in milk processing, e.g. cheese making. *L. monocytogenes* is able to adhere to many different types of surfaces by forming a viable biofilm, and once a diary plant is contaminated, removal of such biofilms has proven to be highly resistant to standard sanitation methods. There is thus a recognised need in the dairy industry to find safe and effective means to control undesirable and/or pathogenic micro-organisms in milk products, and an attempt to screen for suitable bacteriocin-producing lactic acid bacteria has been reported (Buyong et al., 1998 Applied and environmental Microbiology 64: 4842-4845). In the absence of finding bacteriocin-producing lactic acid bacteria capable of growth on milk, there have been attempts to employ a bacteriocin-sorbic acid containing composition, or a sorbic acid-bacteriosin-producing micro-organism composition as a food additive (US 6,780,447).
This approach has the drawback that bacteriocins have a short half-life, being susceptible to proteolytic degradation. Thus there is a particular need to develop a commercial starter culture for the diary industry, which can ensure the quality and safety of milk products including yoghurt and cheese products.

### Summary of the invention

The present invention addresses the need for an anti*-Listerial* micro-organism that can be used in the diary industry to secure the safety and shelf-life of milk products. *Leu. carnosum,* that is currently used for the preservation of meat products, cannot be employed to prevent the growth of *L. monocytogenes* in milk products due to its inability to grow in milk. This inability to grow on milk is due to the fact that *Leu. carnosum* can not ferment lactose, which is the main carbon- and energy-source in milk. However, a lactose-positive strain of *Leu. carnosum,* that is able to grow in milk and milk products, would provide a novel and valuable means for preventing *L. monocytogenes* contamination and growth in dairy products.

Thus, the present invention provides an isolated recombinant *Leuconostoc carnosum* strain capable of growth on milk, or a milk product, that is useful for the natural preservation of dairy products. The isolated recombinant *Leu*. *carnosum* strain of the invention is characterised by its ability to grow on lactose as sole carbon source. The recombinant *Leuconostoc carnosum* strain is further characterised by its ability to produce a bacteriosin, which is toxic to pathogenic micro-organisms found in dairy products, in particular *Listeria monocytogenes.*

In one embodiment the recombinant *Leuconostoc carnosum* strain of the invention comprises one or more genes encoding a lactose transporter and a β-galactosidase or β-glucosidase. Accordingly, the invention encompasses a *Leuconostoc carnosum* strain that is a mutant, produced by mutagenesis of one or more native gene of the *Leuconostoc carnosum* strain.

In an alternative embodiment, the genes in the recombinant *Leuconostoc carnosum* strain, that encode a lactose transporter and β-galactosidase or β-glucosidase, are heterologous genes that are stably inherited in the *Leuconostoc camosum* strain.

The invention discloses a recombinant *Leuconostoc carnosum* strain comprising a gene encoding a β-galactosidase polypeptide having an amino acid sequence at least 60% homologous to SEQ ID NO: 3. Preferably said β-galactosidase is encoded by a nucleic acid molecule that hybridises to a nucleic acid molecule with a nucleotide sequence consisting of SEQ ID NO: 1 or 4, under stringency conditions of no less than 1xSSC at 65°C. The invention further provides a recombinant *Leuconostoc carnosum* strain comprising a gene encoding a lactose transporter polypeptide having the amino acid sequence at least 60% homologous of SEQ ID NO: 2, or a fragment thereof, having functional lactose transporter activity. Preferably said lactose transporter is encoded by a nucleic acid molecule that hybridises to a nucleic acid molecule with a nucleotide sequence consisting of SEQ ID NO: 1 or 6, under stringency conditions of no less than 1xSSC at 65°C. In an alternative embodiment, the recombinant *Leuconostoc carnosum* strain of the invention comprises a nucleic acid molecule having the nucleotide sequence of SEQ ID NO: 1, that comprises a coding sequence for a lactose transporter and a β-galactosidase.

In one embodiment, the recombinant *Leuconostoc carnosum* strain comprises a nucleic acid molecule encoding a lactose transporter and a β-galactosidase that is harboured on a self-replicating plasmid, and in an alternative embodiment the nucleic acid molecule is integrated into the genome (including a bacterial chromosome and/or a native plasmid) of said strain. The nucleic acid molecule, encoding a lactose transporter and a β-galactosidase, may be comprised within an operon, and furthermore be operably linked to a promoter.

The present invention further discloses a chemically defined growth-medium, (Table 3, supplemented with a carbon source e.g. lactose) for the growth and selection of *Leuconostoc carnosum,* including the selection of a lactose-positive *Leuconostoc carnosum* strain.

The invention further provides a starter culture for use in the production of milk products, comprising a recombinant *Leuconostoc carnosum* strain of the invention that is characterised by its ability to grow on lactose as sole carbon source; and encompasses the use of said starter culture or recombinant *Leuconostoc carnosum* strain in the production of a milk product.

In a further embodiment, the invention encompasses a milk product comprising a recombinant *Leuconostoc carnosum* strain of the invention, characterised by its ability to grow on lactose as sole carbon source, wherein the milk product is selected from cheese, butter milk, sour cream, yoghurt and kefir.

Furthermore a method is provided for constructing a recombinant *Leuconostoc carnosum* strain of the invention comprising the steps of: transforming *Leuconostoc carnosum* with at least one nucleic acid molecule encoding a lactose transporter polypeptide and a β-galactosidase polypeptide, and selecting transformed cells of *Leuconostoc carnosum* characterised by the ability to grow on lactose as sole carbon source.

### Brief description of the figures

Figure 1: Growth of *Leu. carnosum* 4010 in the chemically defined medium (Table 3) supplemented with either 1 % (w/v) glucose (minimal medium: -) or 1% (w/v) glucose and 0.34g YNB per liter (minimal medium + YNB: x). Growth followed by OD 620 measurements over time (minutes).
Figure 2. Growth in chemically defined medium (Table 3) of recombinant *Leu. carnosum* SH0020 (-■-) with 1 % (w/v) lactose compared to *Leu. carnosum* 4010 (-●-) with 1% (w/v) glucose. Growth followed by OD 600 measurements over time (minutes).
Figure 3. Specific growth rates of lactose positive recombinant strains of *Leu. carnosum* in chemically defined medium (Table 3) supplemented with 1% (w/v) lactose.

### Definition/abbreviation of terms

### Growth media:

**ABT agar:** containing 1mM MgCl₂, 0.1 mM CaCl₂, 0.01 mM FeCl₃, 15 mM (NH₄)₂SO4, 41.68 mM Na₂HPO₄, 22 mM KH₂PO₄, 51.33 mM NaCl, 2.5 mg/l Thiamin and 20 g/l Bacto Agar, Difco Laboratories, Detroit, USA.
**BHI broth:** Brain heart infusion supplied by Oxoid Ltd, Basingstoke, Hampshire, England.
**GM17 agar:** comprising 1% (w/v) glucose and 48.25 g/l M-17 agar supplied by Oxoid Ltd, Basingstoke, Hampshire, England.
**LB:** Luria-Bertani medium containing 4 g/l NaCl, 5 g/l Yeast extract supplied by Difco, Detroit, USA, 10 g/l Peptone from pancreatically-digested casein supplied by Merck KGaA, Darmstadt, Germany and 20 g/l Bacto Agar, Difco Laboratories, Detroit, USA.
**SA agar:** Synthetic Amino acid agar. The composition of this medium is given in Jensen, P.R. and Hammer, K. (1993) Applied and Environmental Microbiology. 59: 4363-4366.
**YNB:** Yeast Nitrogen Base w/o amino acids and ammonium sulphate, Difco Laboratories, Detroit, USA.

### Additional terms:

**Bacteriocin:** a peptide produced by *Leuconostoc* species that is toxic to *Listeria monocytogenes.*
**CAM:** chloramphenicol
**Hybridisation stringency:** a nucleic acid molecule will hybridise with another nucleic acid molecule sharing sequence homology to form a hybrid, as for example during a Southern Hybridisation assay. The stability of the formed hybrid, under conditions of increasing stringency during the washing step of the assay, is a measure of the sequence homology between the hybridising molecules. Washing under high stringency conditions corresponds to at least 1XSSC at 65°C. Optionally, the washing step can be performed at 0.5XSSC at 65°C.
*Leuconostoc* strains: include *Leuconostoc carnosum,* also designated as *Leu. carnosum;* and *Leuconostoc lactis* also designated as *Leu. lactis.*
**PCR:** polymerase chain reaction performed according to the following general procedure: Two reaction mixes are prepared on ice:
Mix 1: 5 µl 5 mM dNTP-mix, 3 µl 10 µl Forward primer, 3 µl 10 µl Reverse primer, 3 µl 5 ηg/ml template DNA and 31 µg H₂O.
Mix 2: 5µl Elongase enzyme mix provided by Invitrogen, 25 µl Buffer B provided by Invitrogen and 45 µl H₂O.
20 µl of mix 1 and 30 µl of mix 2 are mixed together and the following amplification is performed using a PCR thermocycler:

| Step no. | time (min) | Temperature (°C) |
|---|---|---|
| 1 | 4 | 94 |
| 2 | 1 | 94 |
| 3 | 1 | 50 |
| 4 | 5 | 68 |

Steps number 2-4 are repeated 30 times, whereafter another 20 µl of mix 1 and 35 µl of mix 2 (without the template DNA) are added to the reaction mix and one more cycle of steps 1-4 is performed.
**PTS:** phosphoenolpyruvate-dependent phosphotransferase is a system which facilitates the transport of mono- and di-saccharides.
**Recombinant:** comprises genetic variation in a cell (including bacterial) resulting from a recombinant event such as mutation (random genetic change within a cell's own genetic code). Bacterial mechanisms for exchanging genetic material may also result in a recombinant bacterium that contains a combination of traits from two different parental cells. Different modes of exchange identified in bacteria include:
1. transformation (the transfer of naked DNA from one bacterial cell to another in solution, including dead bacteria),
2. transduction (the transfer of viral, bacterial, or both bacterial and viral DNA from one cell to another via bacteriophage) and;
3. conjugation (the transfer of DNA from one bacterial cell to another via a conjugation pilus).

Recombinant bacteria may also result from the transfer and stable inheritance of foreign DNA produced by genetic engineering. Bacteria, having acquired DNA from any of these events, can then undergo fission and pass the recombined genome to new progeny cells.

**Starter culture:** a culture of one or more food grade lactic acid bacterial strains suitable for addition to milk or milk products in the manufacture of diary products and beverages (e.g. kefir, drinking yogurt).

**Vector:** nucleic acid molecule employed for cloning and optionally expression of a gene. The vector is either capable of self-replication, e.g. plasmid, or is non-self-replicating in a host cell (e.g. *Leu. carnosum*) and functions as an integration plasmid.

**X-gal:** 5-bromo-4-chloro-3-indolyl-β-D-galactopyranoside is a non-inducing chromogenic substrate for β-galactosidase, which hydrolyzes X-Gal forming an intense blue precipitate. X-Gal is utilized for detection and/or selection of β-galactosidase gene activity in transfected/transformed cells.

### Detailed description of the Invention

### Metabolic properties of Leuconostoc:

*Leuconostoc* is a facultative anaerobe, depending on mono- or di-saccharides as a fermentable source of energy. All *Leuconostoc* strains ferment glucose, although the majority of strains prefer fructose. *Leuconostoc* belongs to a subgroup of lactic acid bacteria where the end product of fermentation is less that 90% lactic acid, and may include large amounts of ethanol, acetate and CO₂, produced by a form of heterofermentation (Garvie,El., (1986) Bergey's Manual of Systematic Bacteriology, vol. 2, 9th ed. Eds: Sneath, PHA et al., Williams & Wilkins, Baltimore, p.1071-1075). Heterofermentative organisms have no perfect glycolysis due to the absence of the glycolytic enzyme fructose 1,6-diphosphate aldolase, instead relying on a combination of glycolysis and the phosphoketolase pathway to break down glucose. The nutritional requirements of lactic acid bacteria, including some *Leuconostoc* strains, has been shown to be complex, with a requirement for vitamins, amino acids, purines and pyrimidines, which may account for their limited natural distribution.

### Growth on lactose as sole carbon source:

*Leuconostoc carnosum* subspecies have been shown to ferment the sugars cellobiose, fructose, glucose, mannose, mellibiose, ribose, salicin, sucrose and trehalose, but not lactose. The ability to utilise a fermentable carbohydrate source relies on a transport mechanism for intracellular import of the carbohydrate energy source or its degradation product, and a metabolic pathway for its catabolism. At least three mechanisms for the transport of sugars are known to occur in prokaryotes, namely a permease system, an ATP-binding cassette transporter (ABC transporter), and a phosphoenol pyruvate-dependent phosphotransferase system (PTS).

The PTS system for lactose import in *Lactococcus lactis* and *Lactobacillus casei* is characterised by a trans-membrane protein EIIBC that phosphorylates lactose and transports it into the cell. A phosphorylation chain, comprising Enzyme 1, coupled to histidine protein, coupled to Enzyme IIA, serves to transfer a phosphoryl group from phosphoenol pyruvate through to the transmembrane protein EIIBC (de Vos, WM and Vaughan, EE (1994) FEMS Microbiology Reviews 15: 217-237). The pathway is energetically favourable because PTS mediated lactose uptake has no net ATP cost. The genes encoding this PTS pathway are known, and in *L. lactis* they are located on a so-called `lactose plasmid' which carries the lac operon for utilization of lactose and galactose.

The ATP transporter comprises two transmembrane domains, which mediate sugar import, designated as LacF and LacG *in Agrobacterium radiobacter,* and two cytoplasmic domains (LacK/LacK) that provide energy required to drive uptake via ATP hydrolysis (de Vos, WM and Vaughan, EE (1994) *supra*).

The permease system transports protons or Na⁺ ions and mono- or di-saccharides across the cell membrane. Transport is driven by proton motive force that is generated by a proton or Na⁺ gradient maintained by a H⁺ ATPase pump (de Vos, WM and Vaughan, EE (1994) *supra*). The permease system is known to operate as a proton-sugar symporter or as a sugar-sugar anti-porter. The LacS permease system of *Streptococcus termophilus* is known to operate in two modes, either as an antiporter, whereby LacS transports lactose into the cell and exports galactose, or a galactose/H⁺ symporter. Lactose, imported into the cell, is cleaved by β-galactosidase into glucose and galactose, where galactose export is seen to occur in cells that are unable to metabolise galactose. In lactic acid bacteria, lactose imported into the cell may be further hydrolysed by β-galactosidase into glucose and galactose, where glucose enters the glycolytic and phosphoketolytic pathway, and galactose is metabolised by the Leloir pathway. Accordingly uptake and fermentation of lactose, imported via the permease system, may involve both a permease and a β-galactosidase, coupled to downstream metabolic pathways. Although *Leu. carnosum* is lactose-negative, other *Leuconostoc* species can transport lactose via the permease pathway, e.g. *Leu. lactis* that has a *lacS* encoded permease, and a *lacLM* encoded β-galactosidase. Both the *lacS* and *lacLM* genes have been found on a plasmid (pNZ63) isolated from the *Leu. lactis* strain NZ6009/ DMS 20202. The Leloir pathway enzymes for galactose metabolism in *Leu. lactis* are encoded by chromosomal genes. In contrast, in S. *termophilus,* the genes encoding both LacS, β-galactosidase and the Leloir pathway enzymes, are known to be harboured on a plasmid.

### Isolated Leu. carnosum strain able to grow on lactose as sole carbon source:

The present invention provides a lactose-positive recombinant strain of *Leu. carnosum* which, in one embodiment, has been constructed by introducing genes, coding for both lactose uptake and utilization, into *Leu. carnosum* by transformation. Gene encoded functions required to confer a lactose-positive phenotype in *Leu. carnosum* include at least a lactose transporter, and preferably also an enzyme that catalyses the hydrolysis of lactose or lactose-phosphate, for example, but not limited to, a β-galactosidase or β-glucosidase. Genes encoding lactose transporters that may be introduced into *Leu. carnosum* include both PTS, ABC transporter and permease genes.

Preferred examples include the PTS encoding genes of the lactose operon of *L. lactis,* which is harboured on the 'lactose plasmid'. For example, the plasmid pool from *L. lactis* FHCY-1, comprising the 'lactose plasmid', is isolated and each plasmid in the pool is fused with the replicon and the selective antibiotic marker from the vector pCI372, and transformed into *Leu. carnosum.* Lactose positive recombinant strains of *Leu. carnosum* are selected by screening for chloramphenicol resistance and the ability to grow on lactose as the sole sugar source.

Preferred examples of permease encoding genes that may be used to provide a lactose-positive recombinant *Leu. carnosum* strain, include *lacS* from *Leuconostoc lactis, Lactobacillus plantarum* or *Streptococcus termophilus.* Genes encoding β-galactosidase, which may be introduced into *Leu. carnosum,* include *lacLM* from *Leuconostoc lactis,* the *lacA* from *Lactobacillus plantarum,* LacG from *Streptococcus termophilus* or *LacZ* from *Streptococcus termophilus, Lactococcus lactis* or *E.coli.*

The genes encoding LacS (a transporter for lactose) and LacLM (a β-galactosidase) that may be employed in the present invention are both present on the plasmid pNZ63 from *Leu. lactis* DMS 20202. Likewise a 'lactose plasmid' with genes for the utilization of lactose is present in *Leu. lactis* CHCC 1990 and can thus be used for transformation into *Leu. carnosum* to engineer a lactose positive strain.

One or more genes, of known origin, encoding functions required to confer a lactose-positive phenotype may be introduced into *Leu carnosum* by means of transformation. The nucleic acid molecule comprising said genes may be obtained from genetic material (DNA/RNA) isolated from its source of origin, employing a variety of standard molecular biology procedures, known to a skilled person. In brief DNA (or cDNA derived from RNA), isolated from its source of origin, is restriction digested and cloned as a DNA library in a suitable vector and amplified in a host cell. A clone comprising the gene of interest may subsequently be screened out from the DNA library using standard DNA screening protocols (including colony/DNA hybridisation), thereby providing a pure source of the required nucleic acid molecule. Alternatively, the nucleic acid molecule comprising the gene of interest may be amplified from said genetic material employing for example PCR based technology, using primers homologous to the 5' and 3' termini sequence of the respective gene. In a further approach, a synthetic gene may be assembled from synthetic nucleic acid molecules, having a nucleotide sequence corresponding to the gene of interest.

Nucleic acid molecules comprising one or more genes encoding functions required to confer a lactose-positive phenotype, comprise at least one coding sequence domain, encoding at least one function, that is functionally linked to a promoter domain, wherein said two domains may be of homologous or heterologous origin with respect to each other. The genes encoding functions that confer a lactose-positive phenotype may be located in an operon that is operably-linked to a promoter domain. A promoter domain capable of directing expression of said coding sequence domain is functionally linked to said coding sequence. For example, said coding sequence domain may be functionally linked to a library of synthetic promoter sequences, from which one or more promoter domain functionally linked to said coding sequence domain, and having the desired level of promoter strength (transcription activity) can be selected, as illustrated in Example 1 and described by Solem C and Jensen PR (2002) Applied and Environmental Microbiology 68: 2397-2403.

Nucleic acid molecules may be transformed into *Leu. carnosum* by means of transformation, either directly, or following cloning into a suitable plasmid. Suitable plasmids that replicate and are stably maintained in *Leu. carnosum* include pG+host8 (TET^{R}) (Maguin, E., Prévost, H., Ehrlich,S.D. and Gruss, A. (1996) Journal of Bacteriology 178: 931-935); pCI3340 (CAM^{R}), and pCI372, (Hayes, F., Daly, C. and Fitzgerald, G.F. (1990) Appl. Environ. Microbiol. 56: 202-209) and are thus suitable plasmids for harbouring genes required to confer a lactose-positive phenotype. It may furthermore be desirable to employ a plasmid which does not include any antibiotic resistance genes, e.g. by selecting for transformants with a lac+ phenotype conferred by the plasmid carrying the lactose genes, as shown in example 3. The given examples serve to illustrate the steps of isolating and/amplifying nucleic acid molecules comprising genes encoding lactose transport and β-galactosidase functions, their insertional cloning into a vector, and the transformation of the recombinant vector into a *Leu*. *carnosum* 4010 cell (Budde et al., (2003) International Journal of Food Microbiology. 83: 171-184). Said nucleic acid molecules may be maintained in a host *Leu. carnosum* cell by virtue of being harboured on a stable, maintained plasmid. Alternatively, genes encoding lactose transport and β-galactose functions may be stably integrated into the genome of *Leu. carnosum.* Briefly, nucleic acid molecules comprising said genes are cloned into a vector that is not capable of self-replication in *Leu. carnosum.* The recombinant vector is transformed into *Leu. carnosum,* followed by selection of lac+, gal+ recombinant cells. Integration events are facilitated by homologous recombination between homologous sequences present in both the *Leu. carnosum* genome and in vector sequences flanking said genes. Transformation protocols suitable for the introduction of a vector or nucleic acid molecule into *Leu. carnosum* are described in Example 2 and Helmark S et al., (2004) Appl. Environmental Microbiol. 70: 3695-3699. In a further embodiment, natural plasmids containing the lactose genes found in bacteria closely related to *Leu. carnosum,* e.g. *Leuconostoc lactis* and *Lactococcus lactis,* may be transferred to *Leu. carnosum* by conjugation or by natural transformation.

As illustrated in the given examples, a recombinant *Leu. carnosum* strain with a lactose positive phenotype, may be obtained by introducing at least one nucleic acid molecule encoding a functional lactose transporter, and preferably also a β-galactosidase or β-glucosidase, into a cell of *Leu. carnosum.* A recombinant *Leu. carnosum* strain of the invention thus includes a cell that comprises a nucleic acid molecule encoding a lactose transporter polypeptide having an amino acid sequence that is at least 60%, 65%, 70%, 75%, 80%, 85%, 90% or 95% homologous to the amino acid sequence of SEQ ID NO: 2, or a fragment thereof, conferring functional lactose transporter activity. A nucleic acid molecule encoding said lactose transporter encompasses a nucleic acid molecule that encodes a lactose transporter conferring functional lactose transporter activity and having a nucleic acid sequence that is at least 60%, 65%, 70%, 75%, 80%, 85%, 90% or 95% homologous to the nucleic acid sequence of SEQ ID NO: 6.

Furthermore, a recombinant *Leu. carnosum* strain of the invention includes a cell that comprises a nucleic acid molecule encoding a β-galactosidase polypeptide having an amino acid sequence that is at least 60%, 65%, 70%, 75%, 80%, 85%, 90% or 95% homologous to the amino acid sequence of SEQ ID NO: 3, or a fragment thereof, conferring functional β-galactosidase activity. A nucleic acid molecule encoding said β-galactosidase encompasses a nucleic acid molecule that encodes a β-galactosidase conferring functional β-galactosidase activity and having a nucleic acid sequence that is at least 60%, 65%, 70%, 75%, 80%, 85%, 90% or 95% homologous to the nucleic acid sequence of SEQ ID NO: 4.

In an alternative embodiment, a recombinant *Leu. carnosum* strain expressing a lactose positive phenotype is obtained by mutagenic activation of any native cryptic genes encoding proteins required for lactose metabolism and transport present in the *Leu. carnosum* genome. Suitable mutagenic treatments, well known in the art include, for example, treatment with a chemical mutagen such as ethanemethane sulphonate (EMS) or N-methyl-N'-nitro-N-nitroguanidine (NTG), or exposure to UV light.

Selection of recombinant *Leu. carnosum* cells that are mutants, or are transformed with genes that confer a lactose positive phenotype and β-galactosidase activity may be selected on appropriate growth media: namely a minimal medium or a chemically defined growth-medium supplemented with lactose as sole fermentable sugar carbon in order to select for lactose transport and fermentation; and a growth-medium supplemented with x-gal to detect β-galactosidase activity. The present invention provides the first described chemically defined growth-medium for *Leu. carnosum,* described in Example 3, which may be supplemented with lactose as sole fermentable sugar and used for selecting and maintaining lactose-positive recombinant *Leu. carnosum* cells.

Lactose-positive recombinant *Leu. carnosum* cells may be detected and identified by virtue of their lactose-positive and x-gal positive phenotype. In addition they may be identified by virtue of the presence of heterologous genes encoding lactose permease and β-galactosidase functions. Thus, molecular probes, capable of hybridising to the heterologous genes encoding these functions may be used to identify cells having genetic material comprising these genes, employing standard detection techniques, including DNA hybridisation and PCR amplification and transcript profiling.

### Leuconostoc species produce toxic bacteriocin peptides:

The invention is directed to lactose-positive recombinant *Leu*. *carnosum* and its use in the bio-preservation of milk and milk products. The *Leuconostoc* species, *Leu. carnosum,* has been found to produce bacteriocins that are particularly toxic to *Listeria monocytogenes.* Bacteriocins form pores in the membranes of a sensitive target cell, thereby depleting the trans-membrane potential and pH gradient across the cell membrane and causing leakage of intracellular material. The bacteriocins produced by *Leu. carnosum* belong to Class IIa bacteriocin peptides (also known as camocin or leucocin), characterised by having a molecular mass of <5kDa, heat-stability, and the consensus sequence YGNGVXaaC in the N-terminal part of the peptide, associated with a disulfide bridge. Thus the lactose-positive recombinant *Leu. carnosum* cell of the invention is a cell capable of producing a bacteriosin, and preferably a bacteriosin belonging to the Class IIa bacteriosin. In a particularly preferred form of the invention the lactose-positive *Leu. carnosum* cell produces two bacteriosins classified as leucocin A-4010 and leucocon B-4010 (Budde et al., (2003) International Journal of Food Microbiology. 83: 171- 184). The N-terminal sequences of these peptides are KYYGNGVHCTKSGCSVNWGEAFSAGVHRLA- (leucocin A-4010) and KNYGNGVHCTKKGCSVDWGYAWTNIANNSVMNGLTGGNA-(leucocin B-4010) (Budde et al., (2003) International Journal of Food Microbiology. 83: 171- 184). The disclosed invention may be practised with any recombinant *Leu. carnosum* cell having a lactose-positive phenotype that may be constructed or produced according to the described methods. Subspecies of *Leu. carnosum* that may provide a suitable host cell for the construction of a lactose-positive strain include *Leu. carnosum* 4010 (CHCC 1043) obtainable from Chr.Hansen A/S, 10-12 Boge Allé, DK 2970 Hørsholm as B-SF-43, sold under the trade name Bactoferm; LA44A, LA54A, Tal1a.

### Starter cultures comprising lactose-positive recombinant Leu. carnosum

The lactose-positive recombinant *Leu. carnosum* cell of the invention may be used as a food-grade starter culture in the manufacture of fermented milk products. A starter culture, comprising the lactose-positive recombinant *Leu. carnosum* strain of the invention, may be a pure culture of said strain or alternatively may include a mixture of one or more additional strains of different lactic acid bacterial species, such as *Streptococcus* and *Lactobacillus* species. The selection of strains to be combined with the *Leu. carnosum* strain of the invention will depend on the type of dairy product to be manufactured. Mesophilic cultures comprising species of *Lactococcus, Leuconostoc, and Lactobacillus* are preferred for cheese and butter manufacture, whereas cultures of thermophilic species of *Streptococcus* and *Lactobacillus* are preferred for fermented milk products such as yoghurt. Starter cultures of the present invention may also include fungal cultures, for use in the manufacture of certain types of cheese, for example *Penicillum roqueforti, Penicillum candidum* and *Geotrichum candidum.*

Starter cultures according to the invention may be prepared as concentrates which may be stored and distributed in frozen form, without loss of viability. Alternatively the starter culture may be freeze-dried or lyophillsed, or alternatively stored in liquid form with a preservative. Starter cultures, according to the invention, whether liquid or dried, may be added directly into milk or dairy products as direct vat set (DVS) cultures. However, it is not uncommon in the dairy industry to prepare and maintain bulk starter cultures. Thus the starter culture according to the present invention may be inoculated into heat-treated milk and incubated to allow propagation of the starter culture strains to the desired cell number, in order to prepare a 'bulk starter'. Alternatively, the starter culture of the invention is pre-activated, to diminish its lag-phase of growth, by incubation in a small volume of pre-heated milk for 30 to 60 minutes before adding to the milk for manufacture of a dairy product.

A food-grade starter culture according to the invention, comprising lactose-positive recombinant *Leu. carnosum,* may be used in the manufacture of a fermented milk product including: cheese (pasteurised and un-pasteurised cheese); soft or hard cheese; ripened cheese (including Cheddar, Colby, Edam, Muenster, Gruyere, Emmanthal, Camembert, Parmesan and Romano); blue cheese (including Danish blue and Roquefort); fresh cheese (including mozzarella and Feta); acid coagulated cheese (including cream cheese, Neufchatel, Quarg, Cottage cheese and Queso Blanco); and pasta filata cheese; and other cheese types (Campesino, Chester Danbo, Drabant, Herregård, Manchego, Provolone, Saint Paulin); buttermilk; sour cream; yoghurt; and kefir.

The starter culture of the invention can be employed in a cheese making process that may comprise the steps of:
- equilibrating milk (optionally pasteurised milk) to a temperature suitable for the selected starter culture,
- addition of the starter culture to the milk and incubation to allow fermentation to form a cheese milk with the desired fall in pH,
- coagulation of the cheese milk to produce milk curds by either addition of rennet, or acid (and optionally heat) treatment,
- cutting, draining, pressing of the milk curds, with optional addition of salt.

### Example 1: Construction of lactose positive recombinant strains of Leu. Carnosum comprising Streptococcus thermophilus DNA sequences encoding a lactose uptake transporter and β-galactosidase.

For the production of lactose-positive recombinant strains of *Leu. carnosum,* the following strategy was applied: A plasmid (pSH101) harbouring the genes for utilization of lactose was constructed in the vector pCI372 according to a cloning strategy detailed below, and pSH101 was then introduced in *Leu. carnosum* by means of electroporation and natural transformation. The vector pC1372, that is described by Hayes *et al,* (1990) *supra* and Lucey M, et al, (1993) FEMS Microbiol Lett. 110(3): 249-56, was chosen because it has been shown to replicate in *Leu. carnosum* (Helmark, S., et al., (2004) Applied and Environmental Microbiology. 70: 3695-3699.

Protocol for cloning genes for lactose utilisation: The *lacSZ* nucleic acid molecule [SEQ ID NO:1] contains two genes encoding a lactose uptake transporter [SEQ ID NO: 2], and a β-galactosidase [SEQ ID NO:3]. The two genes present in the *lacSZ* nucleic acid molecule are individually the *lacZ* gene [SEQ ID NO: 4], encoding a β-galactosidase [SEQ ID NO:5], and the *lacS* gene [SEQ ID NO: 6], encoding a lactose uptake transporter [SEQ ID NO: 7]. The *lacSZ* nucleic acid molecule [SEQ ID NO:1] was obtained by means of PCR amplification of chromosomal DNA purified from *Streptococcus thermophilus* (obtained from an isolate from a "sødmælksyoghurt" from Aria Foods amba, Viby J., Denmark. PCR was performed with a forward primer mixture comprising a synthetic randomized promotor sequence (Solem C and Jensen PR, 2002 *supra*):
5'ACGACTAGTGGATCCATNNNNNAGTTTATTCTTGACANNNNNNNNNNN NNNTGRTATAATNNNNAAGTAATAAAATATTCGGAGGAATTTTGAAATGG AAAAATCTAAAGGTCAG -3' (wherein N = 25% each of A, C, G and T; R = 50% each of A and G; SEQ ID NO: 8) and a reverse primer:
5'-GGTACTCGAGGAAGATACTAACACACTAATG-3', (SEQ ID NO: 9) thereby yielding a mixture of *lacSZ* gene fragments fused to promotors of varying strengths.

The fragment mixture was digested with *BamHI* and *Xho1* and the vector pCI372 was digested with *BamHI* and *SaIl* (*SaI1* is compatible with *Xho1*).

The vector DNA was further treated with Shrimp Alkaline Phosphatase (SAP) to prevent re-ligation of the cloning vector. Subsequently, the fragment mixture and the SAP-treated vector DNA were ligated overnight at 16°C using T4 DNA ligase and standard ligation conditions. Similarly, the fragment mixture was ligated under the same conditions to vector DNA that had not been treated with SAP, as a control to monitor vector self-ligation.

Ligation mixtures were transformed into *E. coli* MC-1000 (Boogerd, F.C., et al. (1998) J. Bacteriol., 180, 5855-5859) and *Lactococcus lactis* spp. *cremoris* MG-1363 (Gasson MJ. (1983) J Bacteriol. Apr;154(1):1-9) by electroporation and after a phenotypic induction in BHI broth supplemented with 0.25M sucrose, 27.8mM glucose, 20mM MgCl₂ and 2mM CaCl₂ for a period of two hours, transformants were selected on either lactose minimal agar plates (ABT agar with 1% (w/v) lactose for *E. coli* and Synthetic Amino acids (SA) containing 1% (w/v) lactose for *L. lactis*) or agar plates containing chloramphenicol (CAM) and x-gal (LB containing 10 µg/ml CAM, 100 µg/ml x-gal and 1% (w/v) glycerol for *E. coli* and GM17 agar with the same supplements for *L. lactis*). The *E. coli* MC-1000 and *L. lactis* MG-1363 transformants obtained are shown in Table 1.

**Table 1. Transformants of E. coli MC-1000 and L. lactis MG1363.**

| Strain | SAP-treatment of vector | Ligation | Minimal plates | CAM-x-gal plates (total no. of colonies) | CAM-x-gal plates (blue colonies) |
|---|---|---|---|---|---|
| MC-1000 | Yes | 1Xligation | 0 | 5.E+04 | 2 |
| | No | 1Xligation | 9 | 5.E+04 | 0 |
| | Yes | Religation | 0 | 5.E+04 | 0 |
| | No | Religation | 6 | 5.E+04 | 0 |
| MG-1363 | Yes | 1Xligation | 80 | 1.E+04 | 21 |
| | No | 1Xligation | 92 | 1.E+04 | 15 |
| | Yes | Religation | 1 | 1.E+04 | 0 |
| | No | Religation | 0 | 5.E+03 | 0 |

| | | | | | |
|---|---|---|---|---|---|
| (1Xligation = ligation mixture with a vector to fragment molar ratio of 1:1; religation= ligation mixture comprising vector DNA alone) | | | | | |

The SAP treatment of the vector prior to ligation is shown to be effective by the absence of *E. coli* MC-1000 colonies growing on minimal medium following transformation with SAP treated and self-ligated vector (re-ligation). Ligation of the *lacSZ* fragment into the pCI372 vector was confirmed by growth of *L. lactis* MG-1363 transformed with the ligated plasmid, but not the self-ligated vector, on lactose-supplemented minimal medium plates.

128 of the MG-1363 transformants were chosen for further study, comprising colony numbers: 1-47 selected on CAM-x-gal plates and colony numbers 48-128 selected on lactose minimal medium plates. Plasmid DNA was purified from the selected MG-1363 transformants in such a way that the purified DNA was pooled in four groups: 1) DNA from colony number 1 to 31; 2) from 32 to 62; 3) from 63 to 98; and 4) from 99 to 128 and each of the plasmid pools were transformed into *Leu. carnosum* using electroporation, as well as natural transformation (Helmark, S., et al., (2004) Applied and Environmental Microbiology. 70: 3695-3699) according to the following procedures:

Protocol for electroporation: Competent cells were obtained by growing a culture of *Leu. carnosum* 4010 overnight in BHI broth supplemented with 0.25 M sucrose, 27.8 mM glucose, and 1% (wt/vol) glycine. This culture was diluted in an appropriate volume of the same solution to give an initial optical density at 620nm of preferably about 0.03 to 0.04 and then grown for between 1-2 generations, before transferring the culture to ice for 2 minutes and then harvesting the cells by centrifugation at 5,000 xg for 5 minutes at 4°C. The harvested cells were washed in ice-cold washing solution comprising 0.25 M sucrose and 10% (wt/vol) glycerol, and then re-suspended in washing solution at a cell density corresponding to an OD 620nm of 30, to provide competent cells for electroporation. A transformation mixture, comprising 50µl sample of the *Leu. carnosum* 4010 competent cells and about 0.05µg plasmid DNA, was transferred to a sterile electroporation cuvette with an inter-electrode distance of 0.2 cm, and stored on ice for 5 minutes. The transformation mixture in the cuvette was then subjected to a pulse of 25 µF, 200Ω, and between about 2.5 to 5.0 kV/cm, and immediately there after re-suspended to a final volume of 1ml of pre-temperature equilibrated (about 18 - 25°C) BHI broth supplemented with 0.25M sucrose, 27.8mM glucose, 20mM MgCl₂, and 2mM CaCl₂, and incubated at 25°C for 2 h to allow phenotypic expression of plasmid genes. The transformation mixture was then centrifuged at 4,000 xg for 2 minutes. The pelleted cells were re-suspended in 1ml 0.9% (wt/vol) NaCl, and dilutions were plated and grown at 25°C on lactose-minimal agar plates comprising pre-treated BHI medium [medium that is pre-grown with *Leu. carnosum* in order to remove fermentable carbohydrates; filter sterilized; and supplemented with 1% (w/v) lactose]; and on CAM-x-gal agar plates comprising BHI agar containing 5 µg/ml CAM, 100 µg/ml x-gal and 1 % (w/v) glycerol.

Protocol for natural transformation: transformation was performed essentially according to the above procedure for electroporation, with the exception that the electroporation pulse was omitted. The *Leu. carnosum* 4010 transformants obtained by electroporation or natural transformation are shown in Table 2.

**Table 2. Transformants of Leu. carnosum 4010.**

| Plasmid pool | Electroporation (E)/ Natural Transformation (NT) | Lactose-Minimal | CAM-x-gal (total no. of colonies) | CAM-x-gal (% blue colonies) |
|---|---|---|---|---|
| 1 | E | 0 | 498 | 50 |
| 2 | E | 5 | 762 | 66 |
| 3 | E | 1 | 329 | 45 |
| 4 | E | 0 | 544 | 95 |
| none | E | 0 | 0 | - |
| 1 | NT | 0 | 837 | 50 |
| 2 | NT | 0 | 597 | 75 |
| 3 | NT | 0 | 486 | 50 |
| 4 | NT | 6 | 499 | 95 |
| none | NT | 0 | 0 | - |

The *Leu. carnosum* 4010 colonies detected on CAM-x-gal plates varied significantly in size, with a diameter of up to 1.0 mm, where the smallest colonies had the strongest colour intensity. The colonies detected on lactose minimal plates had a diameter of 0.3-0.5 mm and were slightly yellowish. A total of 45 *Leu. carnosum* 4010 colonies, comprising colonies SH0001-SH0012 (selected on lactose-minimal plates and SH0013-SH0045 (selected on CAM-x-gal medium) were re-streaked on CAM-x-gal plates, and all colonies grew to a diameter of 0.1-1.0 mm with varying, but slightly reduced, colour intensity. The phenotype of nine of the β-galactosidase positive transformants was investigated by growth in a chemically defined medium for *Leu. carnosum* (see Example 3).

### Example 2: Construction of lactose-positive recombinant strains of Leu. Carnosum comprising Leuconostoc lactis DNA sequences encoding a lactose uptake transporter and β-galactosidase.

A lactose-positive *Leu. carnosum* strain is obtained by transferring the genes encoding lactose permease and beta-galactosidase from the close relative, *Leuconostoc lactis.* In *Leuconostoc lactis* the gene for lactose transport; *lacS* [SEQ ID NO: 10] encoding a lactose uptake transporter [SEQ ID NO: 11] and the genes for lactose degradation; *lacLM* [SEQ ID NO: 12] encoding a β-galactosidase composed of the subunits LacL [SEQ ID NO: 13] and LacM [SEQ ID NO: 15] are located on a plasmid (pNZ63) described by Vaughan et al. (1996) Applied and Environmental Microbiology 62:1574-1582 and David et al. (1992) J. Bacteriology 174: 4475-4481. Three different approaches are used for transferring the *Leuconostoc lactis lacS* and *lacLM* genes into *Leu. Carnosum* as given below:
1) Conjugation: the *Leuconostoc lactis* lactose plasmid (pNZ63) is transferred to *Leu. carnosum* by conjugation using a rifampicin-resistant spontaneous mutant of *Leu. carnosum* (Lc-RIF) obtained by mutation and selection on rifampicin-supplemented medium. *Leuconostoc lactis* and Lc-RIF cultures are then grown to an optical density at 600nm of 0.5, and the two cultures are then mixed and allowed to stand for 2 hours at 28°C. Aliquots of 200 microliters of the mixed and incubated cultures are then plated in 10⁰, 10⁻¹, 10⁻², 10⁻³, 10⁻⁴ and 10⁻⁵ dilutions on a chemically-defined growth medium for *Leuconostoc carnosum* (see Example 3) supplemented with 100 micrograms rifampicin and 1% lactose. Under these conditions only *Leuconostoc carnosum* strains that have received the lactose plasmid by conjugation form colonies. These lactose-positive colonies are re-streaked to single colonies and selected for their level of bacteriocin production.
2) Natural transformation: the *Leuconostoc lactis* lactose plasmid (pNZ63) is transferred to *Leu. carnosum* by natural conjugation. A cell- free extract of *Leuconostoc lactis* is first prepared by sonication. A culture of *Leu. carnosum* cells is grown under conditions that renders cells naturally competent (see Example 1 and Helmark *et al, supra* 2004), and the cells are spun down and re-suspended on the chemically-defined growth medium for *Leuconostoc carnosum* (Example 3) without a source of sugar and mixed with the cell-free extract of *Leuconostoc lactis.* After a 1 hour incubation at 28°C, the cells are plated in 10⁰, 10⁻¹, 10⁻², 10⁻³, 10⁻⁴ and 10⁻⁵ dilutions on the chemically-defined growth medium for *Leuconostoc carnosum* supplemented with 1% lactose. Under these conditions only *Leuconostoc carnosum* strains that have received the lactose plasmid by transformation form colonies. The lactose-positive colonies are re-streaked to single colonies and selected for bacteriocin production level.
3) Transformation: the *Leuconostoc lactis lacS* and *lacLM* genes are subcloned and transferred to *Leu. carnosum* by transformation as follows. The genes *lacLM* [SEQ ID NO: 12], (encoding a β-galactosidase [SEQ ID NO: 13 and 15) and *lacS* [SEQ ID NO: 10] (encoding a lactose uptake transporter [SEQ ID NO: 11]), are obtained by means of standard PCR amplification of the respective genes in plasmid DNA of pNZ63 purified from *Leuconostoc lactis* and the amplified fragments cloned into restriction sites on pCI372. After ligation, the DNA is transformed into *Leuconostoc carnosum* by standard electroporation as described above. After transformation the cells are plated in 10⁰, 10⁻¹ 10⁻², 10⁻³, 10⁻⁴ and 10⁻⁵ dilutions on the chemically defined growth medium for *Leuconostoc carnosum* supplemented with 1% lactose. Under these conditions only *Leuconostoc carnosum* strains that have received the lactose genes inserted in pCI372 form colonies. The colonies are re-streaked to single colonies and analyzed for their level of bacteriocin production.

When the genes *lacS* [SEQ ID NO: 10] and *lacLM* [SEQ ID NO: 12] have been transferred to *Leu. carnosum* by any of the three methods described in this example, the presence of the genes can be verified by standard PCR. The transferred plasmid can be purified by standard plasmid purification for very low-copy number plasmids (for example the "Qiagen very low-copy plasmid purification" kit. Then the existence of the genes on the plasmid can be verified by standard PCR.

### Example 3: A chemically defined growth medium for Leuconostoc carnosum.

The provision of a chemically defined growth medium for the propagation of *Leu. carnosum* would greatly facilitate the selection and growth of metabolic mutants, variants or transformants of this micro-organism, including the identification of lactose positive strains. The specific nutritional requirements of *Leu. carnosum* have not previously been described, however, as a lactic acid bacterium, this organism is expected to have complex requirements, including amino acids, vitamins, salts, micronutrients and nucleobases. The nutritional requirements of other *Leuconostoc* species described in the literature (Koser, S.A. (1968) Vitamin Requirements of Bacteria and yeasts, Ch. C. Thomas Publisher, Springfield, Illinois; Garvie, E.I. (1986) Leuconostoc. In: Bergey's Manual of Systematic Bacteriology, vol. 2, 9th ed. P.H.A. Sneath, N.S. Mair, Sharpe M.E., and Holt J.G. (eds). Williams & Wilkins, Baltimore, 1071-1075; Dellaglio, F. et al., (1995) The genus Leuconostoc. In: The genera of lactic acid bacteria. Wood, B.J.B., Holzapfel, W.H. (eds). Blackie Academic & Professional, London, 235-278) was used as a basis for designing a chemically defined medium for *Leu. carnosum* as shown in Table 3.

**Table 3 A chemically defined growth-medium for Leuconostoc carnosum. The composition of 1 I of chemically defined medium* for Leu. Carnosum**

| | | | |
|---|---|---|---|
| **Amino acids** | **(g)** | **Vitamins (g)** | **(g)** |
| L-alanine | 0.6 | Nicotinic acid | 0.002 |
| L-glutamate | 0.6 | Thiamin | 0.002 |
| Proline | 0.6 | PyridoxinHCl | 0.004 |
| L-serine | 0.6 | Ca Pantothenate | 0.002 |
| L-arginine | 0.4 | Biotin | 0.01 |
| Glycine | 0.4 | Folic acid | 0.002 |
| L-lysine | 0.4 | Riboflavin | 0.002 |
| L-phenylalanine | 0.4 | | |
| L-threonine | 0.4 | **Salts (g)** | **(g)** |
| L-asparagine | 0.25 | KH₂PO₄ | 12 |
| L-glutamine | 0.25 | K₂HPO₄ | 10 |
| L-isoleucine | 0.25 | NH₄Cl | 1 |
| L-leucine | 0.25 | MgCl₂ | 0.15 |
| L-methionine | 0.25 | CaCl₂ | 0.015 |
| L-tryptophan | 0.25 | FeCl₃ | 0.0033 |
| L-valine | 0.25 | K₂SO₄ | 0.095 |
| L-cystein | 0.2 | | |
| L-histidine | 0.1 | **Micronutrients: (nmol)** | **(nmol)** |
| L-tyrosine | 0.1 | (NH₄)₆Mo₇O₂₄ | 6 |
| | | H₃BO₄ | 800 |
| **Nucleobases:** | **(g)** | CoCl₂ | 60 |
| Adenine | 0.02 | CuSO₄ | 20 |
| Guanine | 0.02 | MnCl₂ | 160 |
| Uracil | 0.02 | ZnSO₄ | 20 |
| Hypoxanthine | 0.02 | | |

| | | | |
|---|---|---|---|
| *The medium must be supplemented with a fermentable carbohydrate to support the growth of *Leu. carnosum,* | | | |

The chemically defined medium includes 19 amino acids, since the amino acid requirements of *Leuconostoc* species were known to vary significantly. The nucleobase- and vitamin-requirements of hitherto characterized *Leuconostoc* species include four nucleobases; adenine, guanine, xanthine and uracil and 7 vitamins; nicotinic acid, thiamine, pyridoxine, pantothenate, biotin, folic acid and riboflavin, which were all included in the medium, with the exception that xanthine was replaced by hypoxanthine. A fermentable carbohydrate is added to this medium in order to support the growth of *Leu. carnosum.*

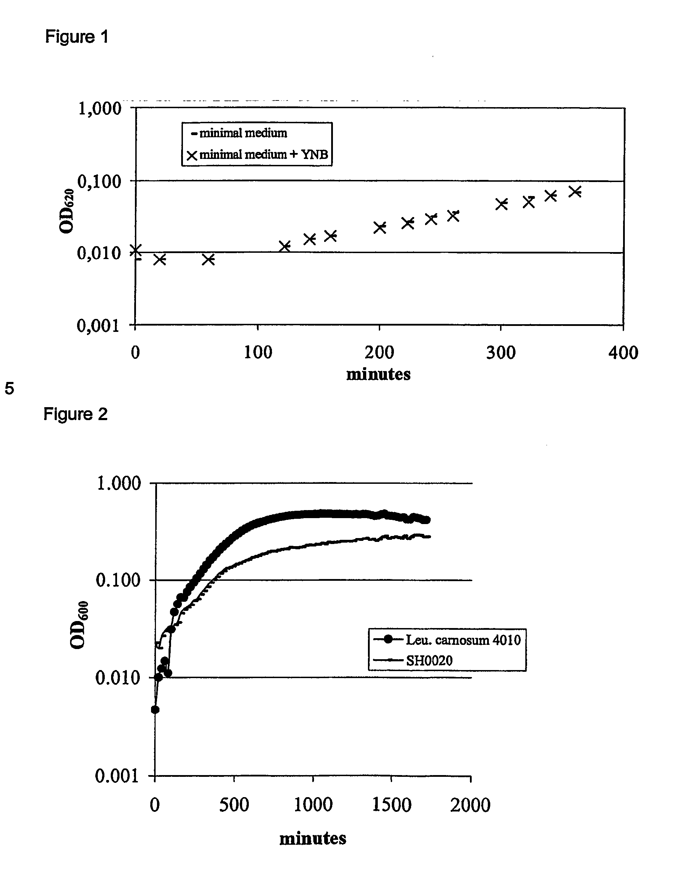

The growth of *Leu. carnosum* 4010 in the chemically defined medium supplemented with 1 % (w/v) glucose is shown in Figure 1. In this medium, *Leu. carnosum* grew to a final OD₆₂₀ of approximately 0.6 and the specific growth rate was 0.44h⁻¹. When YNB (Yeast Nitrogen Base w/o amino acids and ammonium sulphate, Difco, Detroit) was added to the medium at a concentration of 0.34 g/I, *Leu. carnosum* grew to a final OD₆₂₀ of 1.0 and the specific growth rate was in the same range (0.43h⁻¹).

### Example 4: Selection and growth of lactose-positive recombinant Leu. carnosum transformants in chemically defined growth-medium.

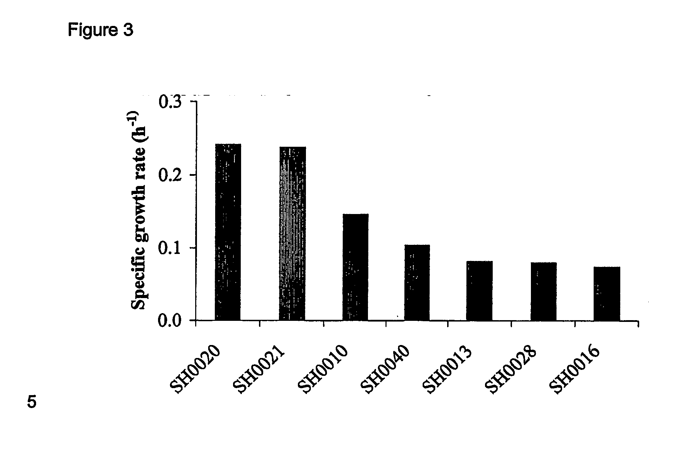

Nine β-galactosidase-positive *Leu. carnosum* 4010 transformants; SH0010, SH0013, SH0016, SH0020, SH0021, SH0024, SH0028, SH0035 and SH0040, obtained according to Example 1 were inoculated in *Leu. carnosum* chemically defined medium supplemented with 1% (w/v) lactose as sole fermentable carbohydrate, as defined in Example 2. The strains SH0024 and SH0035 did not grow. The growth of the remaining strains was followed at 27°C in a Labsystems Bioscreen C from Bie&Bemtsen A/S, Rødovre, Denmark by means of OD₆₀₀ measurements (Figure 2). Due to the different strengths of the randomized promoters, the specific growth rates varied between the strains from 0.074h⁻¹ (for SH0016) to 0.242h⁻¹ (for SH0020), as shown in Figure 3.

### Example 5: Method of detecting a lactose-positive Leu. carnosum of the invention.

A lactose-positive recombinant *Leu. carnosum* of the invention is detected by its ability to utilise lactose as sole carbon source, which is tested by screening for growth of a lactose-positive *Leu. carnosum* colony on a lactose minimal medium. A selective screening medium for detecting said colonies includes the chemically defined growth-medium supplemented with lactose of Example 3 and the steps for performing the screening are as described in Example 4.

The lac+ recombinant *Leu. carnosum* cells can be distinguished from other cultures in a milk product on the basis of their bacteriocin production. Lac+ recombinant *Leu. carnosum* strains may be further identified on the basis of their specific phenotypic properties including: vancomycin resistance; growth characteristics of catalase-negative lactic acid bacteria including growth at low temperatures (1-5°C); production of dextran from sucrose; and an ability to ferment fructose and trehalose apart from sucrose and lactose, but unable to ferment arabinose, arbutin, maltose, raffinose and xylose.

## Claims

1. Use of a recombinant lactose-positive *Leuconostoc carnosum* strain comprising one or more heterologous genes encoding:
- a lactose transporter and
- a β-galactosidase or β-glucosidase
in the production of a milk product.

2. Use of a recombinant *Leuconostoc carnosum* strain according to claim 1, wherein said milk product is selected from the group consisting of cheese, butter milk, sour cream, yoghurt and kefir.

3. Use of a recombinant lactose-positive *Leuconostoc carnosum* strain according to claim 1 or 2, wherein said strain is a mutant and the product of mutagenesis of one or more native gene of the *Leuconostoc carnosum* strain.

4. Use of a recombinant lactose-positive *Leuconostoc carnosum* strain according to claim 1, wherein said:
- lactose transporter and
- β-galactosidase or β-glucosidase
are encoded by heterologous genes stably inherited in the *Leuconostoc carnosum* strain.

5. Use of a recombinant lactose-positive *Leuconostoc carnosum* strain according to claim 4, wherein said β-galactosidase, is a polypeptide having an amino acid sequence of SEQ ID NO: 3, or a fragment thereof, conferring functional β-galactosidase, activity.

6. Use of a recombinant lactose-positive *Leuconostoc carnosum* strain according to claim 5, wherein said β-galactosidase, has the amino acid sequence of SEQ ID NO: 3.

7. Use of a recombinant lactose-positive *Leuconostoc carnosum* strain according to claim 4, wherein said β-galactosidase, comprises an L and an M polypeptide having an amino acid sequence of SEQ ID NO: 13 and 15, respectively, or a fragment thereof, and conferring functional β-galactosidase activity.

8. Use of a recombinant lactose-positive *Leuconostoc carnosum* strain according to claim 4, wherein said β-galactosidase comprises an L and an M polypeptide having the amino acid sequence of SEQ ID NO: 13 and 15, respectively.

9. Use of a recombinant lactose-positive *Leuconostoc carnosum* strain according to claim 5 or 6, wherein said β-galactosidase, is encoded by a nucleic acid molecule that hybridises to a nucleic acid molecule with a nucleotide sequence consisting of SEQ ID NO: 4, under washing stringency conditions of no less than 1xSSC at 65°C.

10. Use of a recombinant lactose-positive *Leuconostoc carnosum* strain according to claim 5 or 6, wherein said nucleic acid molecule has the nucleotide sequence of SEQ ID NO: 1 or 4.

11. Use of a recombinant lactose-positive *Leuconostoc carnosum* strain according to claim 7 or 8, wherein said L and M polypeptide are encoded by a nucleic acid molecule that hybridises to a nucleic acid molecule with a nucleotide sequence consisting of SEQ ID NO: 12, under washing stringency conditions of no less than 1xSSC at 65°C.

12. Use of a recombinant lactose-positive *Leuconostoc carnosum* strain according to claim 11, wherein said said nucleic acid molecule has the nucleotide sequence of SEQ ID NO: 12.

13. Use of a recombinant lactose-positive *Leuconostoc carnosum* strain according to claim 4, wherein said lactose transporter is a polypeptide having an amino acid sequence of SEQ ID NO: 2, or a fragment thereof, conferring functional lactose transporter activity.

14. Use of a recombinant lactose-positive *Leuconostoc carnosum* strain according to claim 4, wherein said lactose transporter is a polypeptide having an amino acid sequence of SEQ ID NO: 11, or a fragment thereof, conferring functional lactose transporter activity.

15. Use of a recombinant lactose-positive *Leuconostoc carnosum* strain according to claim 13, wherein said lactose transporter is encoded by a nucleic acid molecule that hybridises to a nucleic acid molecule with a nucleotide sequence consisting of SEQ ID NO: 6, under washing stringency conditions of no less than 1xSSC at 65°C.

16. Use of a recombinant lactose-positive *Leuconostoc carnosum* strain according to claim 13, wherein said lactose transporter is encoded by a nucleic acid molecule that has the nucleotide sequence of SEQ ID NO: 1 or 6.

17. Use of a recombinant lactose-positive *Leuconostoc carnosum* strain according to claim 14, wherein said lactose transporter is encoded by a nucleic acid molecule that hybridises to a nucleic acid molecule with a nucleotide sequence consisting of SEQ ID NO: 10, under washing stringency conditions of no less than 1xSSC at 65°C.

18. Use of a recombinant lactose-positive *Leuconostoc carnosum* strain according to claim 17, wherein said nucleic acid molecule has the nucleotide sequence of SEQ ID NO: 10.

19. Use of a recombinant lactose-positive *Leuconostoc carnosum* strain according to any one of claims 9-12 and 15-18, wherein said nucleic acid molecule is harboured on a self-replicating plasmid.

20. Use of a recombinant lactose-positive *Leuconostoc carnosum* strain according to claim 19, wherein said plasmid is selected from the group consisting of pCI372, pGhost⁺8, and pCI3340.

21. Use of a recombinant lactose-positive *Leuconostoc carnosum* strain according to any one of claims 9-12 and 15-18, wherein said nucleic acid molecule is integrated into the genome of said strain, said genome including a bacterial chromosome and/or a native plasmid.

22. Use of a recombinant lactose-positive *Leuconostoc carnosum* strain according to any one of claims 9-12 and 15-18, wherein said nucleic acid molecule is comprised within an operon.

23. Use of a recombinant lactose-positive *Leuconostoc carnosum* strain according to any one of claims 9-12, 15-18 and 22, wherein said nucleic acid molecule or operon is operably linked to a homologous or heterologous promoter.

24. Use of a recombinant lactose-positive *Leuconostoc carnosum* strain according to any one of claims 1 to 23, wherein said strain is selected on a chemically defined medium, and further supplemented with lactose, wherein the composition of 1 liter of said medium is:
| | | | |
|---|---|---|---|
| **Amino acids** | **(g)** | **Vitamins (g)** | **(g)** |
| L-alanine | 0.6 | Nicotinic acid | 0.002 |
| L-glutamate | 0.6 | Thiamin | 0.002 |
| Proline | 0.6 | PyridoxinHCl | 0.004 |
| L-serine | 0.6 | Ca Pantothenate | 0.002 |
| L-arginine | 0.4 | Biotin | 0.01 |
| Glycine | 0.4 | Folic acid | 0.002 |
| L-lysine | 0.4 | Riboflavin | 0.002 |
| L-phenylalanine | 0.4 | | |
| L-threonine | 0.4 | **Salts** | **(g)** |
| L-asparagine | 0.25 | KH₂PO₄ | 12 |
| L-glutamine | 0.25 | K₂HPO₄ | 10 |
| L-isoleucine | 0.25 | NH₄Cl | 1 |
| L-leucine | 0.25 | MgCl₂ | 0.15 |
| L-methionine | 0.25 | CaCl₂ | 0.015 |
| L-tryptophan | 0.25 | FeCl₃ | 0.0033 |
| L-valine | 0.25 | K₂SO₄ 0 | 0.095 |
| L-cystein | 0.2 | | |
| L-histidine | 0.1 | **Micronutrients:** | **(nmol)** |
| | | (NH₄)₆Mo₇O₂₄ | 6 |
| L-tyrosine | 0.1 | H₃BO₄ | 800 |
| **Nucleobases:** | **(g)** | CoCl₂ | 60 |
| Adenine | 0.02 | CuSO₄ | 20 |
| Guanine | 0.02 | MnCl₂ | 160 |
| Uracil | 0.02 | ZnSO₄ | 20 |
| Hypoxanthine | 0.02 | | |

25. Use of a recombinant lactose-positive *Leuconostoc carnosum* strain according to any one of claims 19-21, wherein said strain is sensitive to an antibiotic selected from the group consisting of penicillin, chloramphenicol, tetracycline, erythromycine and kanamycin.

26. A recombinant lactose-positive *Leuconostoc carnosum* strain comprising one or more heterologous genes encoding a lactose transporter and - a β-galactosidase or β-glucosidase.

27. A recombinant lactose-positive *Leuconostoc carnosum* strain according to claim 26, wherein,
said genes are stably inherited in said *Leuconostoc carnosum* strain, wherein said lactose transporter is encoded by:
a nucleic acid molecule that hybridises to a nucleic acid molecule with a nucleotide sequence consisting of SEQ ID NO: 6 or 10, under washing stringency conditions of no less than 1xSSC at 65°C, or
a nucleic acid molecule having the nucleotide sequence of SEQ ID NO: 1, 6 or 10 and
wherein said β-galactosidase is encoded by:
a nucleic acid molecule that hybridises to a nucleic acid molecule with a nucleotide sequence consisting of SEQ ID NO: 4 or 12, under washing stringency conditions of no less than 1xSSC at 65°C, or
nucleic acid molecule having the nucleotide sequence of SEQ ID NO: 1, 4 or 12.

28. A recombinant lactose-positive *Leuconostoc carnosum* strain according to claim 26 or 27, wherein said lactose transporter is a polypeptide having an amino acid sequence of SEQ ID NO: 2 or 11, or a fragment thereof, conferring functional lactose transporter activity, and wherein said β-galactosidase is a polypeptide having an amino acid sequence of SEQ ID NO: 3, or
wherein said β-galactosidase comprises an L and an M polypeptide having the amino acid sequence of SEQ ID NO: 13 and 14, respectively or a fragment thereof, conferring functional β-galactosidase activity.

29. A recombinant lactose-positive *Leuconostoc carnosum* strain according to claim 28, wherein said lactose transporter has the amino acid sequence of SEQ ID NO: 2 or 11, and
wherein said β-galactosidase has the amino acid sequence of SEQ ID NO: 3, or
wherein said β-galactosidase comprises an L and an M polypeptide having the amino acid sequence of SEQ ID NO: 13 and 14, respectively.

30. A starter culture for use in the production of a milk product, comprising a recombinant, lactose-positive *Leuconostoc carnosum* strain, wherein said strain comprises one or more heterologous genes encoding:
- a lactose transporter and
- a β-galactosidase or β-glucosidase, and
said starter culture comprising one or more bacterial strains selected from the group consisting of the species *Lactobacillus, Lactococcus, Leuconostoc, Streptococcus* and/or one or more fungal strains selected from the group consisting of the species *Penicillium roqueforti, Penicillium candidum* and *Geotrichum candidum.*

31. Use of a starter culture according to claims 30, wherein said milk product is selected from the group consisting of cheese, butter milk, sour cream, yoghurt and kefir.

32. A milk product comprising a *Leuconostoc carnosum* strain according to claims 29, or a starter culture according to claims 30.

33. A milk product according to claim 32, wherein said milk product is selected from the group consisting of cheese, butter milk, sour cream, yoghurt and kefir.

34. A method for producing a milk product comprising the steps of:
(a) adding a cell culture comprising cells of a *Leuconostoc carnosum* strain according to claim 29, or a starter culture according to claim 30, to a volume of milk,
(b) incubating the product of (a) to form a cheese milk,
(c) coagulating the product of (b) to form milk curds,
(d) separating the milk curds from the product of (c).

35. A method of constructing a recombinant *Leuconostoc carnosum* strain according to any one of claims 1-29, comprising the steps of:
(a) transforming *Leuconostoc carnosum* with at least one nucleic acid molecule encoding a lactose transporter and a β-galactosidase, and
(b) selecting transformed cells of *Leuconostoc carnosum* of step (a) **characterised by** the ability to grow on lactose as sole carbon source.

## Patentansprüche

1. Verwendung eines rekombinanten, lactosepositiven *Leuconostoc carnosum*-Stamms umfassend ein heterologes Gen oder mehrere heterologe Gene, welche für:
- einen Lactosetransporter und
- eine β-Galactosidase oder β-Glucosidase
bei der Herstellung eines Milchproduktes kodieren.

2. Verwendung eines rekombinanten, lactosepositiven *Leuconostoc carnosum*-Stamms nach Anspruch 1, wobei das Milchprodukt aus der Gruppe bestehend aus Käse, Buttermilch, Sauerrahm, Joghurt und Kefir ausgewählt ist.

3. Verwendung eines rekombinanten, lactosepositiven *Leuconostoc carnosum*-Stamms nach Anspruch 1 oder 2, wobei der Stamm ein Mutant ist und das Mutageneseprodukt von einem Gen oder mehreren nativen Genen des *Leuconostoc carnosum*-Stamms ist.

4. Verwendung eines rekombinanten, lactosepositiven *Leuconostoc carnosum*-Stamms nach Anspruch 1, wobei:
- der Lactosetransporter und
- die β-Galactosidase oder β-Glucosidase
von heterologen Genen kodiert sind, welche im *Leuconostoc carnosum-*Stamm stabil geerbt sind.

5. Verwendung eines rekombinanten, lactosepositiven *Leuconostoc carnosum*-Stamms nach Anspruch 4, wobei die β-Galactosidase ein Polypeptid ist, welches eine Aminosäuresequenz von SEQ ID NO: 3 oder ein Fragment davon aufweist und eine funktionelle β-Galactosidaseaktivität konferiert.

6. Verwendung eines rekombinanten, lactosepositiven *Leuconostoc carnosum*-Stamms nach Anspruch 5, wobei die β-Galactosidase die Aminosäuresequenz von SEQ ID NO: 3 aufweist.

7. Verwendung eines rekombinanten, lactosepositiven *Leuconostoc carnosum*-Stamms nach Anspruch 4, wobei die β-Galactosidase ein L- und ein M-Polypeptid umfasst, welche eine Aminosäuresequenz von SEQ ID NO: 13 bzw. 15 oder ein Fragment davon aufweisen und eine funktionelle β-Galactosidaseaktivität konferiert.

8. Verwendung eines rekombinanten, lactosepositiven *Leuconostoc carnosum*-Stamms nach Anspruch 4, wobei die β-Galactosidase ein L- und ein M-Polypeptid umfasst, welche die Aminosäuresequenz von SEQ ID NO: 13 bzw. 15 aufweisen.

9. Verwendung eines rekombinanten, lactosepositiven *Leuconostoc carnosum*-Stamms nach Anspruch 5 oder 6, wobei die β-Galactosidase durch ein Nukleinsäuremolekül kodiert ist, welches zu einem Nukleinsäuremolekül mit einer Nukleotidsequenz bestehend aus SEQ ID NO: 4, unter stringenten Waschbedingungen von mindestens 1xSSC bei 65°C, hybridisiert.

10. Verwendung eines rekombinanten, lactosepositiven *Leuconostoc carnosum*-Stamms nach Anspruch 5 oder 6, wobei das Nukleinsäuremolekül die Nukleotidsequenz von SEQ ID NO: 1 oder 4 aufweist.

11. Verwendung eines rekombinanten, lactosepositiven *Leuconostoc carnosum*-Stamms nach Anspruch 7 oder 8, wobei das L- und das M-Polypeptid durch ein Nukleinsäuremolekül kodiert sind, welches zu einem Nukleinsäuremolekül mit einer Nukleotidsequenz bestehend aus SEQ ID NO: 12, unter stringenten Waschbedingungen von mindestens 1xSSC bei 65°C, hybridisiert.

12. Verwendung eines rekombinanten, lactosepositiven *Leuconostoc carnosum*-Stamms nach Anspruch 11, wobei das Nukleinsäuremolekül die Nukleotidsequenz von SEQ ID NO: 12 aufweist.

13. Verwendung eines rekombinanten, lactosepositiven *Leuconostoc carnosum*-Stamms nach Anspruch 4, wobei der Lactosetransporter ein Polypeptid ist, welches eine Aminosäuresequenz von SEQ ID NO: 2 oder ein Fragment davon aufweist und eine funktionelle Lactosetransporteraktivität konferiert.

14. Verwendung eines rekombinanten, lactosepositiven *Leuconostoc carnosum*-Stamms nach Anspruch 4, wobei der Lactosetransporter ein Polypeptid ist, welches eine Aminosäuresequenz von SEQ ID NO: 11 oder ein Fragment davon aufweist und eine funktionelle Lactosetransporteraktivität konferiert.

15. Verwendung eines rekombinanten, lactosepositiven *Leuconostoc carnosum*-Stamms nach Anspruch 13, wobei der Lactosetransporter durch ein Nukleinsäuremolekül kodiert ist, welches zu einem Nukleinsäuremolekül mit einer Nukleotidsequenz bestehend aus SEQ ID NO: 6, unter stringenten Waschbedingungen von mindestens 1xSSC bei 65°, hybridisiert.

16. Verwendung eines rekombinanten, lactosepositiven *Leuconostoc carnosum*-Stamms nach Anspruch 13, wobei der Lactosetransporter durch ein-Nukleinsäuremolekül kodiert ist, welches die Nukleinsäuresequenz von SEQ ID NO: 1 oder 6 aufweist.

17. Verwendung eines rekombinanten, lactosepositiven *Leuconostoc carnosum*-Stamms nach Anspruch 14, wobei der Lactosetransporter durch ein Nukleinsäuremolekül kodiert ist, welches zu einem Nukleinsäuremolekül mit einer Nukleotidsequenz bestehend aus SEQ ID NO: 10, unter stringenten Waschbedingungen von mindestens 1xSSC bei 65°C, hybridisiert.

18. Verwendung eines rekombinanten, lactosepositiven *Leuconostoc carnosum*-Stamms nach Anspruch 17, wobei das Nukleinsäuremolekül die Nukleotidsequenz von SEQ ID NO: 10 aufweist.

19. Verwendung eines rekombinanten, lactosepositiven *Leuconostoc carnosum*-Stamms nach einem der Ansprüche 9 bis 12 und 15 bis 18, wobei das Nukleinsäuremolekül auf einem selbstreplizierenden Plasmid beherbergt ist.

20. Verwendung eines rekombinanten, lactosepositiven *Leuconostoc carnosum*-Stamms nach Anspruch 19, wobei das Plasmid aus der Gruppe bestehend aus pC1372, pGhost⁺8 und pCI3340 ausgewählt ist.

21. Verwendung eines rekombinanten, lactosepositiven *Leuconostoc carnosum*-Stamms nach einem der Ansprüche 9 bis 12 und 15 bis 18, wobei das Nukleinsäuremolekül in dem Genom des Stamms integriert ist, welches Genom ein baktierielles Chromosom und/oder ein natives Plasmid umfasst.

22. Verwendung eines rekombinanten, lactosepositiven *Leuconostoc carnosum*-Stamms nach einem der Ansprüche 9 bis 12 und 15 bis 18, wobei das Nukleinsäuremolekül innerhalb eines Operons beinhaltet ist.

23. Verwendung eines rekombinanten, lactosepositiven *Leuconostoc carnosum*-Stamms nach einem der Ansprüche 9 bis 12, 15 bis 18 und 22, wobei das Nukleinsäuremolekül mit einem homologen oder heterologen Promotor operabel verbunden ist.

24. Verwendung eines rekombinanten, lactosepositiven *Leuconostoc carnosum*-Stamms nach einem der Ansprüche 1 bis 23, wobei der Stamm auf einem chemisch definierten Medium ausgewählt ist und ferner mit Lactose ergänzt ist, wobei die Zusammensetzung von 1 Liter des Mediums ist wie folgt:
| | | | |
|---|---|---|---|
| **Aminosäuren** | **(g)** | **Vitamine** | **(g)** |
| L-Alanin | 0,6 | Nikotinsäure | 0,002 |
| L-Glutamat | 0,6 | Thiamin | 0,002 |
| Prolin | 0,6 | PyridoxinHCl | 0,004 |
| L-Serin | 0,6 | Ca Pantothenat | 0,002 |
| L-Arginin | 0,4 | Biotin | 0,01 |
| Glycin | 0,4 | Folsäure | 0,002 |
| L-Lysin | 0,4 | Riboflavin | 0,002 |
| L-Phenylalanin | 0,4 | | |
| L-Threonin | 0,4 | **Sälze** | **(g)** |
| L-Asparagin | 0,25 | KH₂PO₄ | 12 |
| L-Glutamin | 0,25 | K₂HPO₄ | 10 |
| L-Isoloeucin | 0,25 | NH₄Cl | 1 |
| L-Leucin | 0,25 | MgCl₂ | 0,15 |
| L-Methionin | 0,25 | CaCl₂ | 0,015 |
| L-Tryptophan | 0,25 | FeCl₃ | 0,0033 |
| L-Valin | 0,25 | K₂SO₄ | 0,095 |
| L-Cystein | 0,2 | | |
| L-Histidin | 0,1 | **Mikronährstoffe:** | **(nmol)** |
| L-Tyrosin | 0,1 | (Nh₄)₆Mo₇O₂₄ | 6 |
| | | H₃BO₄ | 800 |
| **Nukleobasen** | | CoCl₂ | 60 |
| Adenin | 0,02 | CuSO₄ | 20 |
| Guanin | 0,02 | MnCl₂ | 160 |
| Uracil | 0,02 | ZnSO₄ | 20 |
| Hypoxanthin | 0,02 | | |

25. Verwendung eines rekombinanten, lactosepositiven *Leuconostoc carnosum*-Stamms nach einem der Ansprüche 19 bis 21, wobei der Stamm gegen ein Antibiotikum, welches aus der Gruppe bestehend aus Penicillin, Chloramphenicol, Tetracyclin, Erythromycin und Kanamycin ausgewählt ist, empfindlich ist.

26. Rekombinanter, lactosepositiver *Leuconostoc carnosum-Stamm,* welcher ein heterologes Gen oder mehrere heterologe Gene umfasst, welche für einen Lactosetransporter und eine eine β-Galactosidase oder β-Glucosidase kodiert.

27. Rekombinanter, lactosepositiver *Leuconostoc carnosum-Stamm* nach Anspruch 26, wobei
die Gene im *Leuconostoc carnosum-Stamm* stabil geerbt sind,
wobei der Lactosetransporter kodiert ist durch:
ein Nukleinsäuremolekül, welces zu einem Nukleinsäuremolekül mit einer Nukleotidsequenz bestehend aus SEQ ID NO: 6 oder 10, unter stringenten Waschbedingungen von mindestens 1xSSC bei 65°C, hybridisiert oder ein Nukleinsäuremolekül, welches die Nukleotidsequenz von SEQ ID NO: 1, 6 oder 10 aufweist, und
wobei die β-Galactosidase kodiert ist durch:
ein Nukleinsäuremolekül, welches zu einem Nukleinsäuremolekül mit einer Nukleotidsequenz bestehend aus SEQ ID NO: 4 oder 12, unter stringenten Waschbedingungen von mindestens 1xSSC bei 65°C, hybridisiert oder
ein Nukleinsäuremolekül, welches die Nukleotidsequenz von SEQ ID NO: 1, 4 oder 12 aufweist.

28. Rekombinanter, lactosepositiver *Leuconostoc carnosum-Stamm* nach Anspruch 26 oder 27, wobei der Lactosetransporter ein Polypeptid ist, welches eine Aminosäuresequenz von SEQ ID NO: 2 oder 11 oder ein Fragment davon aufweist und eine funktionelle Lactsetransporteraktivität konferiert, und
wobei die β-Galactosidase ein Polypeptid ist, welches eine Aminosäuresequenz von SEQ ID NO: 3 aufweist, oder
wobei die β-Galactosidase ein L- und ein M-Polypeptid umfasst, welche die Aminosäuresequenz von SEQ ID NO: 13 bzw. 14 oder ein Fragment davon aufweisen und eine funktionelle β-Galactosidaseaktivität konferiert.

29. Rekombinanter, lactosepositiver *Leuconostoc carnosum-Stamm* nach Anspruch 28, wobei der Lactosetransporter die Aminosäuresequenz von SEQ ID NO: 2 oder 11 aufweist, und
wobei die β-Galactosidase die Aminosäuresequenz von SEQ ID NO: 3 aufweist, oder
wobei die β-Galactosidase ein L- und ein M-Polypeptid umfasst, welche die Aminosäuresequenz von SEQ ID NO: 13 bzw. 14 aufweisen.

30. Starterkultur zur Verwendung bei der Herstellung eines Milchproduktes, umfassend einen rekombinanten, lactosepositiven *Leuconostoc carnosum-*Stamm, wobei der Stamm ein heterologes Gen oder mehrere heterologe Gene umfasst, welche für
- einen Lactosetransporter und
- eine β-Galactosidase oder β-Glucosidase kodiert, und
die Starterkultur einen bakteriellen Stamm oder mehrere bakterielle Stämme, welche aus der Gruppe bestehend aus der Gattung *Lactobacillus, Lactococcus, Leuconostoc, Streptococcus* ausgewählt sind, und/oder einen fungalen Stamm oder mehrere fungale Stämme, welche aus der Gruppe bestehend aus der Gattung *Penicillium roqueforti, Penicillum candidum* und *Geotrichum candidum* ausgewählt sind, umfasst.

31. Verwendung einer Starterkultur nach Anspruch 30, wobei das Milchprodukt aus der Gruppe bestehend aus Käse, Buttermilch, Sauerrahm, Joghurt und Kefir ausgewählt ist.

32. Milchprodukt welches einen *Leuconostoc carnosum-Stamm* nach Anspruch 29 oder eine Starterkultur nach Anspruch 30 umfasst.

33. Milchpodukt nach Anspruch 32, wobei das Milchprodukt aus der Gruppe bestehend aus Käse, Buttermilch, Sauerrahm, Joghurt und Kefir ausgewählt ist.

34. Verfahren zur Herstellung eines Milchproduktes umfassend die folgenden Schritte:
(a) Hinzufügen einer Zellenkultur umfassend Zellen eines *Leuconostoc carnosum*-Stamms nach Anspruch 29 oder einer Starterkultur nach Anspruch 30 zu einer Menge von Milch,
(b) Inkubieren des Produktes von (a) zur Bildung von Käsemilch,
(c) Koagulieren des Produktes von (b) zur Bildung von Milchkäsebruch,
(d) Abtrennen des Milchkäsebruchs vom Produkt von (c).

35. Verfahren zur Bildung eines rekombinanten *Leuconostoc carnosum-*Stamms nach einem der Ansprüche 1 bis 29, umfassend die folgenden Schritte:
(a) Transformieren von *Leuconostoc carnosum* mit mindestens einem Nukleinsäuremolekül, welches für einen Lactosetransporter und eine β-Galactosidase kodiert, und
(b) Auswählen transformierter Zellen von *Leuconostoc carnosum* aus dem Schritt (a), welche durch die Fähigkeit, auf Lactose als einziger Kohlenstoffquelle zu wachsen, charakterisiert sind.

## Revendications

1. Usage d'une souche de *Leuconostoc carnosum* de recombinaison lactose positive comprenant un ou plusieurs gènes hétérologues codant:
- un transporteur de lactose et
- une β-galactosidase ou une β-glucosidase
dans la production d'un produit laitier.

2. Usage d'une souche de *Leuconostoc carnosum* de recombinaison selon la revendication 1, où ledit produit laitier est sélectionné du groupe composé du fromage, du lait de beurre, de la crème aigre, du yaourt et du képhir.

3. Usage d'une souche de *Leuconostoc carnosum* de recombinaison lactose positive selon la revendication 1 ou 2, où ladite souche est une mutante et le produit de la mutagenèse de l'un ou plusieurs gènes natifs de la souche de *Leuconostoc carnosum.*

4. Usage d'une souche de *Leuconostoc carnosum* de recombinaison lactose positive selon la revendication 1, où lesdits:
- transporteur de lactose et
- β-galactosidase ou β-glucosidase
sont codés par des gènes hétérologues stablement hérités dans la souche de *Leuconostoc carnosum.*

5. Usage d'une souche de *Leuconostoc carnosum* de recombinaison lactose positive selon la revendication 4, où ladite β-galactosidase est un polypeptide ayant une séquence d'acide aminé de SEQ ID NO: 3, ou un fragment de celui-ci, conférant l'activité fonctionnelle de la β-galactosidase.

6. Usage d'une souche de *Leuconostoc carnosum* de recombinaison lactose positive selon la revendication 5, où ladite β-galactosidase présente la séquence d'acide aminé de SEQ ID NO: 3.

7. Usage d'une souche de *Leuconostoc carnosum* de recombinaison lactose positive selon la revendication 4, où ladite β-galactosidase comprend un polypeptide de L et de M ayant une séquence d'acide aminé de SEQ ID NO: 13 et 15 respectivement, ou un fragment de celui-ci, et conférant l'activité fonctionnelle de la β-galactosidase.

8. Usage d'une souche de *Leuconostoc carnosum* de recombinaison lactose positive selon la revendication 4, où ladite β-galactosidase comprend un polypeptide de L et de M ayant la séquence d'acide aminé de SEQ ID NO: 13 et 15, respectivement.

9. Usage d'une souche de *Leuconostoc carnosum* de recombinaison lactose positive selon la revendication 5 ou 6, où ladite β-galactosidase est codée par une molécule d'acide nucléique qui s'hybride à une molécule d'acide nucléique avec une séquence nucléotidique composée de SEQ ID NO: 4, sous les conditions de stringence de lavage qui ne sont pas moins de 1xSSC à 65°C.

10. Usage d'une souche de *Leuconostoc carnosum* de recombinaison lactose positive selon la revendication 5 ou 6, où ladite molécule d'acide nucléique présente la séquence nucléotidique de SEQ ID NO: 1 ou 4.

11. Usage d'une souche de *Leuconostoc carnosum* de recombinaison lactose positive selon la revendication 7 ou 8, où ledit polypeptide de L et de M est codé par une molécule d'acide nucléique qui s'hybride à une molécule d'acide nucléique avec une séquence nucléotidique composée de SEQ ID NO: 12, sous les conditions de stringence de lavage qui ne sont pas moins de 1xSSC à 65°C.

12. Usage d'une souche de *Leuconostoc carnosum* de recombinaison lactose positive selon la revendication 11, où ladite molécule d'acide nucléique présente la séquence nucléotidique de SEQ ID NO: 12.

13. Usage d'une souche de *Leuconostoc carnosum* de recombinaison lactose positive selon la revendication 4, où ledit transporteur de lactose est un polypeptide ayant une séquence d'acide aminé de SEQ ID NO: 2, ou un fragment de celui-ci, conférant l'activité fonctionnelle de transporteur de lactose.

14. Usage d'une souche de *Leuconostoc carnosum* de recombinaison lactose positive selon la revendication 4, où ledit transporteur de lactose est un polypeptide ayant une séquence d'acide aminé de SEQ ID NO: 11, ou un fragment de celui-ci, conférant l'activité fonctionnelle de transporteur de lactose.

15. Usage d'une souche de *Leuconostoc carnosum* de recombinaison lactose positive selon la revendication 13, où ledit transporteur de lactose est codé par une molécule d'acide nucléique qui s'hybride à une molécule d'acide nucléique avec une séquence nucléotidique composée de SEQ ID NO: 6, sous les conditions de stringence de lavage qui ne sont pas moins de 1xSSC à 65°C.

16. Usage d'une souche de *Leuconostoc carnosum* de recombinaison lactose positive selon la revendication 13, où ledit transporteur de lactose est codé par une molécule d'acide nucléique qui présente la séquence nucléotidique de SEQ ID NO: 1 ou 6.

17. Usage d'une souche de *Leuconostoc carnosum* de recombinaison lactose positive selon la revendication 14, où ledit transporteur de lactose est codé par une molécule d'acide nucléique qui s'hybride à une molécule d'acide nucléique avec une séquence nucléotidique composée de SEQ ID NO: 10, sous les conditions de stringence de lavage qui ne sont pas moins de 1xSSC à 65°C.

18. Usage d'une souche de *Leuconostoc carnosum* de recombinaison lactose positive selon la revendication 17, où ladite molécule d'acide nucléique présente la séquence nucléotidique de SEQ ID NO: 10.

19. Usage d'une souche de *Leuconostoc carnosum* de recombinaison lactose positive selon l'une quelconque des revendications 9 à 12 et 15 à 18, où ladite molécule d'acide nucléique est hébergée sur un plasmide autoréplicatif.

20. Usage d'une souche de *Leuconostoc carnosum* de recombinaison lactose positive selon la revendication 19, où ledit plasmide est sélectionné du groupe se composant de pC1372, de pGhost⁺8 et de pC13340.

21. Usage d'une souche de *Leuconostoc carnosum* de recombinaison lactose positive selon l'une quelconque des revendications 9 à 12 et 15 à 18, où ladite molécule d'acide nucléique est intégrée dans le génome de ladite souche, ledit génome comprenant un chromosome bactérien et/ou un plasmide natif.

22. Usage d'une souche de *Leuconostoc carnosum* de recombinaison lactose positive selon l'une quelconque des revendications 9 à 12 et 15 à 18, où ladite molécule d'acide nucléique est comprise dans un opéron.

23. Usage d'une souche de *Leuconostoc carnosum* de recombinaison lactose positive selon l'une quelconque des revendications 9 à 12, 15 à 18 et 22, où ladite molécule d'acide nucléique ou ledit opéron est fonctionnellement lié à un promoteur homologue ou hétérologue.

24. Usage d'une souche de *Leuconostoc carnosum* de recombinaison lactose positive selon l'une quelconque des revendications 1 à 23, où ladite souche est sélectionnée sur un support chimiquement défini, et en outre complétée avec le lactose, où la composition de 1 litre dudit support est :
| | | | |
|---|---|---|---|
| **Acides aminés** | **(g)** | **Vitamines** | **(g)** |
| L-alanine | 0,6 | Acide nicotinique | 0,002 |
| L-glutamate | 0,6 | Thiamine | 0,002 |
| Proline | 0,6 | Pyridoxine HCl | 0,004 |
| L-serine | 0,6 | Ca-Pantothénate | 0,002 |
| L-arginine | 0,4 | Biotine | 0,01 |
| Glycine | 0,4 | Acide folique | 0,002 |
| L-lysine | 0,4 | Riboflavine | 0,002 |
| L-phenylalanine | 0,4 | | |
| L-threonine | 0,4 | **Sels** | **(g)** |
| L-asparagine | 0,25 | KH₂PO₄ | 12 |
| L-glutamine | 0,25 | K₂HPO₄ | 10 |
| L-isoloeucine | 0,25 | NH₄Cl | 1 |
| L-leucine | 0,25 | MgCl₂ | 0,15 |
| L-methionine | 0,25 | CaCl₂ | 0,015 |
| L-tryptophane | 0,25 | FeCl₃ | 0,0033 |
| L-valine | 0,25 | K₂SO₄ | 0,095 |
| L-cysteine | 0,2 | | |
| L-histidine | 0,1 | **Micronutriments:** | **(nmol)** |
| L-tyrosine | 0,1 | (NH₄)₆Mo₇O₂₄ | 6 |
| | | H₃BO₄ | 800 |
| **Nucléobases** | **(g)** | CoCl₂ | 60 |
| Adenine | 0,02 | CuSO₄ | 20 |
| Guanine | 0,02 | MnCl₂ | 160 |
| Uracile | 0,02 | ZnSO₄ | 20 |
| Hypoxanthine | 0,02 | | |

25. Usage d'une souche de *Leuconostoc carnosum* de recombinaison lactose positive selon l'une quelconque des revendications 19 à 21, où ladite souche est sensible à un antibiotique sélectionné du groupe composé de la pénicilline, du chloramphénicol, de la tétracycline, de l'érythromycine et de la kanamycine.

26. Souche de *Leuconostoc carnosum* de recombinaison lactose positive comprenant un ou plusieurs gènes hétérologues codant un transporteur de lactose et une β-galactosidase ou une β-glucosidase.

27. Souche de *Leuconostoc carnosum* de recombinaison lactose positive selon la revendication 26, où
lesdits gènes sont stablement hérités dans la souche de *Leuconostoc carnosum,*
où ledit transporteur de lactose est codé par :
une molécule d'acide nucléique qui s'hybride à une molécule d'acide nucléique avec une séquence nucléotidique composée de SEQ ID NO: 6 ou 10,
sous les conditions de stringence de lavage qui ne sont pas moins de 1xSSC à 65°C, ou
une molécule d'acide nucléique avec une séquence nucléotidique de SEQ ID NO: 1, 6 ou 10 et
où la β-galactosidase est codée par:
une molécule d'acide nucléique qui s'hybride à une molécule d'acide nucléique avec une séquence nucléotidique composée de SEQ ID NO: 4 ou 12, sous les conditions de stringence de lavage qui ne sont pas moins de 1xSSC à 65°C, ou
une molécule d'acide nucléique avec une séquence nucléotidique de SEQ ID NO: 1, 4 ou 12.

28. Souche de *Leuconostoc carnosum* de recombinaison lactose positive selon la revendication 26 ou 27, où ledit transporteur de lactose est un polypeptide ayant une séquence d'acide aminé de SEQ ID NO: 2 ou 11, ou un fragment de celui-ci, conférant l'activité fonctionnelle de transporteur de lactose, et où ladite β-galactosidase est un polypeptide ayant une séquence d'acide aminé de SEQ ID NO: 3, ou
où ladite β-galactosidase comprend un polypeptide de L et de M ayant la séquence d'acide aminé de SEQ ID NO: 13 et 14, respectivement, ou un fragment de celui-ci, conférant l'activité fonctionnelle de la β-galactosidase.

29. Souche de *Leuconostoc carnosum* de recombinaison lactose positive selon la revendication 28, où ledit transporteur de lactose présente la séquence d'acide aminé de SEQ ID NO: 2 ou 11, et
où ladite β-galactosidase présente une séquence d'acide aminé de SEQ ID NO: 3, ou
où ladite β-galactosidase présente un polypeptide de L et de M ayant la séquence d'acide aminé de SEQ ID NO: 13 et 14, respectivement.

30. Culture de starter pour l'usage dans la production d'un produit laitier, comprenant une souche de *Leuconostoc carnosum* de recombinaison lactose positive, où ladite souche comprend un ou plusieurs gènes hétérologues codant:
- un transporteur de lactose et
- une β-galactosidase ou une β-glucosidase, et
ladite culture de starter comprenant une ou plusieurs souches bactériennes choisies parmi le groupe composé des espèces de *Lactobacillus, Lactococcus, Leuconostoc, Streptococcus* et/ou une ou plusieurs souches fongiques choisies parmi le groupe composé des espèces de *Penicilium roqueforti, Penicillium candidum* et *Geotrichum candidum.*

31. Usage d'une culture de starter selon la revendication 30, où ledit produit laitier est choisi parmi le groupe composé du fromage, du lait de beurre, de la crème aigre, du yaourt et du képhir.

32. Produit laitier comprenant une souche de *Leuconostoc carnosum* selon la revendication 29, ou une culture de starter selon la revendication 30.

33. Produit laitier selon la revendication 32, où ledit produit laitier est choisi du groupe composé du fromage, du lait de beurre, de la crème aigre, du yaourt et du képhir.

34. Procédé pour la production d'un produit laitier comprenant les étapes de :
(a) ajoutant une culture de cellule comprenant des cellules d'une souche de *Leuconostoc camosum* selon la revendication 29, ou une culture de starter selon la revendication 30, à un volume de lait,
(b) incubant le produit de (a) pour former un lait de fromage,
(c) coagulant le produit de (b) pour former les caillés de lait,
(d) séparation des caillés de lait du produit de (c).

35. Procédé pour la construction d'une souche de *Leuconostoc carnosum* recombinant selon toutes de revendications 1-29, comprenant les étapes de:
(a) transformer *Leuconostoc carnosum* avec au moins une molécule d'acide nucléique codant un transporteur de lactose et une β-galactosidase, et
(b) sélectionner des cellules transformées de *Leuconostoc carnosum* de l'étape (a) **caractérisées par** la capacité de croître sur le lactose comme la seule source de carbone.


### SEQUENCE LISTING

<110> Technical Univeristy of Denmark
   Ruhdal Jensen, Peter
<120> Lactose positive recombinant Leuconostoc strain
<130> P200401357DK
<160> 15
<170> PatentIn version 3.2
<210> 1
   <211> 5206
   <212> DNA
   <213> Streptococcus thermophilus
<220>
   <221> misc_structure
   <222> (1)..(15)
<220>
   <221> promoter
   <222> (16)..(94)
<220>
   <221> misc_feature
   <222> (18)..(22)
   <223> n is a, c, g, or t
<220>
   <221> misc_feature
   <222> (38)..(51)
   <223> n is a, c, g, or t
<220>
   <221> misc_feature
   <222> (61)..(64)
   <223> n is a, c, g, or t
<220>
   <221> CDS
   <222> (95)..(1996)
   <223> Lac S CDS
<220>
   <221> misc_feature
   <222> (1997)..(2002)
   <223> non CDS
<220>
   <221> CDS
   <222> (2003)..(5080)
   <223> LacZ CDS
<400> 1
   acgactagtg gatccatnnn nnagtttatt cttgacannn nnnnnnnnnn ntgrtataat    60
   nnnnaagtaa taaaatattc ggaggaattt tgaa atg gaa aaa tct aaa ggt cag   115
                                         Met Glu Lys Ser Lys Gly Gln
                                         1               5
   atg aag tct cgt tta tcc tac gca gct ggt gct ttt ggt aac gac gtc     163
   Met Lys Ser Arg Leu Ser Tyr Ala Ala Gly Ala Phe Gly Asn Asp Val
           10                  15                  20
   ttc tat gca acc ttg tca aca tac ttt atc atg ttt gtg aca act cac      211
   Phe Tyr Ala Thr Leu Ser Thr Tyr Phe Ile Met Phe Val Thr Thr His
       25                  30                  35
   ttg ttt aac aca ggt gat cca aag caa aat agt cac tac gta cta tta      259
   Leu Phe Asn Thr Gly Asp Pro Lys Gln Asn Ser His Tyr Val Leu Leu
   40                  45                  50                  55
   atc act aac att atc tct att ttg cgt atc ttg gaa gta ttt atc gat      307
   Ile Thr Asn Ile Ile Ser Ile Leu Arg Ile Leu Glu Val Phe Ile Asp
                   60                  65                  70
   cca ttg atc ggt aat atg att gat aac act aat act aag tat ggt aaa      355
   Pro Leu Ile Gly Asn Met Ile Asp Asn Thr Asn Thr Lys Tyr Gly Lys
               75                  80                  85
   ttc aaa cca tgg gta gtt ggt ggt ggt atc atc agt tct atc acc ttg      403
   Phe Lys Pro Trp Val Val Gly Gly Gly Ile Ile Ser Ser Ile Thr Leu
           90                  95                  100
   ttg ttt ctc tta acc gat ata ggt ggt ttg aat aaa aca aat cct ttc      451
   Leu Phe Leu Leu Thr Asp Ile Gly Gly Leu Asn Lys Thr Asn Pro Phe
       105                 110                 115
   ttg tat ctt gta ctt ttt gga att atc tac ctt gta atg gat gtc ttc      499
   Leu Tyr Leu Val Leu Phe Gly Ile Ile Tyr Leu Val Met Asp Val Phe
   120                 125                 130                 135
   tac tcg att aaa gat atc ggt ttc tgg tca atg att cct gcc ttg tct      547
   Tyr Ser Ile Lys Asp Ile Gly Phe Trp Ser Met Ile Pro Ala Leu Ser
                   140                 145                 150
   ctt gat agt cac gaa cgt gaa aaa atg gca act ttt gcc cgt att ggt      595
   Leu Asp Ser His Glu Arg Glu Lys Met Ala Thr Phe Ala Arg Ile Gly
               155                 160                 165
   tcg acg att ggt gcc aat att gta ggt gtt gcc atc atg cca atc gat      643
   Ser Thr Ile Gly Ala Asn Ile Val Gly Val Ala Ile Met Pro Ile Asp
           170                 175                 180
   ttg tta ttc tct atg acg cac aat agt ggc tct gga gat aaa tct gga      691
   Leu Leu Phe Ser Met Thr His Asn Ser Gly Ser Gly Asp Lys Ser Gly
       185                 190                 195
   tgg ttc tgg tat gca ttt atc gta gat ctc aat ggt gtg att aca tca      739
   Trp Phe Trp Tyr Ala Phe Ile Val Asp Leu Asn Gly Val Ile Thr Ser
   200                 205                 210                 215
   att gct gtt ggt att ggt aca cgt gaa gtt gag tca aaa att cat gat      787
   Ile Ala Val Gly Ile Gly Thr Arg Glu Val Glu Ser Lys Ile His Asp
                   220                 225                 230
   aat aac gaa aaa act agc ctt aaa caa gtc ttt aag gtt ctt ggt caa      835
   Asn Asn Glu Lys Thr Ser Leu Lys Gln Val Phe Lys Val Leu Gly Gln
               235                 240                 245
   aac gac caa atg atg tgg tta tct ctt gga tat tgg ttc tat ggt ctt      883
   Asn Asp Gln Met Met Trp Leu Ser Leu Gly Tyr Trp Phe Tyr Gly Leu
           250                 255                 260
   ggt att aat aca ctt aat gct ctt caa ctt tat tat ttc aca ttt atc      931
   Gly Ile Asn Thr Leu Asn Ala Leu Gln Leu Tyr Tyr Phe Thr Phe Ile
       265                 270                 275
   ctt ggt gat tca ggt aaa tac tca att ctt tac gga ttg aat aca gtt      979
   Leu Gly Asp Ser Gly Lys Tyr Ser Ile Leu Tyr Gly Leu Asn Thr Val
   280                 285                 290                 295
   gtt ggt ttg gtt tca gtt tca ctc ttc cct acc cta gct gat aaa ttc 1027
   Val Gly Leu Val Ser Val Ser Leu Phe Pro Thr Leu Ala Asp Lys Phe
   300 305 310
   aac cgt aaa cgt ttg ttc tac gga tgt att gca gta atg ctc ggg ggt 1075
   Asn Arg Lys Arg Leu Phe Tyr Gly Cys Ile Ala Val Met Leu Gly Gly
   315 320 325
   atc gga ata ttt agt att gca ggt aca tca ctt cca ata atc ttg act 1123
   Ile Gly Ile Phe Ser Ile Ala Gly Thr Ser Leu Pro Ile Ile Leu Thr
   330 335 340
   gca gct gaa ctc ttc ttc att cca caa cct ctt gtg ttc ctt gtt gtc 1171
   Ala Ala Glu Leu Phe Phe Ile Pro Gln Pro Leu Val Phe Leu Val Val
   345 350 355
   ttt atg att atc tct gac tca gta gaa tat ggt caa tgg aaa acg gga 1219
   Phe Met Ile Ile Ser Asp Ser Val Glu Tyr Gly Gln Trp Lys Thr Gly
   360 365 370 375
   cac cgt gat gaa tca ctt act ttg tca gtt cgt cca ctt att gat aaa 1267
   His Arg Asp Glu Ser Leu Thr Leu Ser Val Arg Pro Leu Ile Asp Lys
   380 385 390
   ctt ggt ggt gcg atg tca aac tgg ctt gtt tct aca ttt gcc gta gct 1315
   Leu Gly Gly Ala Met Ser Asn Trp Leu Val Ser Thr Phe Ala Val Ala
   395 400 405
   gcc ggt atg aca aca ggt gcc tca gca tca aca att aca aca cat caa 1363
   Ala Gly Met Thr Thr Gly Ala Ser Ala Ser Thr Ile Thr Thr His Gln
   410 415 420
   cag ttt atc ttt aag ctt ggc atg ttt gct ttc cca gca gca aca atg 1411
   Gln Phe Ile Phe Lys Leu Gly Met Phe Ala Phe Pro Ala Ala Thr Met
   425 430 435
   ctt atc ggt gcc ttc att gtt gct cgt aaa atc act ttg act gaa gca 1459
   Leu Ile Gly Ala Phe Ile Val Ala Arg Lys Ile Thr Leu Thr Glu Ala
   440 445 450 455
   cgt cac gct aaa att gtt gaa gaa ttg gaa cat cgc ttt agc gta gca 1507
   Arg His Ala Lys Ile Val Glu Glu Leu Glu His Arg Phe Ser Val Ala
   460 465 470
   act tct gaa aat gaa gtt aaa gct aac gtc gta tct ctt gta acc cct 1555
   Thr Ser Glu Asn Glu Val Lys Ala Asn Val Val Ser Leu Val Thr Pro
   475 480 485
   aca act ggt tat ttg gtt gat ctc tca agt gtt aat gat gaa cac ttt 1603
   Thr Thr Gly Tyr Leu Val Asp Leu Ser Ser Val Asn Asp Glu His Phe
   490 495 500
   gct tca ggt agc atg ggt aaa ggt ttc gcc att aaa cct act gat gga 1651
   Ala Ser Gly Ser Met Gly Lys Gly Phe Ala Ile Lys Pro Thr Asp Gly
   505 510 515
   gct gtc ttt gca cca att agt ggt acc att cgt caa att ctt cct act 1699
   Ala Val Phe Ala Pro Ile Ser Gly Thr Ile Arg Gln Ile Leu Pro Thr
   520 525 530 535
   cgc cat gca gtt ggt att gaa agt gaa gat ggt gtc att gtt ctt atc 1747
   Arg His Ala Val Gly Ile Glu Ser Glu Asp Gly Val Ile Val Leu Ile
   540 545 550
   cac gtt ggc atc gga aca gtt aaa ctt aat ggt gaa gga ttc att agt 1795
   His Val Gly Ile Gly Thr Val Lys Leu Asn Gly Glu Gly Phe Ile Ser
   555 560 565
   tac gta gaa caa ggt gat cat gtt gaa gtt gga caa aaa ctt ctt gag      1843
   Tyr Val Glu Gln Gly Asp His Val Glu Val Gly Gln Lys Leu Leu Glu
           570                 575                 580
   ttc tgg tca cca att att gag aaa aat ggt ctt gat gac aca gta ctt      1891
   Phe Trp Ser Pro Ile Ile Glu Lys Asn Gly Leu Asp Asp Thr Val Leu
       585                 590                 595
   gtc act gta act aat tca gaa aaa ttc agt gct ttc cat ctt gaa caa      1939
   Val Thr Val Thr Asn Ser Glu Lys Phe Ser Ala Phe His Leu Glu Gln
   600                 605                 610                 615
   aaa gtt gga gaa aag gta gaa gct ttg tct gaa gtt att acc ttc aaa      1987
   Lys Val Gly Glu Lys Val Glu Ala Leu Ser Glu Val Ile Thr Phe Lys
                   620                 625                 630
   aaa gga gaa taatct atg aac atg act gaa aaa att caa act tat tta       2035
   Lys Gly Glu        Met Asn Met Thr Glu Lys Ile Gln Thr Tyr Leu
                      635                 640                 645
   aac gat cca aag att gtt agc gtt aat act gtt gat gct cac tca gat      2083
   Asn Asp Pro Lys Ile Val Ser Val Asn Thr Val Asp Ala His Ser Asp
                   650                 655                 660
   cat aag tat ttt gaa tct ctt gaa gaa ttt tct gaa ggg gag atg aag      2131
   His Lys Tyr Phe Glu Ser Leu Glu Glu Phe Ser Glu Gly Glu Met Lys
               665                 670                 675
   tta aga caa tct ctt aat gga aaa tgg aaa att cac tat gct cag aat      2179
   Leu Arg Gln Ser Leu Asn Gly Lys Trp Lys Ile His Tyr Ala Gln Asn
           680                 685                 690
   aca aat cag gtt tta aaa gac ttt tat aaa aca gaa ttt gat gaa act      2227
   Thr Asn Gln Val Leu Lys Asp Phe Tyr Lys Thr Glu Phe Asp Glu Thr
       695                 700                 705
   gat ttg aat ttc atc aat gta cca ggt cat tta gag ctt caa ggt ttt      2275
   Asp Leu Asn Phe Ile Asn Val Pro Gly His Leu Glu Leu Gln Gly Phe
   710                 715                 720                 725
   ggt tct cca caa tat gtg aat acc caa tat cct tgg gat ggt aaa gaa      2323
   Gly Ser Pro Gln Tyr Val Asn Thr Gln Tyr Pro Trp Asp Gly Lys Glu
                   730                 735                 740
   ttc ctt cgt cca cct caa gtt cct caa gaa tca aat gct gtt gca tca      2371
   Phe Leu Arg Pro Pro Gln Val Pro Gln Glu Ser Asn Ala Val Ala Ser
               745                 750                 755
   tac gtt aaa cat ttt acc ttg aat gat gca tta aaa gat aaa aaa gta      2419
   Tyr Val Lys His Phe Thr Leu Asn Asp Ala Leu Lys Asp Lys Lys Val
           760                 765                 770
   ttt atc tca ttc caa ggg gtt gct act tcc atc ttt gta tgg gtc aat      2467
   Phe Ile Ser Phe Gln Gly Val Ala Thr Ser Ile Phe Val Trp Val Asn
       775                 780                 785
   ggt aac ttc gta gga tac agt gaa gat tca ttt aca cct agt gaa ttt      2515
   Gly Asn Phe Val Gly Tyr Ser Glu Asp Ser Phe Thr Pro Ser Glu Phe
   790                 795                 800                 805
   gaa att agt gat tac ctt gtt gaa ggt gat aac aag ttg gcg gta gct      2563
   Glu Ile Ser Asp Tyr Leu Val Glu Gly Asp Asn Lys Leu Ala Val Ala
                   810                 815                 820
   gtt tat cgt tac tct aca gca agc tgg ttg gaa gac caa gac ttc tgg      2611
   Val Tyr Arg Tyr Ser Thr Ala Ser Trp Leu Glu Asp Gln Asp Phe Trp
               825                 830                 835
   aga ctt tac ggt att ttt aga gat gtt tac ttg tat gct att cca aaa     2659
   Arg Leu Tyr Gly Ile Phe Arg Asp Val Tyr Leu Tyr Ala Ile Pro Lys
           840                 845                 850
   gtt cac gtt caa gat ctc ttt gtt aag gga gat tat gat tac caa aca     2707
   Val His Val Gln Asp Leu Phe Val Lys Gly Asp Tyr Asp Tyr Gln Thr
       855                 860                 865
   aaa gca ggt caa ttg gat att gat ttg aag act gtt ggt gat tat gaa     2755
   Lys Ala Gly Gln Leu Asp Ile Asp Leu Lys Thr Val Gly Asp Tyr Glu
   870                 875                 880                 885
   gac aag aag att aaa tat gtt ctt tca gat tat gaa ggc atc gtt aca     2803
   Asp Lys Lys Ile Lys Tyr Val Leu Ser Asp Tyr Glu Gly Ile Val Thr
                   890                 895                 900
   gaa ggt gat gca tct gtt aat ggt gac ggt gaa cta tct gta agt ctt     2851
   Glu Gly Asp Ala Ser Val Asn Gly Asp Gly Glu Leu Ser Val Ser Leu
               905                 910                 915
   gaa aat ctt aaa atc aaa cct tgg agt gct gaa agt cct aaa ctt tac     2899
   Glu Asn Leu Lys Ile Lys Pro Trp Ser Ala Glu Ser Pro Lys Leu Tyr
           920                 925                 930
   gat ttg atc ctt cat gtt ttg gat gat gac caa gtt gtt gaa gtc gtt     2947
   Asp Leu Ile Leu His Val Leu Asp Asp Asp Gln Val Val Glu Val Val
       935                 940                 945
   cca gtt aaa gtt gga ttt aga cgc ttt gaa att aaa gat aaa ctt atg     2995
   Pro Val Lys Val Gly Phe Arg Arg Phe Glu Ile Lys Asp Lys Leu Met
   950                 955                 960                 965
   ctt ttg aat ggt aag aga att gtc ttt aaa ggg gtt aac aga cac gaa     3043
   Leu Leu Asn Gly Lys Arg Ile Val Phe Lys Gly Val Asn Arg His Glu
                   970                 975                 980
   ttt aac gct aga aca gga cgt tgt atc act gaa gaa gat atg ctt tgg     3091
   Phe Asn Ala Arg Thr Gly Arg Cys Ile Thr Glu Glu Asp Met Leu Trp
               985                 990                 995
   gat atc aaa  gtg atg aaa caa cat  aac atc aat gct gtt  cgt act     3136
   Asp Ile Lys  Val Met Lys Gln His  Asn Ile Asn Ala Val  Arg Thr
           1000                 1005                 1010
   tca cac tat  cct aac cat aca cgt  tgg tat gaa ttg tgt  gat gaa     3181
   Ser His Tyr  Pro Asn His Thr Arg  Trp Tyr Glu Leu Cys  Asp Glu
           1015                 1020                 1025
   tat gga ctt  tat gtt atc gat gaa  gcc aac ctt gaa aca  cac ggt     3226
   Tyr Gly Leu  Tyr Val Ile Asp Glu  Ala Asn Leu Glu Thr  His Gly
           1030                 1035                 1040
   aca tgg caa  aaa ctt ggt cta tgc  gaa cct tca tgg aat  atc cca     3271
   Thr Trp Gln  Lys Leu Gly Leu Cys  Glu Pro Ser Trp Asn  Ile Pro
           1045                 1050                 1055
   gct agt gaa  cca gaa tgg ttg cct  gct tgt ttg gat cgt  gcc aat     3316
   Ala Ser Glu  Pro Glu Trp Leu Pro  Ala Cys Leu Asp Arg  Ala Asn
           1060                 1065                 1070
   aac atg ttc  caa cgc gat aag aac  cac gct agt gtt atc  att tgg     3361
   Asn Met Phe  Gln Arg Asp Lys Asn  His Ala Ser Val Ile  Ile Trp
           1075                 1080                 1085
   tct tgt ggt  aat gaa tca tat gct  ggt aaa gat att gct  gac atg     3406
   Ser Cys Gly  Asn Glu Ser Tyr Ala  Gly Lys Asp Ile Ala  Asp Met
           1090                 1095                 1100
   gct gat tac ttc cgt agt gtt gac aat act cgt cca gtt cac tat     3451
   Ala Asp Tyr Phe Arg Ser Val Asp Asn Thr Arg Pro Val His Tyr
           1105                1110                1115
   gaa ggt gtt gca tgg tgt cgt gag ttt gat tac att aca gac atc     3496
   Glu Gly Val Ala Trp Cys Arg Glu Phe Asp Tyr Ile Thr Asp Ile
           1120                1125                1130
   gaa agt cgt atg tat gcg aaa cca gct gat atc gaa gaa tac ctc     3541
   Glu Ser Arg Met Tyr Ala Lys Pro Ala Asp Ile Glu Glu Tyr Leu
           1135                1140                1145
   aca act ggt aaa cta gtt gat ctt tca agc gtt agt gat aaa cac     3586
   Thr Thr Gly Lys Leu Val Asp Leu Ser Ser Val Ser Asp Lys His
           1150                1155                1160
   ttt gct tca ggt aac cta act aac aaa cct caa aaa cct tat att     3631
   Phe Ala Ser Gly Asn Leu Thr Asn Lys Pro Gln Lys Pro Tyr Ile
           1165                1170                1175
   tca tgt gaa tac atg cac aca atg ggt aac tct ggt ggt gga ttg     3676
   Ser Cys Glu Tyr Met His Thr Met Gly Asn Ser Gly Gly Gly Leu
           1180                1185                1190
   caa ctc tac act gac tta gag aaa tat cca gaa tac caa ggt gga     3721
   Gln Leu Tyr Thr Asp Leu Glu Lys Tyr Pro Glu Tyr Gln Gly Gly
           1195                1200                1205
   ttt att tgg gac ttc att gac caa gct att tac aaa aca ctt cca     3766
   Phe Ile Trp Asp Phe Ile Asp Gln Ala Ile Tyr Lys Thr Leu Pro
           1210                1215                1220
   aat ggt agc gaa ttc cta tca tat ggt ggt gac tgg cat gat aga     3811
   Asn Gly Ser Glu Phe Leu Ser Tyr Gly Gly Asp Trp His Asp Arg
           1225                1230                1235
   cct tct gac tac gaa ttt tgt gga aat ggt atc gtc ttt gca gat     3856
   Pro Ser Asp Tyr Glu Phe Cys Gly Asn Gly Ile Val Phe Ala Asp
           1240                1245                1250
   cgt acc cta act cca aaa ctt caa aca gtt aaa cat ctt tac tct     3901
   Arg Thr Leu Thr Pro Lys Leu Gln Thr Val Lys His Leu Tyr Ser
           1255                1260                1265
   aat att aag att gct gtt gat gaa aaa tca gta act atc aag aat     3946
   Asn Ile Lys Ile Ala Val Asp Glu Lys Ser Val Thr Ile Lys Asn
           1270                1275                1280
   gat aat ctc ttc gaa gat ctt tct gct tat act ttc cta gct aga     3991
   Asp Asn Leu Phe Glu Asp Leu Ser Ala Tyr Thr Phe Leu Ala Arg
           1285                1290                1295
   gtt tac gaa gat ggt aga aaa gtt agt gaa agt gaa tat cac ttt     4036
   Val Tyr Glu Asp Gly Arg Lys Val Ser Glu Ser Glu Tyr His Phe
           1300                1305                1310
   gat gtt aaa cca ggc gaa gaa gca aca ttc cca gtt aac ttt gta     4081
   Asp Val Lys Pro Gly Glu Glu Ala Thr Phe Pro Val Asn Phe Val
           1315                1320                1325
   gtc gag gct tca aat tct gaa caa att tac gaa gtt gct tgt gtt     4126
   Val Glu Ala Ser Asn Ser Glu Gln Ile Tyr Glu Val Ala Cys Val
           1330                1335                1340
   ctg agg gaa gca act gaa tgg gct cct aaa ggt cat gaa att gtt     4171
   Leu Arg Glu Ala Thr Glu Trp Ala Pro Lys Gly His Glu Ile Val
           1345                1350                1355
   cgt ggt caa  tat gtt gtt gaa aag  att agc act gaa aca  cca gtt    4216
   Arg Gly Gln  Tyr Val Val Glu Lys  Ile Ser Thr Glu Thr  Pro Val
           1360                 1365                 1370
   aaa gca cct  ttg aat gtt gtt gaa  ggc gac ttc aac atc  ggt att    4261
   Lys Ala Pro  Leu Asn Val Val Glu  Gly Asp Phe Asn Ile  Gly Ile
           1375                 1380                 1385
   caa gga caa  aac ttc tca atc ttg  ctt tca cgt gca caa  aat act    4306
   Gln Gly Gln  Asn Phe Ser Ile Leu  Leu Ser Arg Ala Gln  Asn Thr
           1390                 1395                 1400
   tta gta tct  gct aag tat aat ggt  gtt gaa ttc att gag  aaa ggt    4351
   Leu Val Ser  Ala Lys Tyr Asn Gly  Val Glu Phe Ile Glu  Lys Gly
           1405                 1410                 1415
   cct aaa ctt  agc ttc act cgt gct  tac act gac aac gat  cgt ggt    4396
   Pro Lys Leu  Ser Phe Thr Arg Ala  Tyr Thr Asp Asn Asp  Arg Gly
           1420                 1425                 1430
   gct gga tat  cca ttc gaa atg gca  ggc tgg aag gtt gct  gga aac    4441
   Ala Gly Tyr  Pro Phe Glu Met Ala  Gly Trp Lys Val Ala  Gly Asn
           1435                 1440                 1445
   tat agt aaa  gtt aca gat act caa  att caa atc gaa gac  gac tct    4486
   Tyr Ser Lys  Val Thr Asp Thr Gln  Ile Gln Ile Glu Asp  Asp Ser
           1450                 1455                 1460
   gtt aaa gtg  act tat gtt cat gaa  ttg cca ggc ttg tct  gat gtc    4531
   Val Lys Val  Thr Tyr Val His Glu  Leu Pro Gly Leu Ser  Asp Val
           1465                 1470                 1475
   gaa gtt aag  gta act tat caa gtt  gat tac aag ggt cga  atc ttt    4576
   Glu Val Lys  Val Thr Tyr Gln Val  Asp Tyr Lys Gly Arg  Ile Phe
           1480                 1485                 1490
   gtt act gca  aac tat gat ggt aaa  gca ggt ttg cca aac  ttc cct    4621
   Val Thr Ala  Asn Tyr Asp Gly Lys  Ala Gly Leu Pro Asn  Phe Pro
           1495                 1500                 1505
   gaa ttt ggt  tta gaa ttt gct atc  ggt tca caa ttt aca  aac ctt    4666
   Glu Phe Gly  Leu Glu Phe Ala Ile  Gly Ser Gln Phe Thr  Asn Leu
           1510                 1515                 1520
   agc tat tat  gga tac ggt gca gaa  gaa agc tac cgt gat  aaa ctt    4711
   Ser Tyr Tyr  Gly Tyr Gly Ala Glu  Glu Ser Tyr Arg Asp  Lys Leu
           1525                 1530                 1535
   cct ggt gcc  tat ctt ggt cga tat  gaa aca tct gtt gaa  aag aca    4756
   Pro Gly Ala  Tyr Leu Gly Arg Tyr  Glu Thr Ser Val Glu  Lys Thr
           1540                 1545                 1550
   ttt gct cca  tat cta atg cca caa  gaa tct ggt aat cac  tat ggt    4801
   Phe Ala Pro  Tyr Leu Met Pro Gln  Glu Ser Gly Asn His  Tyr Gly
           1555                 1560                 1565
   act cgt gaa  ttc aca gta tct gat  gat aac cat aat ggt  ctt aaa    4846
   Thr Arg Glu  Phe Thr Val Ser Asp  Asp Asn His Asn Gly  Leu Lys
           1570                 1575                 1580
   ttc acc gca  ctt aat aaa gca ttc  gaa ttc agt gct ttg  cgt aac    4891
   Phe Thr Ala  Leu Asn Lys Ala Phe  Glu Phe Ser Ala Leu  Arg Asn
           1585                 1590                 1595
   act act gaa  caa att gaa aat gct  cgt caa caa tat gag  ttg caa    4936
   Thr Thr Glu  Gln Ile Glu Asn Ala  Arg Gln Gln Tyr Glu  Leu Gln
           1600                 1605                 1610
   gaa tct gat  gct aca tgg att aaa  gtg ctt gct gct caa  atg ggt     4981
   Glu Ser Asp  Ala Thr Trp Ile Lys  Val Leu Ala Ala Gln  Met Gly
           1615                 1620                 1625
   gta ggt ggt  gac gac aca tgg ggt  gct cca gtt cat gac  gaa ttc     5026
   Val Gly Gly  Asp Asp Thr Trp Gly  Ala Pro Val His Asp  Glu Phe
           1630                 1635                 1640
   ttg ctt agc  tca gca gat agc tat  caa tta agc ttc atg  att gaa     5071
   Leu Leu Ser  Ser Ala Asp Ser Tyr  Gln Leu Ser Phe Met  Ile Glu
           1645                 1650                 1655
   cca cta aat  taggtagtta ttaagaatct agttgtacat tatttgataa           5120
   Pro Leu Asn
           1660
   ttataaaaga gaagctttgg aggcttctct tttcatgttt taaaggagat taaatcatta   5180
   gtgtgttagt atcttcctcg agtacc                                        5206
<210> 2
   <211> 634
   <212> PRT
   <213> Streptococcus thermophilus
<400> 2
   Met Glu Lys Ser Lys Gly Gln Met Lys Ser Arg Leu Ser Tyr Ala Ala
   1               5                   10                  15
   Gly Ala Phe Gly Asn Asp Val Phe Tyr Ala Thr Leu Ser Thr Tyr Phe
               20                  25                  30
   Ile Met Phe Val Thr Thr His Leu Phe Asn Thr Gly Asp Pro Lys Gln
           35                  40                  45
   Asn Ser His Tyr Val Leu Leu Ile Thr Asn Ile Ile Ser Ile Leu Arg
       50                  55                  60
   Ile Leu Glu Val Phe Ile Asp Pro Leu Ile Gly Asn Met Ile Asp Asn
   65                  70                  75                  80
   Thr Asn Thr Lys Tyr Gly Lys Phe Lys Pro Trp Val Val Gly Gly Gly
                   85                  90                  95
   Ile Ile Ser Ser Ile Thr Leu Leu Phe Leu Leu Thr Asp Ile Gly Gly
               100                 105                 110
   Leu Asn Lys Thr Asn Pro Phe Leu Tyr Leu Val Leu Phe Gly Ile Ile
           115                 120                 125
   Tyr Leu Val Met Asp Val Phe Tyr Ser Ile Lys Asp Ile Gly Phe Trp
       130                 135                 140
   Ser Met Ile Pro Ala Leu Ser Leu Asp Ser His Glu Arg Glu Lys Met
   145                 150                 155                 160
   Ala Thr Phe Ala Arg Ile Gly Ser Thr Ile Gly Ala Asn Ile Val Gly
   165 170 175
   Val Ala Ile Met Pro Ile Asp Leu Leu Phe Ser Met Thr His Asn Ser
   180 185 190
   Gly Ser Gly Asp Lys Ser Gly Trp Phe Trp Tyr Ala Phe Ile Val Asp
   195 200 205
   Leu Asn Gly Val Ile Thr Ser Ile Ala Val Gly Ile Gly Thr Arg Glu
   210 215 220
   Val Glu Ser Lys Ile His Asp Asn Asn Glu Lys Thr Ser Leu Lys Gln
   225 230 235 240
   Val Phe Lys Val Leu Gly Gln Asn Asp Gln Met Met Trp Leu Ser Leu
   245 250 255
   Gly Tyr Trp Phe Tyr Gly Leu Gly Ile Asn Thr Leu Asn Ala Leu Gln
   260 265 270
   Leu Tyr Tyr Phe Thr Phe Ile Leu Gly Asp Ser Gly Lys Tyr Ser Ile
   275 280 285
   Leu Tyr Gly Leu Asn Thr Val Val Gly Leu Val Ser Val Ser Leu Phe
   290 295 300
   Pro Thr Leu Ala Asp Lys Phe Asn Arg Lys Arg Leu Phe Tyr Gly Cys
   305 310 315 320
   Ile Ala Val Met Leu Gly Gly Ile Gly Ile Phe Ser Ile Ala Gly Thr
   325 330 335
   Ser Leu Pro Ile Ile Leu Thr Ala Ala Glu Leu Phe Phe Ile Pro Gln
   340 345 350
   Pro Leu Val Phe Leu Val Val Phe Met Ile Ile Ser Asp Ser Val Glu
   355 360 365
   Tyr Gly Gln Trp Lys Thr Gly His Arg Asp Glu Ser Leu Thr Leu Ser
   370 375 380
   Val Arg Pro Leu Ile Asp Lys Leu Gly Gly Ala Met Ser Asn Trp Leu
   385 390 395 400
   Val Ser Thr Phe Ala Val Ala Ala Gly Met Thr Thr Gly Ala Ser Ala
   405 410 415
   Ser Thr Ile Thr Thr His Gln Gln Phe Ile Phe Lys Leu Gly Met Phe
   420 425 430
   Ala Phe Pro Ala Ala Thr Met Leu Ile Gly Ala Phe Ile Val Ala Arg
   435 440 445
   Lys Ile Thr Leu Thr Glu Ala Arg His Ala Lys Ile Val Glu Glu Leu
   450 455 460
   Glu His Arg Phe Ser Val Ala Thr Ser Glu Asn Glu Val Lys Ala Asn
   465 470 475 480
   Val Val Ser Leu Val Thr Pro Thr Thr Gly Tyr Leu Val Asp Leu Ser
   485 490 495
   Ser Val Asn Asp Glu His Phe Ala Ser Gly Ser Met Gly Lys Gly Phe
   500 505 510
   Ala Ile Lys Pro Thr Asp Gly Ala Val Phe Ala Pro Ile Ser Gly Thr
   515 520 525
   Ile Arg Gln Ile Leu Pro Thr Arg His Ala Val Gly Ile Glu Ser Glu
   530 535 540
   Asp Gly Val Ile Val Leu Ile His Val Gly Ile Gly Thr Val Lys Leu
   545 550 555 560
   Asn Gly Glu Gly Phe Ile Ser Tyr Val Glu Gln Gly Asp His Val Glu
   565 570 575
   Val Gly Gln Lys Leu Leu Glu Phe Trp Ser Pro Ile Ile Glu Lys Asn
   580 585 590
   Gly Leu Asp Asp Thr Val Leu Val Thr Val Thr Asn Ser Glu Lys Phe
   595 600 605
   Ser Ala Phe His Leu Glu Gln Lys Val Gly Glu Lys Val Glu Ala Leu
   610 615 620
   Ser Glu Val Ile Thr Phe Lys Lys Gly Glu
   625 630
<210> 3
   <211> 1026
   <212> PRT
   <213> streptococcus thermophilus
<400> 3
   Met Asn Met Thr Glu Lys Ile Gln Thr Tyr Leu Asn Asp Pro Lys Ile
   1 5 10 15
   Val Ser Val Asn Thr Val Asp Ala His Ser Asp His Lys Tyr Phe Glu
   20 25 30
   Ser Leu Glu Glu Phe Ser Glu Gly Glu Met Lys Leu Arg Gln Ser Leu
   35 40 45
   Asn Gly Lys Trp Lys Ile His Tyr Ala Gln Asn Thr Asn Gln Val Leu
   50 55 60
   Lys Asp Phe Tyr Lys Thr Glu Phe Asp Glu Thr Asp Leu Asn Phe Ile
   65 70 75 80
   Asn Val Pro Gly His Leu Glu Leu Gln Gly Phe Gly Ser Pro Gln Tyr
   85 90 95
   Val Asn Thr Gln Tyr Pro Trp Asp Gly Lys Glu Phe Leu Arg Pro Pro
   100 105 110
   Gln Val Pro Gln Glu Ser Asn Ala Val Ala Ser Tyr Val Lys His Phe
   115 120 125
   Thr Leu Asn Asp Ala Leu Lys Asp Lys Lys Val Phe Ile Ser Phe Gln
   130 135 140
   Gly Val Ala Thr Ser Ile Phe Val Trp Val Asn Gly Asn Phe Val Gly
   145 150 155 160
   Tyr Ser Glu Asp Ser Phe Thr Pro Ser Glu Phe Glu Ile Ser Asp Tyr
   165 170 175
   Leu Val Glu Gly Asp Asn Lys Leu Ala Val Ala Val Tyr Arg Tyr Ser
   180 185 190
   Thr Ala Ser Trp Leu Glu Asp Gln Asp Phe Trp Arg Leu Tyr Gly Ile
   195 200 205
   Phe Arg Asp Val Tyr Leu Tyr Ala Ile Pro Lys Val His Val Gln Asp
   210 215 220
   Leu Phe Val Lys Gly Asp Tyr Asp Tyr Gln Thr Lys Ala Gly Gln Leu
   225 230 235 240
   Asp Ile Asp Leu Lys Thr Val Gly Asp Tyr Glu Asp Lys Lys Ile Lys
   245 250 255
   Tyr Val Leu Ser Asp Tyr Glu Gly Ile Val Thr Glu Gly Asp Ala Ser
   260 265 270
   Val Asn Gly Asp Gly Glu Leu Ser Val Ser Leu Glu Asn Leu Lys Ile
   275 280 285
   Lys Pro Trp Ser Ala Glu Ser Pro Lys Leu Tyr Asp Leu Ile Leu His
   290 295 300
   Val Leu Asp Asp Asp Gln Val Val Glu Val Val Pro Val Lys Val Gly
   305 310 315 320
   Phe Arg Arg Phe Glu Ile Lys Asp Lys Leu Met Leu Leu Asn Gly Lys
   325 330 335
   Arg Ile Val Phe Lys Gly Val Asn Arg His Glu Phe Asn Ala Arg Thr
   340 345 350
   Gly Arg Cys Ile Thr Glu Glu Asp Met Leu Trp Asp Ile Lys Val Met
   355 360 365
   Lys Gln His Asn Ile Asn Ala Val Arg Thr Ser His Tyr Pro Asn His
   370 375 380
   Thr Arg Trp Tyr Glu Leu Cys Asp Glu Tyr Gly Leu Tyr Val Ile Asp
   385 390 395 400
   Glu Ala Asn Leu Glu Thr His Gly Thr Trp Gln Lys Leu Gly Leu Cys
   405 410 415
   Glu Pro Ser Trp Asn Ile Pro Ala Ser Glu Pro Glu Trp Leu Pro Ala
   420 425 430
   Cys Leu Asp Arg Ala Asn Asn Met Phe Gln Arg Asp Lys Asn His Ala
   435 440 445
   Ser Val Ile Ile Trp Ser Cys Gly Asn Glu Ser Tyr Ala Gly Lys Asp
   450 455 460
   Ile Ala Asp Met Ala Asp Tyr Phe Arg Ser Val Asp Asn Thr Arg Pro
   465 470 475 480
   Val His Tyr Glu Gly Val Ala Trp Cys Arg Glu Phe Asp Tyr Ile Thr
   485 490 495
   Asp Ile Glu Ser Arg Met Tyr Ala Lys Pro Ala Asp Ile Glu Glu Tyr
   500 505 510
   Leu Thr Thr Gly Lys Leu Val Asp Leu Ser Ser Val Ser Asp Lys His
   515 520 525
   Phe Ala Ser Gly Asn Leu Thr Asn Lys Pro Gln Lys Pro Tyr Ile Ser
   530 535 540
   Cys Glu Tyr Met His Thr Met Gly Asn Ser Gly Gly Gly Leu Gln Leu
   545 550 555 560
   Tyr Thr Asp Leu Glu Lys Tyr Pro Glu Tyr Gln Gly Gly Phe Ile Trp
   565 570 575
   Asp Phe Ile Asp Gln Ala Ile Tyr Lys Thr Leu Pro Asn Gly Ser Glu
   580 585 590
   Phe Leu Ser Tyr Gly Gly Asp Trp His Asp Arg Pro Ser Asp Tyr Glu
   595 600 605
   Phe Cys Gly Asn Gly Ile Val Phe Ala Asp Arg Thr Leu Thr Pro Lys
   610 615 620
   Leu Gln Thr Val Lys His Leu Tyr Ser Asn Ile Lys Ile Ala Val Asp
   625 630 635 640
   Glu Lys Ser Val Thr Ile Lys Asn Asp Asn Leu Phe Glu Asp Leu Ser
   645 650 655
   Ala Tyr Thr Phe Leu Ala Arg Val Tyr Glu Asp Gly Arg Lys Val Ser
   660 665 670
   Glu Ser Glu Tyr His Phe Asp Val Lys Pro Gly Glu Glu Ala Thr Phe
   675 680 685
   Pro Val Asn Phe Val Val Glu Ala Ser Asn Ser Glu Gln Ile Tyr Glu
   690 695 700
   Val Ala Cys Val Leu Arg Glu Ala Thr Glu Trp Ala Pro Lys Gly His
   705 710 715 720
   Glu Ile Val Arg Gly Gln Tyr Val Val Glu Lys Ile Ser Thr Glu Thr
   725 730 735
   Pro Val Lys Ala Pro Leu Asn Val Val Glu Gly Asp Phe Asn Ile Gly
   740 745 750
   Ile Gln Gly Gln Asn Phe Ser Ile Leu Leu Ser Arg Ala Gln Asn Thr
   755 760 765
   Leu Val Ser Ala Lys Tyr Asn Gly Val Glu Phe Ile Glu Lys Gly Pro
   770 775 780
   Lys Leu Ser Phe Thr Arg Ala Tyr Thr Asp Asn Asp Arg Gly Ala Gly
   785 790 795 800
   Tyr Pro Phe Glu Met Ala Gly Trp Lys Val Ala Gly Asn Tyr Ser Lys
   805 810 815
   Val Thr Asp Thr Gln Ile Gln Ile Glu Asp Asp Ser Val Lys Val Thr
   820 825 830
   Tyr Val His Glu Leu Pro Gly Leu Ser Asp Val Glu Val Lys Val Thr
   835 840 845
   Tyr Gln Val Asp Tyr Lys Gly Arg Ile Phe Val Thr Ala Asn Tyr Asp
   850 855 860
   Gly Lys Ala Gly Leu Pro Asn Phe Pro Glu Phe Gly Leu Glu Phe Ala
   865 870 875 880
   Ile Gly Ser Gln Phe Thr Asn Leu Ser Tyr Tyr Gly Tyr Gly Ala Glu
   885 890 895
   Glu Ser Tyr Arg Asp Lys Leu Pro Gly Ala Tyr Leu Gly Arg Tyr Glu
   900 905 910
   Thr Ser Val Glu Lys Thr Phe Ala Pro Tyr Leu Met Pro Gln Glu Ser
   915 920 925
   Gly Asn His Tyr Gly Thr Arg Glu Phe Thr Val Ser Asp Asp Asn His
   930 935 940
   Asn Gly Leu Lys Phe Thr Ala Leu Asn Lys Ala Phe Glu Phe Ser Ala
   945 950 955 960
   Leu Arg Asn Thr Thr Glu Gln Ile Glu Asn Ala Arg Gln Gln Tyr Glu
   965 970 975
   Leu Gln Glu Ser Asp Ala Thr Trp Ile Lys Val Leu Ala Ala Gln Met
   980 985 990
   Gly Val Gly Gly Asp Asp Thr Trp Gly Ala Pro Val His Asp Glu Phe
   995 1000 1005
   Leu Leu Ser Ser Ala Asp Ser Tyr Gln Leu Ser Phe Met Ile Glu
   1010 1015 1020
   Pro Leu Asn
   1025
<210> 4
   <211> 3081
   <212> DNA
   <213> Streptococcus thermophilus
<220>
   <221> CDS
   <222> (1)..(3078)
   <223> LacZ CDS
<400> 4
   atg aac atg act gaa aaa att caa act tat tta aac gat cca aag att       48
   Met Asn Met Thr Glu Lys Ile Gln Thr Tyr Leu Asn Asp Pro Lys Ile
   1               5                   10                  15
   gtt agc gtt aat act gtt gat gct cac tca gat cat aag tat ttt gaa       96
   Val Ser Val Asn Thr Val Asp Ala His Ser Asp His Lys Tyr Phe Glu
               20                  25                  30
   tct ctt gaa gaa ttt tct gaa ggg gag atg aag tta aga caa tct ctt      144
   Ser Leu Glu Glu Phe Ser Glu Gly Glu Met Lys Leu Arg Gln Ser Leu
           35                  40                  45
   aat gga aaa tgg aaa att cac tat gct cag aat aca aat cag gtt tta     192
   Asn Gly Lys Trp Lys Ile His Tyr Ala Gln Asn Thr Asn Gln Val Leu
       50                  55                  60
   aaa gac ttt tat aaa aca gaa ttt gat gaa act gat ttg aat ttc atc     240
   Lys Asp Phe Tyr Lys Thr Glu Phe Asp Glu Thr Asp Leu Asn Phe Ile
   65                  70                  75                  80
   aat gta cca ggt cat tta gag ctt caa ggt ttt ggt tct cca caa tat     288
   Asn Val Pro Gly His Leu Glu Leu Gln Gly Phe Gly Ser Pro Gln Tyr
                   85                  90                  95
   gtg aat acc caa tat cct tgg gat ggt aaa gaa ttc ctt cgt cca cct     336
   Val Asn Thr Gln Tyr Pro Trp Asp Gly Lys Glu Phe Leu Arg Pro Pro
               100                 105                 110
   caa gtt cct caa gaa tca aat gct gtt gca tca tac gtt aaa cat ttt     384
   Gln Val Pro Gln Glu Ser Asn Ala Val Ala Ser Tyr Val Lys His Phe
           115                 120                 125
   acc ttg aat gat gca tta aaa gat aaa aaa gta ttt atc tca ttc caa     432
   Thr Leu Asn Asp Ala Leu Lys Asp Lys Lys Val Phe Ile Ser Phe Gln
       130                 135                 140
   ggg gtt gct act tcc atc ttt gta tgg gtc aat ggt aac ttc gta gga     480
   Gly Val Ala Thr Ser Ile Phe Val Trp Val Asn Gly Asn Phe Val Gly
   145                 150                 155                 160
   tac agt gaa gat tca ttt aca cct agt gaa ttt gaa att agt gat tac     528
   Tyr Ser Glu Asp Ser Phe Thr Pro Ser Glu Phe Glu Ile Ser Asp Tyr
                   165                 170                 175
   ctt gtt gaa ggt gat aac aag ttg gcg gta gct gtt tat cgt tac tct     576
   Leu Val Glu Gly Asp Asn Lys Leu Ala Val Ala Val Tyr Arg Tyr Ser
               180                 185                 190
   aca gca agc tgg ttg gaa gac caa gac ttc tgg aga ctt tac ggt att     624
   Thr Ala Ser Trp Leu Glu Asp Gln Asp Phe Trp Arg Leu Tyr Gly Ile
           195                 200                 205
   ttt aga gat gtt tac ttg tat gct att cca aaa gtt cac gtt caa gat     672
   Phe Arg Asp Val Tyr Leu Tyr Ala Ile Pro Lys Val His Val Gln Asp
       210                 215                 220
   ctc ttt gtt aag gga gat tat gat tac caa aca aaa gca ggt caa ttg     720
   Leu Phe Val Lys Gly Asp Tyr Asp Tyr Gln Thr Lys Ala Gly Gln Leu
   225                 230                 235                 240
   gat att gat ttg aag act gtt ggt gat tat gaa gac aag aag att aaa     768
   Asp Ile Asp Leu Lys Thr Val Gly Asp Tyr Glu Asp Lys Lys Ile Lys
                   245                 250                 255
   tat gtt ctt tca gat tat gaa ggc atc gtt aca gaa ggt gat gca tct     816
   Tyr Val Leu Ser Asp Tyr Glu Gly Ile Val Thr Glu Gly Asp Ala Ser
               260                 265                 270
   gtt aat ggt gac ggt gaa cta tct gta agt ctt gaa aat ctt aaa atc     864
   Val Asn Gly Asp Gly Glu Leu Ser Val Ser Leu Glu Asn Leu Lys Ile
           275                 280                 285
   aaa cct tgg agt gct gaa agt cct aaa ctt tac gat ttg atc ctt cat     912
   Lys Pro Trp Ser Ala Glu Ser Pro Lys Leu Tyr Asp Leu Ile Leu His
       290                 295                 300
   gtt ttg gat gat gac caa gtt gtt gaa gtc gtt cca gtt aaa gtt gga     960
   Val Leu Asp Asp Asp Gln Val Val Glu Val Val Pro Val Lys Val Gly
   305                 310                 315                 320
   ttt aga cgc ttt gaa att aaa gat aaa ctt atg ctt ttg aat ggt aag      1008
   Phe Arg Arg Phe Glu Ile Lys Asp Lys Leu Met Leu Leu Asn Gly Lys
                   325                 330                 335
   aga att gtc ttt aaa ggg gtt aac aga cac gaa ttt aac gct aga aca      1056
   Arg Ile Val Phe Lys Gly Val Asn Arg His Glu Phe Asn Ala Arg Thr
               340                 345                 350
   gga cgt tgt atc act gaa gaa gat atg ctt tgg gat atc aaa gtg atg      1104
   Gly Arg Cys Ile Thr Glu Glu Asp Met Leu Trp Asp Ile Lys Val Met
           355                 360                 365
   aaa caa cat aac atc aat gct gtt cgt act tca cac tat cct aac cat      1152
   Lys Gln His Asn Ile Asn Ala Val Arg Thr Ser His Tyr Pro Asn His
       370                 375                 380
   aca cgt tgg tat gaa ttg tgt gat gaa tat gga ctt tat gtt atc gat      1200
   Thr Arg Trp Tyr Glu Leu Cys Asp Glu Tyr Gly Leu Tyr Val Ile Asp
   385                 390                 395                 400
   gaa gcc aac ctt gaa aca cac ggt aca tgg caa aaa ctt ggt cta tgc      1248
   Glu Ala Asn Leu Glu Thr His Gly Thr Trp Gln Lys Leu Gly Leu Cys
                   405                 410                 415
   gaa cct tca tgg aat atc cca gct agt gaa cca gaa tgg ttg cct gct      1296
   Glu Pro Ser Trp Asn Ile Pro Ala Ser Glu Pro Glu Trp Leu Pro Ala
               420                 425                 430
   tgt ttg gat cgt gcc aat aac atg ttc caa cgc gat aag aac cac gct      1344
   Cys Leu Asp Arg Ala Asn Asn Met Phe Gln Arg Asp Lys Asn His Ala
           435                 440                 445
   agt gtt atc att tgg tct tgt ggt aat gaa tca tat gct ggt aaa gat      1392
   Ser Val Ile Ile Trp Ser Cys Gly Asn Glu Ser Tyr Ala Gly Lys Asp
       450                 455                 460
   att gct gac atg gct gat tac ttc cgt agt gtt gac aat act cgt cca      1440
   Ile Ala Asp Met Ala Asp Tyr Phe Arg Ser Val Asp Asn Thr Arg Pro
   465                 470                 475                 480
   gtt cac tat gaa ggt gtt gca tgg tgt cgt gag ttt gat tac att aca      1488
   Val His Tyr Glu Gly Val Ala Trp Cys Arg Glu Phe Asp Tyr Ile Thr
                   485                 490                 495
   gac atc gaa agt cgt atg tat gcg aaa cca gct gat atc gaa gaa tac      1536
   Asp Ile Glu Ser Arg Met Tyr Ala Lys Pro Ala Asp Ile Glu Glu Tyr
               500                 505                 510
   ctc aca act ggt aaa cta gtt gat ctt tca agc gtt agt gat aaa cac      1584
   Leu Thr Thr Gly Lys Leu Val Asp Leu Ser Ser Val Ser Asp Lys His
           515                 520                 525
   ttt gct tca ggt aac cta act aac aaa cct caa aaa cct tat att tca      1632
   Phe Ala Ser Gly Asn Leu Thr Asn Lys Pro Gln Lys Pro Tyr Ile Ser
       530                 535                 540
   tgt gaa tac atg cac aca atg ggt aac tct ggt ggt gga ttg caa ctc      1680
   Cys Glu Tyr Met His Thr Met Gly Asn Ser Gly Gly Gly Leu Gln Leu
   545                 550                 555                 560
   tac act gac tta gag aaa tat cca gaa tac caa ggt gga ttt att tgg      1728
   Tyr Thr Asp Leu Glu Lys Tyr Pro Glu Tyr Gln Gly Gly Phe Ile Trp
                   565                 570                 575
   gac ttc att gac caa gct att tac aaa aca ctt cca aat ggt agc gaa      1776
   Asp Phe Ile Asp Gln Ala Ile Tyr Lys Thr Leu Pro Asn Gly Ser Glu
               580                 585                 590
   ttc cta tca tat ggt ggt gac tgg cat gat aga cct tct gac tac gaa      1824
   Phe Leu Ser Tyr Gly Gly Asp Trp His Asp Arg Pro Ser Asp Tyr Glu
           595                 600                 605
   ttt tgt gga aat ggt atc gtc ttt gca gat cgt acc cta act cca aaa      1872
   Phe Cys Gly Asn Gly Ile Val Phe Ala Asp Arg Thr Leu Thr Pro Lys
       610                 615                 620
   ctt caa aca gtt aaa cat ctt tac tct aat att aag att gct gtt gat      1920
   Leu Gln Thr Val Lys His Leu Tyr Ser Asn Ile Lys Ile Ala Val Asp
   625                 630                 635                 640
   gaa aaa tca gta act atc aag aat gat aat ctc ttc gaa gat ctt tct      1968
   Glu Lys Ser Val Thr Ile Lys Asn Asp Asn Leu Phe Glu Asp Leu Ser
                   645                 650                 655
   gct tat act ttc cta gct aga gtt tac gaa gat ggt aga aaa gtt agt      2016
   Ala Tyr Thr Phe Leu Ala Arg Val Tyr Glu Asp Gly Arg Lys Val Ser
               660                 665                 670
   gaa agt gaa tat cac ttt gat gtt aaa cca ggc gaa gaa gca aca ttc      2064
   Glu Ser Glu Tyr His Phe Asp Val Lys Pro Gly Glu Glu Ala Thr Phe
           675                 680                 685
   cca gtt aac ttt gta gtc gag gct tca aat tct gaa caa att tac gaa      2112
   Pro Val Asn Phe Val Val Glu Ala Ser Asn Ser Glu Gln Ile Tyr Glu
       690                 695                 700
   gtt gct tgt gtt ctg agg gaa gca act gaa tgg gct cct aaa ggt cat      2160
   Val Ala Cys Val Leu Arg Glu Ala Thr Glu Trp Ala Pro Lys Gly His
   705                 710                 715                 720
   gaa att gtt cgt ggt caa tat gtt gtt gaa aag att agc act gaa aca      2208
   Glu Ile Val Arg Gly Gln Tyr Val Val Glu Lys Ile Ser Thr Glu Thr
                   725                 730                 735
   cca gtt aaa gca cct ttg aat gtt gtt gaa ggc gac ttc aac atc ggt      2256
   Pro Val Lys Ala Pro Leu Asn Val Val Glu Gly Asp Phe Asn Ile Gly
               740                 745                 750
   att caa gga caa aac ttc tca atc ttg ctt tca cgt gca caa aat act      2304
   Ile Gln Gly Gln Asn Phe Ser Ile Leu Leu Ser Arg Ala Gln Asn Thr
           755                 760                 765
   tta gta tct gct aag tat aat ggt gtt gaa ttc att gag aaa ggt cct      2352
   Leu Val Ser Ala Lys Tyr Asn Gly Val Glu Phe Ile Glu Lys Gly Pro
       770                 775                 780
   aaa ctt agc ttc act cgt gct tac act gac aac gat cgt ggt gct gga      2400
   Lys Leu Ser Phe Thr Arg Ala Tyr Thr Asp Asn Asp Arg Gly Ala Gly
   785                 790                 795                 800
   tat cca ttc gaa atg gca ggc tgg aag gtt gct gga aac tat agt aaa      2448
   Tyr Pro Phe Glu Met Ala Gly Trp Lys Val Ala Gly Asn Tyr Ser Lys
                   805                 810                 815
   gtt aca gat act caa att caa atc gaa gac gac tct gtt aaa gtg act      2496
   Val Thr Asp Thr Gln Ile Gln Ile Glu Asp Asp Ser Val Lys Val Thr
               820                 825                 830
   tat gtt cat gaa ttg cca ggc ttg tct gat gtc gaa gtt aag gta act      2544
   Tyr Val His Glu Leu Pro Gly Leu Ser Asp Val Glu Val Lys Val Thr
           835                 840                 845
   tat caa gtt gat tac aag ggt cga atc ttt gtt act gca aac tat gat      2592
   Tyr Gln Val Asp Tyr Lys Gly Arg Ile Phe Val Thr Ala Asn Tyr Asp
   850 855 860
   ggt aaa gca ggt ttg cca aac ttc cct gaa ttt ggt tta gaa ttt gct 2640
   Gly Lys Ala Gly Leu Pro Asn Phe Pro Glu Phe Gly Leu Glu Phe Ala
   865 870 875 880
   atc ggt tca caa ttt aca aac ctt agc tat tat gga tac ggt gca gaa 2688
   Ile Gly Ser Gln Phe Thr Asn Leu Ser Tyr Tyr Gly Tyr Gly Ala Glu
   885 890 895
   gaa agc tac cgt gat aaa ctt cct ggt gcc tat ctt ggt cga tat gaa 2736
   Glu Ser Tyr Arg Asp Lys Leu Pro Gly Ala Tyr Leu Gly Arg Tyr Glu
   900 905 910
   aca tct gtt gaa aag aca ttt gct cca tat cta atg cca caa gaa tct 2784
   Thr Ser Val Glu Lys Thr Phe Ala Pro Tyr Leu Met Pro Gln Glu Ser
   915 920 925
   ggt aat cac tat ggt act cgt gaa ttc aca gta tct gat gat aac cat 2832
   Gly Asn His Tyr Gly Thr Arg Glu Phe Thr Val Ser Asp Asp Asn His
   930 935 940
   aat ggt ctt aaa ttc acc gca ctt aat aaa gca ttc gaa ttc agt gct 2880
   Asn Gly Leu Lys Phe Thr Ala Leu Asn Lys Ala Phe Glu Phe Ser Ala
   945 950 955 960
   ttg cgt aac act act gaa caa att gaa aat gct cgt caa caa tat gag 2928
   Leu Arg Asn Thr Thr Glu Gln Ile Glu Asn Ala Arg Gln Gln Tyr Glu
   965 970 975
   ttg caa gaa tct gat gct aca tgg att aaa gtg ctt gct gct caa atg 2976
   Leu Gln Glu Ser Asp Ala Thr Trp Ile Lys Val Leu Ala Ala Gln Met
   980 985 990
   ggt gta ggt ggt gac gac aca tgg ggt gct cca gtt cat gac gaa ttc 3024
   Gly Val Gly Gly Asp Asp Thr Trp Gly Ala Pro Val His Asp Glu Phe
   995 1000 1005
   ttg ctt agc tca gca gat agc tat caa tta agc ttc atg att gaa 3069
   Leu Leu Ser Ser Ala Asp Ser Tyr Gln Leu Ser Phe Met Ile Glu
   1010 1015 1020
   cca cta aat tag 3081
   Pro Leu Asn
   1025
<210> 5
   <211> 1026
   <212> PRT
   <213> streptococcus thermophilus
<400> 5
   Met Asn Met Thr Glu Lys Ile Gln Thr Tyr Leu Asn Asp Pro Lys Ile
   1               5                   10                  15
   Val Ser Val Asn Thr Val Asp Ala His Ser Asp His Lys Tyr Phe Glu
               20                  25                  30
   Ser Leu Glu Glu Phe Ser Glu Gly Glu Met Lys Leu Arg Gln Ser Leu
           35                  40                  45
   Asn Gly Lys Trp Lys Ile His Tyr Ala Gln Asn Thr Asn Gln Val Leu
   50 55 60
   Lys Asp Phe Tyr Lys Thr Glu Phe Asp Glu Thr Asp Leu Asn Phe Ile
   65 70 75 80
   Asn Val Pro Gly His Leu Glu Leu Gln Gly Phe Gly Ser Pro Gln Tyr
   85 90 95
   Val Asn Thr Gln Tyr Pro Trp Asp Gly Lys Glu Phe Leu Arg Pro Pro
   100 105 110
   Gln Val Pro Gln Glu Ser Asn Ala Val Ala Ser Tyr Val Lys His Phe
   115 120 125
   Thr Leu Asn Asp Ala Leu Lys Asp Lys Lys Val Phe Ile Ser Phe Gln
   130 135 140
   Gly Val Ala Thr Ser Ile Phe Val Trp Val Asn Gly Asn Phe Val Gly
   145 150 155 160
   Tyr Ser Glu Asp Ser Phe Thr Pro Ser Glu Phe Glu Ile Ser Asp Tyr
   165 170 175
   Leu Val Glu Gly Asp Asn Lys Leu Ala Val Ala Val Tyr Arg Tyr Ser
   180 185 190
   Thr Ala Ser Trp Leu Glu Asp Gln Asp Phe Trp Arg Leu Tyr Gly Ile
   195 200 205
   Phe Arg Asp Val Tyr Leu Tyr Ala Ile Pro Lys Val His Val Gln Asp
   210 215 220
   Leu Phe Val Lys Gly Asp Tyr Asp Tyr Gln Thr Lys Ala Gly Gln Leu
   225 230 235 240
   Asp Ile Asp Leu Lys Thr Val Gly Asp Tyr Glu Asp Lys Lys Ile Lys
   245 250 255
   Tyr Val Leu Ser Asp Tyr Glu Gly Ile Val Thr Glu Gly Asp Ala Ser
   260 265 270
   Val Asn Gly Asp Gly Glu Leu Ser Val Ser Leu Glu Asn Leu Lys Ile
   275 280 285
   Lys Pro Trp Ser Ala Glu Ser Pro Lys Leu Tyr Asp Leu Ile Leu His
   290 295 300
   Val Leu Asp Asp Asp Gln Val Val Glu Val Val Pro Val Lys Val Gly
   305 310 315 320
   Phe Arg Arg Phe Glu Ile Lys Asp Lys Leu Met Leu Leu Asn Gly Lys
   325 330 335
   Arg Ile Val Phe Lys Gly Val Asn Arg His Glu Phe Asn Ala Arg Thr
   340 345 350
   Gly Arg Cys Ile Thr Glu Glu Asp Met Leu Trp Asp Ile Lys Val Met
   355 360 365
   Lys Gln His Asn Ile Asn Ala Val Arg Thr Ser His Tyr Pro Asn His
   370 375 380
   Thr Arg Trp Tyr Glu Leu Cys Asp Glu Tyr Gly Leu Tyr Val Ile Asp
   385 390 395 400
   Glu Ala Asn Leu Glu Thr His Gly Thr Trp Gln Lys Leu Gly Leu Cys
   405 410 415
   Glu Pro Ser Trp Asn Ile Pro Ala Ser Glu Pro Glu Trp Leu Pro Ala
   420 425 430
   Cys Leu Asp Arg Ala Asn Asn Met Phe Gln Arg Asp Lys Asn His Ala
   435 440 445
   Ser Val Ile Ile Trp Ser Cys Gly Asn Glu Ser Tyr Ala Gly Lys Asp
   450 455 460
   Ile Ala Asp Met Ala Asp Tyr Phe Arg Ser Val Asp Asn Thr Arg Pro
   465 470 475 480
   Val His Tyr Glu Gly Val Ala Trp Cys Arg Glu Phe Asp Tyr Ile Thr
   485 490 495
   Asp Ile Glu Ser Arg Met Tyr Ala Lys Pro Ala Asp Ile Glu Glu Tyr
   500 505 510
   Leu Thr Thr Gly Lys Leu Val Asp Leu Ser Ser Val Ser Asp Lys His
   515 520 525
   Phe Ala Ser Gly Asn Leu Thr Asn Lys Pro Gln Lys Pro Tyr Ile Ser
   530 535 540
   Cys Glu Tyr Met His Thr Met Gly Asn Ser Gly Gly Gly Leu Gln Leu
   545 550 555 560
   Tyr Thr Asp Leu Glu Lys Tyr Pro Glu Tyr Gln Gly Gly Phe Ile Trp
   565 570 575
   Asp Phe Ile Asp Gln Ala Ile Tyr Lys Thr Leu Pro Asn Gly Ser Glu
   580 585 590
   Phe Leu Ser Tyr Gly Gly Asp Trp His Asp Arg Pro Ser Asp Tyr Glu
   595 600 605
   Phe Cys Gly Asn Gly Ile Val Phe Ala Asp Arg Thr Leu Thr Pro Lys
   610 615 620
   Leu Gln Thr Val Lys His Leu Tyr Ser Asn Ile Lys Ile Ala Val Asp
   625 630 635 640
   Glu Lys Ser Val Thr Ile Lys Asn Asp Asn Leu Phe Glu Asp Leu Ser
   645 650 655
   Ala Tyr Thr Phe Leu Ala Arg Val Tyr Glu Asp Gly Arg Lys Val Ser
   660 665 670
   Glu Ser Glu Tyr His Phe Asp Val Lys Pro Gly Glu Glu Ala Thr Phe
   675 680 685
   Pro Val Asn Phe Val Val Glu Ala Ser Asn Ser Glu Gln Ile Tyr Glu
   690 695 700
   Val Ala Cys Val Leu Arg Glu Ala Thr Glu Trp Ala Pro Lys Gly His
   705 710 715 720
   Glu Ile Val Arg Gly Gln Tyr Val Val Glu Lys Ile Ser Thr Glu Thr
   725 730 735
   Pro Val Lys Ala Pro Leu Asn Val Val Glu Gly Asp Phe Asn Ile Gly
   740 745 750
   Ile Gln Gly Gln Asn Phe Ser Ile Leu Leu Ser Arg Ala Gln Asn Thr
   755 760 765
   Leu Val Ser Ala Lys Tyr Asn Gly Val Glu Phe Ile Glu Lys Gly Pro
   770 775 780
   Lys Leu Ser Phe Thr Arg Ala Tyr Thr Asp Asn Asp Arg Gly Ala Gly
   785 790 795 800
   Tyr Pro Phe Glu Met Ala Gly Trp Lys Val Ala Gly Asn Tyr Ser Lys
   805 810 815
   Val Thr Asp Thr Gln Ile Gln Ile Glu Asp Asp Ser Val Lys Val Thr
   820 825 830
   Tyr Val His Glu Leu Pro Gly Leu Ser Asp Val Glu Val Lys Val Thr
   835 840 845
   Tyr Gln Val Asp Tyr Lys Gly Arg Ile Phe Val Thr Ala Asn Tyr Asp
   850 855 860
   Gly Lys Ala Gly Leu Pro Asn Phe Pro Glu Phe Gly Leu Glu Phe Ala
   865 870 875 880
   Ile Gly Ser Gln Phe Thr Asn Leu Ser Tyr Tyr Gly Tyr Gly Ala Glu
   885 890 895
   Glu Ser Tyr Arg Asp Lys Leu Pro Gly Ala Tyr Leu Gly Arg Tyr Glu
   900 905 910
   Thr Ser Val Glu Lys Thr Phe Ala Pro Tyr Leu Met Pro Gln Glu Ser
   915 920 925
   Gly Asn His Tyr Gly Thr Arg Glu Phe Thr Val Ser Asp Asp Asn His
   930 935 940
   Asn Gly Leu Lys Phe Thr Ala Leu Asn Lys Ala Phe Glu Phe Ser Ala
   945 950 955 960
   Leu Arg Asn Thr Thr Glu Gln Ile Glu Asn Ala Arg Gln Gln Tyr Glu
   965 970 975
   Leu Gln Glu Ser Asp Ala Thr Trp Ile Lys Val Leu Ala Ala Gln Met
   980 985 990
   Gly Val Gly Gly Asp Asp Thr Trp Gly Ala Pro Val His Asp Glu Phe
   995 1000 1005
   Leu Leu Ser Ser Ala Asp Ser Tyr Gln Leu Ser Phe Met Ile Glu
   1010 1015 1020
   Pro Leu Asn
   1025
<210> 6
   <211> 1905
   <212> DNA
   <213> Streptococcus thermophilus
<220>
   <221> CDS
   <222> (1)..(1902)
   <223> LacS CDS
<400> 6
   atg gaa aaa tct aaa ggt cag atg aag tct cgt tta tcc tac gca gct        48
   Met Glu Lys Ser Lys Gly Gln Met Lys Ser Arg Leu Ser Tyr Ala Ala
   1               5                   10                  15
   ggt gct ttt ggt aac gac gtc ttc tat gca acc ttg tca aca tac ttt        96
   Gly Ala Phe Gly Asn Asp Val Phe Tyr Ala Thr Leu Ser Thr Tyr Phe
               20                  25                  30
   atc atg ttt gtg aca act cac ttg ttt aac aca ggt gat cca aag caa       144
   Ile Met Phe Val Thr Thr His Leu Phe Asn Thr Gly Asp Pro Lys Gln
           35                  40                  45
   aat agt cac tac gta cta tta atc act aac att atc tct att ttg cgt       192
   Asn Ser His Tyr Val Leu Leu Ile Thr Asn Ile Ile Ser Ile Leu Arg
       50                  55                  60
   atc ttg gaa gta ttt atc gat cca ttg atc ggt aat atg att gat aac      240
   Ile Leu Glu Val Phe Ile Asp Pro Leu Ile Gly Asn Met Ile Asp Asn
   65                  70                  75                  80
   act aat act aag tat ggt aaa ttc aaa cca tgg gta gtt ggt ggt ggt      288
   Thr Asn Thr Lys Tyr Gly Lys Phe Lys Pro Trp Val Val Gly Gly Gly
                   85                  90                  95
   atc atc agt tct atc acc ttg ttg ttt ctc tta acc gat ata ggt ggt      336
   Ile Ile Ser Ser Ile Thr Leu Leu Phe Leu Leu Thr Asp Ile Gly Gly
               100                 105                 110
   ttg aat aaa aca aat cct ttc ttg tat ctt gta ctt ttt gga att atc      384
   Leu Asn Lys Thr Asn Pro Phe Leu Tyr Leu Val Leu Phe Gly Ile Ile
           115                 120                 125
   tac ctt gta atg gat gtc ttc tac tcg att aaa gat atc ggt ttc tgg      432
   Tyr Leu Val Met Asp Val Phe Tyr Ser Ile Lys Asp Ile Gly Phe Trp
       130                 135                 140
   tca atg att cct gcc ttg tct ctt gat agt cac gaa cgt gaa aaa atg      480
   Ser Met Ile Pro Ala Leu Ser Leu Asp Ser His Glu Arg Glu Lys Met
   145                 150                 155                 160
   gca act ttt gcc cgt att ggt tcg acg att ggt gcc aat att gta ggt      528
   Ala Thr Phe Ala Arg Ile Gly Ser Thr Ile Gly Ala Asn Ile Val Gly
                   165                 170                 175
   gtt gcc atc atg cca atc gat ttg tta ttc tct atg acg cac aat agt      576
   Val Ala Ile Met Pro Ile Asp Leu Leu Phe Ser Met Thr His Asn Ser
               180                 185                 190
   ggc tct gga gat aaa tct gga tgg ttc tgg tat gca ttt atc gta gat      624
   Gly Ser Gly Asp Lys Ser Gly Trp Phe Trp Tyr Ala Phe Ile Val Asp
           195                 200                 205
   ctc aat ggt gtg att aca tca att gct gtt ggt att ggt aca cgt gaa      672
   Leu Asn Gly Val Ile Thr Ser Ile Ala Val Gly Ile Gly Thr Arg Glu
       210                 215                 220
   gtt gag tca aaa att cat gat aat aac gaa aaa act agc ctt aaa caa      720
   Val Glu Ser Lys Ile His Asp Asn Asn Glu Lys Thr Ser Leu Lys Gln
   225                 230                 235                 240
   gtc ttt aag gtt ctt ggt caa aac gac caa atg atg tgg tta tct ctt      768
   Val Phe Lys Val Leu Gly Gln Asn Asp Gln Met Met Trp Leu Ser Leu
                   245                 250                 255
   gga tat tgg ttc tat ggt ctt ggt att aat aca ctt aat gct ctt caa      816
   Gly Tyr Trp Phe Tyr Gly Leu Gly Ile Asn Thr Leu Asn Ala Leu Gln
               260                 265                 270
   ctt tat tat ttc aca ttt atc ctt ggt gat tca ggt aaa tac tca att      864
   Leu Tyr Tyr Phe Thr Phe Ile Leu Gly Asp Ser Gly Lys Tyr Ser Ile
           275                 280                 285
   ctt tac gga ttg aat aca gtt gtt ggt ttg gtt tca gtt tca ctc ttc      912
   Leu Tyr Gly Leu Asn Thr Val Val Gly Leu Val Ser Val Ser Leu Phe
       290                 295                 300
   cct acc cta gct gat aaa ttc aac cgt aaa cgt ttg ttc tac gga tgt      960
   Pro Thr Leu Ala Asp Lys Phe Asn Arg Lys Arg Leu Phe Tyr Gly Cys
   305                 310                 315                 320
   att gca gta atg ctc ggg ggt atc gga ata ttt agt att gca ggt aca     1008
   Ile Ala Val Met Leu Gly Gly Ile Gly Ile Phe Ser Ile Ala Gly Thr
   325 330 335
   tca ctt cca ata atc ttg act gca gct gaa ctc ttc ttc att cca caa 1056
   Ser Leu Pro Ile Ile Leu Thr Ala Ala Glu Leu Phe Phe Ile Pro Gln
   340 345 350
   cct ctt gtg ttc ctt gtt gtc ttt atg att atc tct gac tca gta gaa 1104
   Pro Leu Val Phe Leu Val Val Phe Met Ile Ile Ser Asp Ser Val Glu
   355 360 365
   tat ggt caa tgg aaa acg gga cac cgt gat gaa tca ctt act ttg tca 1152
   Tyr Gly Gln Trp Lys Thr Gly His Arg Asp Glu Ser Leu Thr Leu Ser
   370 375 380
   gtt cgt cca ctt att gat aaa ctt ggt ggt gcg atg tca aac tgg ctt 1200
   Val Arg Pro Leu Ile Asp Lys Leu Gly Gly Ala Met Ser Asn Trp Leu
   385 390 395 400
   gtt tct aca ttt gcc gta gct gcc ggt atg aca aca ggt gcc tca gca 1248
   Val Ser Thr Phe Ala Val Ala Ala Gly Met Thr Thr Gly Ala Ser Ala
   405 410 415
   tca aca att aca aca cat caa cag ttt atc ttt aag ctt ggc atg ttt 1296
   Ser Thr Ile Thr Thr His Gln Gln Phe Ile Phe Lys Leu Gly Met Phe
   420 425 430
   gct ttc cca gca gca aca atg ctt atc ggt gcc ttc att gtt gct cgt 1344
   Ala Phe Pro Ala Ala Thr Met Leu Ile Gly Ala Phe Ile Val Ala Arg
   435 440 445
   aaa atc act ttg act gaa gca cgt cac gct aaa att gtt gaa gaa ttg 1392
   Lys Ile Thr Leu Thr Glu Ala Arg His Ala Lys Ile Val Glu Glu Leu
   450 455 460
   gaa cat cgc ttt agc gta gca act tct gaa aat gaa gtt aaa gct aac 1440
   Glu His Arg Phe Ser Val Ala Thr Ser Glu Asn Glu Val Lys Ala Asn
   465 470 475 480
   gtc gta tct ctt gta acc cct aca act ggt tat ttg gtt gat ctc tca 1488
   Val Val Ser Leu Val Thr Pro Thr Thr Gly Tyr Leu Val Asp Leu Ser
   485 490 495
   agt gtt aat gat gaa cac ttt gct tca ggt agc atg ggt aaa ggt ttc 1536
   Ser Val Asn Asp Glu His Phe Ala Ser Gly Ser Met Gly Lys Gly Phe
   500 505 510
   gcc att aaa cct act gat gga gct gtc ttt gca cca att agt ggt acc 1584
   Ala Ile Lys Pro Thr Asp Gly Ala Val Phe Ala Pro Ile Ser Gly Thr
   515 520 525
   att cgt caa att ctt cct act cgc cat gca gtt ggt att gaa agt gaa 1632
   Ile Arg Gln Ile Leu Pro Thr Arg His Ala Val Gly Ile Glu Ser Glu
   530 535 540
   gat ggt gtc att gtt ctt atc cac gtt ggc atc gga aca gtt aaa ctt 1680
   Asp Gly Val Ile Val Leu Ile His Val Gly Ile Gly Thr Val Lys Leu
   545 550 555 560
   aat ggt gaa gga ttc att agt tac gta gaa caa ggt gat cat gtt gaa 1728
   Asn Gly Glu Gly Phe Ile Ser Tyr Val Glu Gln Gly Asp His Val Glu
   565 570 575
   gtt gga caa aaa ctt ctt gag ttc tgg tca cca att att gag aaa aat 1776
   Val Gly Gln Lys Leu Leu Glu Phe Trp Ser Pro Ile Ile Glu Lys Asn
   580 585 590
   ggt ctt gat gac aca gta ctt gtc act gta act aat tca gaa aaa ttc 1824
   Gly Leu Asp Asp Thr Val Leu Val Thr Val Thr Asn Ser Glu Lys Phe
           595                 600                     605
   agt gct ttc cat ctt gaa caa aaa gtt gga gaa aag gta gaa gct ttg      1872
   Ser Ala Phe His Leu Glu Gln Lys Val Gly Glu Lys Val Glu Ala Leu
       610                 615                     620
   tct gaa gtt att acc ttc aaa aaa gga gaa taa                          1905
   Ser Glu Val Ile Thr Phe Lys Lys Gly Glu
   625                 630
<210> 7
   <211> 634
   <212> PRT
   <213> Streptococcus thermophilus
<400> 7
   Met Glu Lys Ser Lys Gly Gln Met Lys Ser Arg Leu Ser Tyr Ala Ala
   1               5                   10                  15
   Gly Ala Phe Gly Asn Asp Val Phe Tyr Ala Thr Leu Ser Thr Tyr Phe
               20                  25                  30
   Ile Met Phe Val Thr Thr His Leu Phe Asn Thr Gly Asp Pro Lys Gln
           35                  40                  45
   Asn Ser His Tyr Val Leu Leu Ile Thr Asn Ile Ile Ser Ile Leu Arg
       50                  55                  60
   Ile Leu Glu Val Phe Ile Asp Pro Leu Ile Gly Asn Met Ile Asp Asn
   65                  70                  75                  80
   Thr Asn Thr Lys Tyr Gly Lys Phe Lys Pro Trp Val Val Gly Gly Gly
                   85                  90                  95
   Ile Ile Ser Ser Ile Thr Leu Leu Phe Leu Leu Thr Asp Ile Gly Gly
               100                 105                 110
   Leu Asn Lys Thr Asn Pro Phe Leu Tyr Leu Val Leu Phe Gly Ile Ile
           115                 120                 125
   Tyr Leu Val Met Asp Val Phe Tyr Ser Ile Lys Asp Ile Gly Phe Trp
       130                 135                 140
   Ser Met Ile Pro Ala Leu Ser Leu Asp Ser His Glu Arg Glu Lys Met
   145                 150                 155                 160
   Ala Thr Phe Ala Arg Ile Gly Ser Thr Ile Gly Ala Asn Ile Val Gly
                   165                 170                 175
   Val Ala Ile Met Pro Ile Asp Leu Leu Phe Ser Met Thr His Asn Ser
               180                 185                 190
   Gly Ser Gly Asp Lys Ser Gly Trp Phe Trp Tyr Ala Phe Ile Val Asp
   195 200 205
   Leu Asn Gly Val Ile Thr Ser Ile Ala Val Gly Ile Gly Thr Arg Glu
   210 215 220
   Val Glu Ser Lys Ile His Asp Asn Asn Glu Lys Thr Ser Leu Lys Gln
   225 230 235 240
   Val Phe Lys Val Leu Gly Gln Asn Asp Gln Met Met Trp Leu Ser Leu
   245 250 255
   Gly Tyr Trp Phe Tyr Gly Leu Gly Ile Asn Thr Leu Asn Ala Leu Gln
   260 265 270
   Leu Tyr Tyr Phe Thr Phe Ile Leu Gly Asp Ser Gly Lys Tyr Ser Ile
   275 280 285
   Leu Tyr Gly Leu Asn Thr Val Val Gly Leu Val Ser Val Ser Leu Phe
   290 295 300
   Pro Thr Leu Ala Asp Lys Phe Asn Arg Lys Arg Leu Phe Tyr Gly Cys
   305 310 315 320
   Ile Ala Val Met Leu Gly Gly Ile Gly Ile Phe Ser Ile Ala Gly Thr
   325 330 335
   Ser Leu Pro Ile Ile Leu Thr Ala Ala Glu Leu Phe Phe Ile Pro Gln
   340 345 350
   Pro Leu Val Phe Leu Val Val Phe Met Ile Ile Ser Asp Ser Val Glu
   355 360 365
   Tyr Gly Gln Trp Lys Thr Gly His Arg Asp Glu Ser Leu Thr Leu Ser
   370 375 380
   Val Arg Pro Leu Ile Asp Lys Leu Gly Gly Ala Met Ser Asn Trp Leu
   385 390 395 400
   Val Ser Thr Phe Ala Val Ala Ala Gly Met Thr Thr Gly Ala Ser Ala
   405 410 415
   Ser Thr Ile Thr Thr His Gln Gln Phe Ile Phe Lys Leu Gly Met Phe
   420 425 430
   Ala Phe Pro Ala Ala Thr Met Leu Ile Gly Ala Phe Ile Val Ala Arg
   435 440 445
   Lys Ile Thr Leu Thr Glu Ala Arg His Ala Lys Ile Val Glu Glu Leu
   450 455 460
   Glu His Arg Phe Ser Val Ala Thr Ser Glu Asn Glu Val Lys Ala Asn
   465 470 475 480
   Val Val Ser Leu Val Thr Pro Thr Thr Gly Tyr Leu Val Asp Leu Ser
   485 490 495
   Ser Val Asn Asp Glu His Phe Ala Ser Gly Ser Met Gly Lys Gly Phe
   500 505 510
   Ala Ile Lys Pro Thr Asp Gly Ala Val Phe Ala Pro Ile Ser Gly Thr
   515 520 525
   Ile Arg Gln Ile Leu Pro Thr Arg His Ala Val Gly Ile Glu Ser Glu
   530 535 540
   Asp Gly Val Ile Val Leu Ile His Val Gly Ile Gly Thr Val Lys Leu
   545 550 555 560
   Asn Gly Glu Gly Phe Ile Ser Tyr Val Glu Gln Gly Asp His Val Glu
   565 570 575
   Val Gly Gln Lys Leu Leu Glu Phe Trp Ser Pro Ile Ile Glu Lys Asn
   580 585 590
   Gly Leu Asp Asp Thr Val Leu Val Thr Val Thr Asn Ser Glu Lys Phe
   595 600 605
   Ser Ala Phe His Leu Glu Gln Lys Val Gly Glu Lys Val Glu Ala Leu
   610 615 620
   Ser Glu Val Ile Thr Phe Lys Lys Gly Glu
   625 630
<210> 8
   <211> 115
   <212> DNA
   <213> synthetic
<220>
   <221> misc_feature
   <223> N = 25% each of A, C, G and T; R = 50% each of A and G
<220>
   <221> misc_feature
   <222> (18)..(22)
   <223> n is a, c, g, or t
<220>
   <221> misc_feature
   <222> (38)..(51)
   <223> n is a, c, g, or t
<220>
   <221> misc_feature
   <222> (61)..(64)
   <223> n is a, c, g, or t
<400> 8
   acgactagtg gatccatnnn nnagtttatt cttgacannn nnnnnnnnnn ntgrtataat 60
   nnnnaagtaa taaaatattc ggaggaattt tgaaatggaa aaatctaaag gtcag 115
<210> 9
   <211> 31
   <212> DNA
   <213> synthetic
<220>
   <221> misc_feature
   <223> LacSZ synthetic primer
<400> 9
   ggtactcgag gaagatacta acacactaat g 31
<210> 10
   <211> 2130
   <212> DNA
   <213> Leuconostoc lactis
<220>
   <221> 5'UTR
   <222> (1)..(110)
<220>
   <221> CDS
   <222> (111)..(2030)
<220>
   <221> 3'UTR
   <222> (2031)..(2130)
<400> 10
   gatagggttg aacaacaaaa cgggaatatt attttagcta ctccaagtgc actacattgg     60
   aaccatatca ggttatcgta gcaacaaagg actgatttag gagaaagaat atg aaa       116
                                                          Met Lys
                                                          1
   gac ata act aaa cag aag ttt tct cga aat aag cta gta gaa atg att      164
   Asp Ile Thr Lys Gln Lys Phe Ser Arg Asn Lys Leu Val Glu Met Ile
           5                   10                  15
   tct ttt gca ctt ggt aat cta ggg cac gct gca ttc tat ggt gca tta      212
   Ser Phe Ala Leu Gly Asn Leu Gly His Ala Ala Phe Tyr Gly Ala Leu
       20                  25                  30
   agc act tac ttc atc gtt tat gtc acg agt ggt atg ttt gat ggg tta      260
   Ser Thr Tyr Phe Ile Val Tyr Val Thr Ser Gly Met Phe Asp Gly Leu
   35                  40                  45                  50
   ccg caa tcg gta gca aac aag tta att ggt tta att acg gct ctt gta      308
   Pro Gln Ser Val Ala Asn Lys Leu Ile Gly Leu Ile Thr Ala Leu Val
                   55                  60                  65
   gta att att cgt ttg gcc gaa gta att att gat ccg att ctt gga aat      356
   Val Ile Ile Arg Leu Ala Glu Val Ile Ile Asp Pro Ile Leu Gly Asn
               70                  75                  80
   att gta gat aat aca aaa aca cga tgg gga aaa ttt aaa cct tgg caa      404
   Ile Val Asp Asn Thr Lys Thr Arg Trp Gly Lys Phe Lys Pro Trp Gln
           85                  90                  95
   gta att ggc gcg gtt gtt agt tca gtg tta tta gtt gta ata ttt act      452
   Val Ile Gly Ala Val Val Ser Ser Val Leu Leu Val Val Ile Phe Thr
       100                 105                 110
   gga att ttt ggg ttg gct cat att aat tgg att gct ttt gcg atc gtt      500
   Gly Ile Phe Gly Leu Ala His Ile Asn Trp Ile Ala Phe Ala Ile Val
   115                 120                 125                 130
   ttc aca gtt tta ttt att tta cta gat att ttt tat tca ttt gca gat      548
   Phe Thr Val Leu Phe Ile Leu Leu Asp Ile Phe Tyr Ser Phe Ala Asp
                   135                 140                 145
   gtt gcc tat tgg gga atg gtg ccg gct ata tct gaa gac agt aaa gag      596
   Val Ala Tyr Trp Gly Met Val Pro Ala Ile Ser Glu Asp Ser Lys Glu
               150                 155                 160
   aga gga att ttt act tcc tta ggg agt ttt act ggt tct att ggt tgg      644
   Arg Gly Ile Phe Thr Ser Leu Gly Ser Phe Thr Gly Ser Ile Gly Trp
           165                 170                 175
   aat ggt ctt acg atg att gtt gtt ccg gtc acc aca tac ttc act ttt      692
   Asn Gly Leu Thr Met Ile Val Val Pro Val Thr Thr Tyr Phe Thr Phe
       180                 185                 190
   atc gca act gga aaa cac gaa caa ggg cct tcg ggg tgg ttt gga ttt      740
   Ile Ala Thr Gly Lys His Glu Gln Gly Pro Ser Gly Trp Phe Gly Phe
   195                 200                 205                 210
   tca ata gtt gtt tcc att gta gcc gtt tta tca gct ttg gca gta gcc      788
   Ser Ile Val Val Ser Ile Val Ala Val Leu Ser Ala Leu Ala Val Ala
                   215                 220                 225
   ttt gga act aaa gaa aaa gat aac ttg att cga aat gct gca aca aaa      836
   Phe Gly Thr Lys Glu Lys Asp Asn Leu Ile Arg Asn Ala Ala Thr Lys
               230                 235                 240
   aaa aca agc att aaa gat gta ttt tca ggt att att cat aat gac caa      884
   Lys Thr Ser Ile Lys Asp Val Phe Ser Gly Ile Ile His Asn Asp Gln
           245                 250                 255
   att tta tgg atc agc tta gca tat cta atg tat tca cta gcg tat gtt      932
   Ile Leu Trp Ile Ser Leu Ala Tyr Leu Met Tyr Ser Leu Ala Tyr Val
       260                 265                 270
   gtg act aat ggt gtt tta ttt tac ttc ttt aag ttt gta cta gga aaa      980
   Val Thr Asn Gly Val Leu Phe Tyr Phe Phe Lys Phe Val Leu Gly Lys
   275                 280                 285                 290
   cca aac gaa ttt tgg att gct ggt gct att gca aca gta ata ggg ttc     1028
   Pro Asn Glu Phe Trp Ile Ala Gly Ala Ile Ala Thr Val Ile Gly Phe
                   295                 300                 305
   tct act gct ccg ttg tat ccc gtt cta aat aag ttt atc acc agg aaa     1076
   Ser Thr Ala Pro Leu Tyr Pro Val Leu Asn Lys Phe Ile Thr Arg Lys
               310                 315                 320
   gtg tta ttt agt att ggg caa atg gca atg atc cta tca tat ctg ttt     1124
   Val Leu Phe Ser Ile Gly Gln Met Ala Met Ile Leu Ser Tyr Leu Phe
           325                 330                 335
   ttt ata ttc gga aaa aca aat atg atg atg gtt aca ata gga ctt att     1172
   Phe Ile Phe Gly Lys Thr Asn Met Met Met Val Thr Ile Gly Leu Ile
       340                 345                 350
   ttg ttt aat ttt act ttt gcg caa ctg gtt gtt gtc tta tca cta act     1220
   Leu Phe Asn Phe Thr Phe Ala Gln Leu Val Val Val Leu Ser Leu Thr
   355                 360                 365                 370
   gac tct att gaa tac gga caa tta aaa aat ggt aat cgc aac gaa gct      1268
   Asp Ser Ile Glu Tyr Gly Gln Leu Lys Asn Gly Asn Arg Asn Glu Ala
                   375                 380                 385
   gtt gtc ttg gcg gtt cga cct atg tta gat aag ata act ggt gct ttt      1316
   Val Val Leu Ala Val Arg Pro Met Leu Asp Lys Ile Thr Gly Ala Phe
               390                 395                 400
   tca aac gga ctc gtc gga gca att gca att acg gcg ggc atg act gga      1364
   Ser Asn Gly Leu Val Gly Ala Ile Ala Ile Thr Ala Gly Met Thr Gly
           405                 410                 415
   tca gct acc gca gga gat att agt gca tca aaa att aat acc ttt gaa      1412
   Ser Ala Thr Ala Gly Asp Ile Ser Ala Ser Lys Ile Asn Thr Phe Glu
       420                 425                 430
   att tat gct ttt tac aca cca ctt ctt ttt tct att cta gct ttg gtt      1460
   Ile Tyr Ala Phe Tyr Thr Pro Leu Leu Phe Ser Ile Leu Ala Leu Val
   435                 440                 445                 450
   ata ttc ttg tgg aaa gtt aaa att acc gag aaa aaa cat gcc gaa atc      1508
   Ile Phe Leu Trp Lys Val Lys Ile Thr Glu Lys Lys His Ala Glu Ile
                   455                 460                 465
   gtt att gaa ctg gaa aaa act tta tca agt ggc gct aaa aaa gct aat      1556
   Val Ile Glu Leu Glu Lys Thr Leu Ser Ser Gly Ala Lys Lys Ala Asn
               470                 475                 480
   act tct gaa gtg aat gtt gaa ctt gaa gaa ata ttt gca cct gcc tca      1604
   Thr Ser Glu Val Asn Val Glu Leu Glu Glu Ile Phe Ala Pro Ala Ser
           485                 490                 495
   gga caa aaa aag cta ctt aat gag gtt gat ggc aac aca ctt act ggt      1652
   Gly Gln Lys Lys Leu Leu Asn Glu Val Asp Gly Asn Thr Leu Thr Gly
       500                 505                 510
   att ggt ttt gct att gat ccg gaa gaa ggt aat tta ttt gca cca ttt      1700
   Ile Gly Phe Ala Ile Asp Pro Glu Glu Gly Asn Leu Phe Ala Pro Phe
   515                 520                 525                 530
   gat ggc aaa gtc gac ttt act ttt tct aca aaa cat gtt ttg ggt gtt      1748
   Asp Gly Lys Val Asp Phe Thr Phe Ser Thr Lys His Val Leu Gly Val
                   535                 540                 545
   gta tct aac aac gga tta aaa gca att att cat gta gga ata ggc act      1796
   Val Ser Asn Asn Gly Leu Lys Ala Ile Ile His Val Gly Ile Gly Thr
               550                 555                 560
   atc aat atg cga ggt gcg ggg ttt gtg tca cat tat gtg gac ggc caa      1844
   Ile Asn Met Arg Gly Ala Gly Phe Val Ser His Tyr Val Asp Gly Gln
           565                 570                 575
   ttg ttc aaa aag gga gat ctt ttg atg acc ttt gat aag aaa tta att      1892
   Leu Phe Lys Lys Gly Asp Leu Leu Met Thr Phe Asp Lys Lys Leu Ile
       580                 585                 590
   acg aaa aac ggg tat caa gat gac att att atg tac ttt acc caa cca      1940
   Thr Lys Asn Gly Tyr Gln Asp Asp Ile Ile Met Tyr Phe Thr Gln Pro
   595                 600                 605                 610
   gaa aac att ata gat gtt caa caa att gat aat cgt gtc gtc aaa caa      1988
   Glu Asn Ile Ile Asp Val Gln Gln Ile Asp Asn Arg Val Val Lys Gln
                   615                 620                 625
   ggg gag aag ata gca aaa tta aca ttc agg agt gaa aga taa              2030
   Gly Glu Lys Ile Ala Lys Leu Thr Phe Arg Ser Glu Arg
               630                 635
   cttaaaggtg ggccggaaag tctgaactta aaaagagatt ggtgggtagt aatcactaca 2090
   atcgcaataa ctagttttct ttcattttca gtatcggctt 2130
<210> 11
   <211> 639
   <212> PRT
   <213> Leuconostoc lactis
<400> 11
   Met Lys Asp Ile Thr Lys Gln Lys Phe Ser Arg Asn Lys Leu Val Glu
   1 5 10 15
   Met Ile Ser Phe Ala Leu Gly Asn Leu Gly His Ala Ala Phe Tyr Gly
   20 25 30
   Ala Leu Ser Thr Tyr Phe Ile Val Tyr Val Thr Ser Gly Met Phe Asp
   35 40 45
   Gly Leu Pro Gln Ser Val Ala Asn Lys Leu Ile Gly Leu Ile Thr Ala
   50 55 60
   Leu Val Val Ile Ile Arg Leu Ala Glu Val Ile Ile Asp Pro Ile Leu
   65 70 75 80
   Gly Asn Ile Val Asp Asn Thr Lys Thr Arg Trp Gly Lys Phe Lys Pro
   85 90 95
   Trp Gln Val Ile Gly Ala Val Val Ser Ser Val Leu Leu Val Val Ile
   100 105 110
   Phe Thr Gly Ile Phe Gly Leu Ala His Ile Asn Trp Ile Ala Phe Ala
   115 120 125
   Ile Val Phe Thr Val Leu Phe Ile Leu Leu Asp Ile Phe Tyr Ser Phe
   130 135 140
   Ala Asp Val Ala Tyr Trp Gly Met Val Pro Ala Ile Ser Glu Asp Ser
   145 150 155 160
   Lys Glu Arg Gly Ile Phe Thr Ser Leu Gly Ser Phe Thr Gly Ser Ile
   165 170 175
   Gly Trp Asn Gly Leu Thr Met Ile Val Val Pro Val Thr Thr Tyr Phe
   180 185 190
   Thr Phe Ile Ala Thr Gly Lys His Glu Gln Gly Pro Ser Gly Trp Phe
   195 200 205
   Gly Phe Ser Ile Val Val Ser Ile Val Ala Val Leu Ser Ala Leu Ala
   210 215 220
   Val Ala Phe Gly Thr Lys Glu Lys Asp Asn Leu Ile Arg Asn Ala Ala
   225                 230                 235                 240
   Thr Lys Lys Thr Ser Ile Lys Asp Val Phe Ser Gly Ile Ile His Asn
                   245                 250                 255
   Asp Gln Ile Leu Trp Ile Ser Leu Ala Tyr Leu Met Tyr Ser Leu Ala
               260                 265                 270
   Tyr Val Val Thr Asn Gly Val Leu Phe Tyr Phe Phe Lys Phe Val Leu
           275                 280                 285
   Gly Lys Pro Asn Glu Phe Trp Ile Ala Gly Ala Ile Ala Thr Val Ile
       290                 295                 300
   Gly Phe Ser Thr Ala Pro Leu Tyr Pro Val Leu Asn Lys Phe Ile Thr
   305                 310                 315                 320
   Arg Lys Val Leu Phe Ser Ile Gly Gln Met Ala Met Ile Leu Ser Tyr
                   325                 330                 335
   Leu Phe Phe Ile Phe Gly Lys Thr Asn Met Met Met Val Thr Ile Gly
               340                 345                 350
   Leu Ile Leu Phe Asn Phe Thr Phe Ala Gln Leu Val Val Val Leu Ser
           355                 360                 365
   Leu Thr Asp Ser Ile Glu Tyr Gly Gln Leu Lys Asn Gly Asn Arg Asn
       370                 375                 380
   Glu Ala Val Val Leu Ala Val Arg Pro Met Leu Asp Lys Ile Thr Gly
   385                 390                 395                 400
   Ala Phe Ser Asn Gly Leu Val Gly Ala Ile Ala Ile Thr Ala Gly Met
                   405                 410                 415
   Thr Gly Ser Ala Thr Ala Gly Asp Ile Ser Ala Ser Lys Ile Asn Thr
               420                 425                 430
   Phe Glu Ile Tyr Ala Phe Tyr Thr Pro Leu Leu Phe Ser Ile Leu Ala
           435                 440                 445
   Leu Val Ile Phe Leu Trp Lys Val Lys Ile Thr Glu Lys Lys His Ala
       450                 455                 460
   Glu Ile Val Ile Glu Leu Glu Lys Thr Leu Ser Ser Gly Ala Lys Lys
   465                 470                 475                 480
   Ala Asn Thr Ser Glu Val Asn Val Glu Leu Glu Glu Ile Phe Ala Pro
                   485                 490                 495
   Ala Ser Gly Gln Lys Lys Leu Leu Asn Glu Val Asp Gly Asn Thr Leu
   500 505 510
   Thr Gly Ile Gly Phe Ala Ile Asp Pro Glu Glu Gly Asn Leu Phe Ala
   515 520 525
   Pro Phe Asp Gly Lys Val Asp Phe Thr Phe Ser Thr Lys His Val Leu
   530 535 540
   Gly Val Val Ser Asn Asn Gly Leu Lys Ala Ile Ile His Val Gly Ile
   545 550 555 560
   Gly Thr Ile Asn Met Arg Gly Ala Gly Phe Val Ser His Tyr Val Asp
   565 570 575
   Gly Gln Leu Phe Lys Lys Gly Asp Leu Leu Met Thr Phe Asp Lys Lys
   580 585 590
   Leu Ile Thr Lys Asn Gly Tyr Gln Asp Asp Ile Ile Met Tyr Phe Thr
   595 600 605
   Gln Pro Glu Asn Ile Ile Asp Val Gln Gln Ile Asp Asn Arg Val Val
   610 615 620
   Lys Gln Gly Glu Lys Ile Ala Lys Leu Thr Phe Arg Ser Glu Arg
   625 630 635
<210> 12
   <211> 3195
   <212> DNA
   <213> Leuconostoc lactis
<220>
   <221> 5'UTR
   <222> (1)..(276)
<220>
   <221> CDS
   <222> (277)..(2157)
   <223> LacL CDS
<220>
   <221> 3'UTR
   <222> (2157)..(3195)
<400> 12
   gtcgaccgaa ggtccgtatt catcggatag atcagtatat gaacggcctt gattgtgcat      60
   ctcaacaaga gactgcttaa attctttttt gtagcgaatc atattaaaaa atgtctccta     120
   tgcgattagt ttacagggca gaagaaaatc tgtccgtaaa tctagcataa gagcatttag     180
   ttaaaattta actaaatgac atatataaat ttaatatttt gttttataat aattgtaagc     240
   gttttttatt tatgtaactt tgaaaggatc ttcctc atg caa gct aat ctt caa       294
                                           Met Gln Ala Asn Leu Gln
                                           1               5
   tgg tta gat gac cca gaa gtc ttc cgg gtc aac caa tta cct gca cat 342
   Trp Leu Asp Asp Pro Glu Val Phe Arg Val Asn Gln Leu Pro Ala His
   10 15 20
   agt gat cac cat tat tat cac gac aca gca gaa ttc aaa acg ggt agt 390
   Ser Asp His His Tyr Tyr His Asp Thr Ala Glu Phe Lys Thr Gly Ser
   25 30 35
   cgc ttc atc aag agt ctc aat ggc gct tgg cgt ttt aac ttc gcc aag 438
   Arg Phe Ile Lys Ser Leu Asn Gly Ala Trp Arg Phe Asn Phe Ala Lys
   40 45 50
   aca ccg gct gaa cgc cca gtt gat ttt tat caa ccc gat ttc gat gca 486
   Thr Pro Ala Glu Arg Pro Val Asp Phe Tyr Gln Pro Asp Phe Asp Ala
   55 60 65 70
   acc gac ttt gat acg att caa gtt ccc ggt cat att gaa cta gcc ggc 534
   Thr Asp Phe Asp Thr Ile Gln Val Pro Gly His Ile Glu Leu Ala Gly
   75 80 85
   tat ggt caa att caa tac att aac acg cta tac cca tgg gaa ggt aaa 582
   Tyr Gly Gln Ile Gln Tyr Ile Asn Thr Leu Tyr Pro Trp Glu Gly Lys
   90 95 100
   att tac cgt cgc cca ccg tat acc ctc aat caa gat caa tta aca cca 630
   Ile Tyr Arg Arg Pro Pro Tyr Thr Leu Asn Gln Asp Gln Leu Thr Pro
   105 110 115
   ggc cta ttc agc gac gct gcg gac aac acc gtc ggc tcg tac ctc aaa 678
   Gly Leu Phe Ser Asp Ala Ala Asp Asn Thr Val Gly Ser Tyr Leu Lys
   120 125 130
   acc ttc gat ctc gac gat gtt ttt aaa ggg caa cgt att atc att cag 726
   Thr Phe Asp Leu Asp Asp Val Phe Lys Gly Gln Arg Ile Ile Ile Gln
   135 140 145 150
   ttc caa ggg gta gaa gaa gcc ctg tac gtc tgg tta aat ggc cat ttt 774
   Phe Gln Gly Val Glu Glu Ala Leu Tyr Val Trp Leu Asn Gly His Phe
   155 160 165
   att ggc tac tct gaa gat agt ttc acc cct tca gaa ttt gat ttg acg 822
   Ile Gly Tyr Ser Glu Asp Ser Phe Thr Pro Ser Glu Phe Asp Leu Thr
   170 175 180
   ccg tat att cag gac caa ggt aac gtt tta gcg gtt cgg gtc tac aaa 870
   Pro Tyr Ile Gln Asp Gln Gly Asn Val Leu Ala Val Arg Val Tyr Lys
   185 190 195
   cac agt act gct gcc ttt att gaa gac caa gat atg ttc cgt ttc tct 918
   His Ser Thr Ala Ala Phe Ile Glu Asp Gln Asp Met Phe Arg Phe Ser
   200 205 210
   ggt att ttc cgt gac gtc aat ata ctg gcg gag cct gct agc cat att 966
   Gly Ile Phe Arg Asp Val Asn Ile Leu Ala Glu Pro Ala Ser His Ile
   215 220 225 230
   act gat ttg gac atc cga cca gtt cca aat gcc aat ctc aaa agt ggt 1014
   Thr Asp Leu Asp Ile Arg Pro Val Pro Asn Ala Asn Leu Lys Ser Gly
   235 240 245
   gag ctc aac atc act act aaa gta acc ggc gaa cca gcc act tta gcg 1062
   Glu Leu Asn Ile Thr Thr Lys Val Thr Gly Glu Pro Ala Thr Leu Ala
   250 255 260
   ctg acc gtt aaa gac cat gac ggg cga gta ctg acg agt caa acg caa 1110
   Leu Thr Val Lys Asp His Asp Gly Arg Val Leu Thr Ser Gln Thr Gln
   265 270 275
   acc ggt agt ggc agt gta acc ttt gat act atg tta ttc gac caa ctg 1158
   Thr Gly Ser Gly Ser Val Thr Phe Asp Thr Met Leu Phe Asp Gln Leu
   280 285 290
   cac ttg tgg tca cca caa acg ccg tat ctc tat caa ttg aca att gaa 1206
   His Leu Trp Ser Pro Gln Thr Pro Tyr Leu Tyr Gln Leu Thr Ile Glu
   295 300 305 310
   gtt tac gat gct gat cac caa ctc ttg gaa gtc gtc cca tat cag ttt 1254
   Val Tyr Asp Ala Asp His Gln Leu Leu Glu Val Val Pro Tyr Gln Phe
   315 320 325
   ggg ttc cgg acg gtc gag ctg cgc gat gac aaa gtc att tac gtc aac 1302
   Gly Phe Arg Thr Val Glu Leu Arg Asp Asp Lys Val Ile Tyr Val Asn
   330 335 340
   aat aaa cgg ttg gtg atc aac ggg gtt aac cgg cac gaa tgg aac gcc 1350
   Asn Lys Arg Leu Val Ile Asn Gly Val Asn Arg His Glu Trp Asn Ala
   345 350 355
   cac acc ggt cgc gtt atc agt atg gct gat atg cgc gct gat atc caa 1398
   His Thr Gly Arg Val Ile Ser Met Ala Asp Met Arg Ala Asp Ile Gln
   360 365 370
   acc atg tta gct aac aat atc aat gcc gat cgg acc tgc cat tat cct 1446
   Thr Met Leu Ala Asn Asn Ile Asn Ala Asp Arg Thr Cys His Tyr Pro
   375 380 385 390
   gat caa tta cct tgg tat caa tta tgt gac gag gcc ggt atc tac cta 1494
   Asp Gln Leu Pro Trp Tyr Gln Leu Cys Asp Glu Ala Gly Ile Tyr Leu
   395 400 405
   atg gcc gaa acc aac ctc gaa tcg cac ggg tca tgg caa aag atg ggg 1542
   Met Ala Glu Thr Asn Leu Glu Ser His Gly Ser Trp Gln Lys Met Gly
   410 415 420
   gct atc gag cct tct tac aat gtt cct ggc gat aat cca cac tgg cca 1590
   Ala Ile Glu Pro Ser Tyr Asn Val Pro Gly Asp Asn Pro His Trp Pro
   425 430 435
   gca gcg gtg atc gac cgg gcc cgt tca aac tac gaa tgg ttt aaa aac 1638
   Ala Ala Val Ile Asp Arg Ala Arg Ser Asn Tyr Glu Trp Phe Lys Asn
   440 445 450
   cac cca tca atc att ttt tgg tca ctt ggc aat gaa tcg tat gct ggc 1686
   His Pro Ser Ile Ile Phe Trp Ser Leu Gly Asn Glu Ser Tyr Ala Gly
   455 460 465 470
   gaa gat atc gcg gcg atg cag gct ttt tat aaa gaa cac gat gat tca 1734
   Glu Asp Ile Ala Ala Met Gln Ala Phe Tyr Lys Glu His Asp Asp Ser
   475 480 485
   cga ctc gtc cac tac gaa ggc gtt ttc tac aca cca gaa tta aaa gat 1782
   Arg Leu Val His Tyr Glu Gly Val Phe Tyr Thr Pro Glu Leu Lys Asp
   490 495 500
   cgc att tct gat gtt gaa agt cgg atg tac gaa aag ccc caa aat att 1830
   Arg Ile Ser Asp Val Glu Ser Arg Met Tyr Glu Lys Pro Gln Asn Ile
   505 510 515
   gta gct tac ttg gaa gat aac cca acc aaa cct ttc cta aat tgt gaa 1878
   Val Ala Tyr Leu Glu Asp Asn Pro Thr Lys Pro Phe Leu Asn Cys Glu
   520 525 530
   tat atg cat gac atg ggg aat tct ctg ggc ggt atg caa tca tat aat 1926
   Tyr Met His Asp Met Gly Asn Ser Leu Gly Gly Met Gln Ser Tyr Asn
   535 540 545 550
   gat ttg atc gac aag tat cca atg tat caa ggt ggc ttt att tgg gac      1974
   Asp Leu Ile Asp Lys Tyr Pro Met Tyr Gln Gly Gly Phe Ile Trp Asp
                   555                 560                 565
   ttt att gat caa gca ctc ttc gtt cat gac cca atc acc gac caa gac      2022
   Phe Ile Asp Gln Ala Leu Phe Val His Asp Pro Ile Thr Asp Gln Asp
               570                 575                 580
   gtg ctc cgg tat ggc ggt gat ttc gac gaa cgc cac tcc gat tat gca      2070
   Val Leu Arg Tyr Gly Gly Asp Phe Asp Glu Arg His Ser Asp Tyr Ala
           585                 590                 595
   ttc tcc ggt aac ggc tta atg ttt gcc gac cgg aca cca aaa cca gca      2118
   Phe Ser Gly Asn Gly Leu Met Phe Ala Asp Arg Thr Pro Lys Pro Ala
       600                 605                 610
   atg caa gag gtg aaa tat tat tat ggc tta cac aaa taa tcaactacac       2167
   Met Gln Glu Val Lys Tyr Tyr Tyr Gly Leu His Lys
   615                 620                 625
   gttatttacg gcgacgggag cttaggacta cagggggcta atttccacta cctctttagc   2227
   tacgaacgtg ggggacttga atcactcgtc gtcaacgata aagagtggct ctatcgtaca    2287
   cccacgccca tgttttggcg ggcgacaacc gataatgatc acggtagcgg cttttcagtc    2347
   aaatccgcac agtggtacgc ggccgataag ttctcaactt gtcaagatat cgaattgacg    2407
   gttgacgacc aaccagtcac accgttacca atcgcgccac tcaataacaa atacacggat    2467
   cacgaaatcg ccacgaaagt ctctctggct taccacttcg ttaccacgac cgttcctagt    2527
   accatcgtca cagtgactta tacggtgaca gcagacggtc agatcaatat cgccacccat    2587
   tatagcggtc agtctgattt gccagagcta cccgcatttg gtctgcggtt tatcatgcca    2647
   actaccgcga ccggcttcga ctataccggt ttgtccggtg agacttatcc tgaccggctg    2707
   gctggcgcaa cacacgggca attccacgtt gacagtctgc cagtcacacc atacttggtc    2767
   ccacaagaat gcggcatgca catgcaaact gaacaagtga cagtaacgcg atcaacaaca    2827
   caaaataacg ctgaccacga caacacacca ttcagtttaa catttagcca aaccgatgca    2887
   ccattcgcct tcagctgcct tccctatacc gctgctgaac tagaaaacgc aacacacatg    2947
   gaagaattac cattagcacg gcgaacggtc ttatcaatct acggtgccgt tcgtggggtc    3007
   ggtggcattg acagttgggg aacggacgta gaagccccat atcatatcct cgctaatcaa    3067
   gacattgact tcagctttaa cattcatttc taaaaattaa tttgatttca aaagaatcgc    3127
   tccggcagtt atttgccaga gcgttctttt aatatgtttt atggctgagc tttagtcctt    3187
   tgaagtga                                                             3195
<210> 13
   <211> 626
   <212> PRT
   <213> Leuconostoc lactis
<400> 13
   Met Gln Ala Asn Leu Gln Trp Leu Asp Asp Pro Glu Val Phe Arg Val
   1               5                   10                  15
   Asn Gln Leu Pro Ala His Ser Asp His His Tyr Tyr His Asp Thr Ala
   20 25 30
   Glu Phe Lys Thr Gly Ser Arg Phe Ile Lys Ser Leu Asn Gly Ala Trp
   35 40 45
   Arg Phe Asn Phe Ala Lys Thr Pro Ala Glu Arg Pro Val Asp Phe Tyr
   50 55 60
   Gln Pro Asp Phe Asp Ala Thr Asp Phe Asp Thr Ile Gln Val Pro Gly
   65 70 75 80
   His Ile Glu Leu Ala Gly Tyr Gly Gln Ile Gln Tyr Ile Asn Thr Leu
   85 90 95
   Tyr Pro Trp Glu Gly Lys Ile Tyr Arg Arg Pro Pro Tyr Thr Leu Asn
   100 105 110
   Gln Asp Gln Leu Thr Pro Gly Leu Phe Ser Asp Ala Ala Asp Asn Thr
   115 120 125
   Val Gly Ser Tyr Leu Lys Thr Phe Asp Leu Asp Asp Val Phe Lys Gly
   130 135 140
   Gln Arg Ile Ile Ile Gln Phe Gln Gly Val Glu Glu Ala Leu Tyr Val
   145 150 155 160
   Trp Leu Asn Gly His Phe Ile Gly Tyr Ser Glu Asp Ser Phe Thr Pro
   165 170 175
   Ser Glu Phe Asp Leu Thr Pro Tyr Ile Gln Asp Gln Gly Asn Val Leu
   180 185 190
   Ala Val Arg Val Tyr Lys His Ser Thr Ala Ala Phe Ile Glu Asp Gln
   195 200 205
   Asp Met Phe Arg Phe Ser Gly Ile Phe Arg Asp Val Asn Ile Leu Ala
   210 215 220
   Glu Pro Ala Ser His Ile Thr Asp Leu Asp Ile Arg Pro Val Pro Asn
   225 230 235 240
   Ala Asn Leu Lys Ser Gly Glu Leu Asn Ile Thr Thr Lys Val Thr Gly
   245 250 255
   Glu Pro Ala Thr Leu Ala Leu Thr Val Lys Asp His Asp Gly Arg Val
   260 265 270
   Leu Thr Ser Gln Thr Gln Thr Gly Ser Gly Ser Val Thr Phe Asp Thr
   275 280 285
   Met Leu Phe Asp Gln Leu His Leu Trp Ser Pro Gln Thr Pro Tyr Leu
   290 295 300
   Tyr Gln Leu Thr Ile Glu Val Tyr Asp Ala Asp His Gln Leu Leu Glu
   305 310 315 320
   Val Val Pro Tyr Gln Phe Gly Phe Arg Thr Val Glu Leu Arg Asp Asp
   325 330 335
   Lys Val Ile Tyr Val Asn Asn Lys Arg Leu Val Ile Asn Gly Val Asn
   340 345 350
   Arg His Glu Trp Asn Ala His Thr Gly Arg Val Ile Ser Met Ala Asp
   355 360 365
   Met Arg Ala Asp Ile Gln Thr Met Leu Ala Asn Asn Ile Asn Ala Asp
   370 375 380
   Arg Thr Cys His Tyr Pro Asp Gln Leu Pro Trp Tyr Gln Leu Cys Asp
   385 390 395 400
   Glu Ala Gly Ile Tyr Leu Met Ala Glu Thr Asn Leu Glu Ser His Gly
   405 410 415
   Ser Trp Gln Lys Met Gly Ala Ile Glu Pro Ser Tyr Asn Val Pro Gly
   420 425 430
   Asp Asn Pro His Trp Pro Ala Ala Val Ile Asp Arg Ala Arg Ser Asn
   435 440 445
   Tyr Glu Trp Phe Lys Asn His Pro Ser Ile Ile Phe Trp Ser Leu Gly
   450 455 460
   Asn Glu Ser Tyr Ala Gly Glu Asp Ile Ala Ala Met Gln Ala Phe Tyr
   465 470 475 480
   Lys Glu His Asp Asp Ser Arg Leu Val His Tyr Glu Gly Val Phe Tyr
   485 490 495
   Thr Pro Glu Leu Lys Asp Arg Ile Ser Asp Val Glu Ser Arg Met Tyr
   500 505 510
   Glu Lys Pro Gln Asn Ile Val Ala Tyr Leu Glu Asp Asn Pro Thr Lys
   515 520 525
   Pro Phe Leu Asn Cys Glu Tyr Met His Asp Met Gly Asn Ser Leu Gly
   530 535 540
   Gly Met Gln Ser Tyr Asn Asp Leu Ile Asp Lys Tyr Pro Met Tyr Gln
   545 550 555 560
   Gly Gly Phe Ile Trp Asp Phe Ile Asp Gln Ala Leu Phe Val His Asp
   565 570 575
   Pro Ile Thr Asp Gln Asp Val Leu Arg Tyr Gly Gly Asp Phe Asp Glu
   580 585 590
   Arg His Ser Asp Tyr Ala Phe Ser Gly Asn Gly Leu Met Phe Ala Asp
   595 600 605
   Arg Thr Pro Lys Pro Ala Met Gln Glu Val Lys Tyr Tyr Tyr Gly Leu
   610 615 620
   His Lys
   625
<210> 14
   <211> 3195
   <212> DNA
   <213> Leuconostoc lactis
<220>
   <221> 5'UTR
   <222> (1)..(2140)
<220>
   <221> CDS
   <222> (2141)..(3100)
<220>
   <221> 3'UTR
   <222> (3101)..(3195)
   <223> CDS: LacM gene
<400> 14
   gtcgaccgaa ggtccgtatt catcggatag atcagtatat gaacggcctt gattgtgcat   60
   ctcaacaaga gactgcttaa attctttttt gtagcgaatc atattaaaaa atgtctccta   120
   tgcgattagt ttacagggca gaagaaaatc tgtccgtaaa tctagcataa gagcatttag   180
   ttaaaattta actaaatgac atatataaat ttaatatttt gttttataat aattgtaagc   240
   gtttttattt tatgtaactt tgaaaggatc ttcctcatgc aagctaatct tcaatggtta   300
   gatgacccag aagtcttccg ggtcaaccaa ttacctgcac atagtgatca ccattattat   360
   cacgacacag cagaattcaa aacgggtagt cgcttcatca agagtctcaa tggcgcttgg   420
   cgttttaact tcgccaagac accggctgaa cgcccagttg atttttatca acccgatttc   480
   gatgcaaccg actttgatac gattcaagtt cccggtcata ttgaactagc cggctatggt   540
   caaattcaat acattaacac gctataccca tgggaaggta aaatttaccg tcgcccaccg   600
   tataccctca atcaagatca attaacacca ggcctattca gcgacgctgc ggacaacacc   660
   gtcggctcgt acctcaaaac cttcgatctc gacgatgttt ttaaagggca acgtattatc   720
   attcagttcc aaggggtaga agaagccctg tacgtctggt taaatggcca ttttattggc   780
   tactctgaag atagtttcac cccttcagaa tttgatttga cgccgtatat tcaggaccaa   840
   ggtaacgttt tagcggttcg ggtctacaaa cacagtactg ctgcctttat tgaagaccaa    900
   gatatgttcc gtttctctgg tattttccgt gacgtcaata tactggcgga gcctgctagc    960
   catattactg atttggacat ccgaccagtt ccaaatgcca atctcaaaag tggtgagctc   1020
   aacatcacta ctaaagtaac cggcgaacca gccactttag cgctgaccgt taaagaccat   1080
   gacgggcgag tactgacgag tcaaacgcaa accggtagtg gcagtgtaac ctttgatact   1140
   atgttattcg accaactgca cttgtggtca ccacaaacgc cgtatctcta tcaattgaca   1200
   attgaagttt acgatgctga tcaccaactc ttggaagtcg tcccatatca gtttgggttc   1260
   cggacggtcg agctgcgcga tgacaaagtc atttacgtca acaataaacg gttggtgatc   1320
   aacggggtta accggcacga atggaacgcc cacaccggtc gcgttatcag tatggctgat   1380
   atgcgcgctg atatccaaac catgttagct aacaatatca atgccgatcg gacctgccat   1440
   tatcctgatc aattaccttg gtatcaatta tgtgacgagg ccggtatcta cctaatggcc   1500
   gaaaccaacc tcgaatcgca cgggtcatgg caaaagatgg gggctatcga gccttcttac   1560
   aatgttcctg gcgataatcc acactggcca gcagcggtga tcgaccgggc ccgttcaaac   1620
   tacgaatggt ttaaaaacca cccatcaatc attttttggt cacttggcaa tgaatcgtat   1680
   gctggcgaag atatcgcggc gatgcaggct ttttataaag aacacgatga ttcacgactc   1740
   gtccactacg aaggcgtttt ctacacacca gaattaaaag atcgcatttc tgatgttgaa   1800
   agtcggatgt acgaaaagcc ccaaaatatt gtagcttact tggaagataa cccaaccaaa   1860
   cctttcctaa attgtgaata tatgcatgac atggggaatt ctctgggcgg tatgcaatca   1920
   tataatgatt tgatcgacaa gtatccaatg tatcaaggtg gctttatttg ggactttatt   1980
   gatcaagcac tcttcgttca tgacccaatc accgaccaag acgtgctccg gtatggcggt   2040
   gatttcgacg aacgccactc cgattatgca ttctccggta acggcttaat gtttgccgac   2100
   cggacaccaa aaccagcaat gcaagaggtg aaatattatt atg gct tac aca aat     2155
                                             Met Ala Tyr Thr Asn
                                             1               5
   aat caa cta cac gtt att tac ggc gac ggg agc tta gga cta cag ggg     2203
   Asn Gln Leu His Val Ile Tyr Gly Asp Gly Ser Leu Gly Leu Gln Gly
                   10                  15                  20
   gct aat ttc cac tac ctc ttt agc tac gaa cgt ggg gga ctt gaa tca     2251
   Ala Asn Phe His Tyr Leu Phe Ser Tyr Glu Arg Gly Gly Leu Glu Ser
               25                  30                  35
   ctc gtc gtc aac gat aaa gag tgg ctc tat cgt aca ccc acg ccc atg     2299
   Leu Val Val Asn Asp Lys Glu Trp Leu Tyr Arg Thr Pro Thr Pro Met
           40                  45                  50
   ttt tgg cgg gcg aca acc gat aat gat cac ggt agc ggc ttt tca gtc     2347
   Phe Trp Arg Ala Thr Thr Asp Asn Asp His Gly Ser Gly Phe Ser Val
       55                  60                  65
   aaa tcc gca cag tgg tac gcg gcc gat aag ttc tca act tgt caa gat     2395
   Lys Ser Ala Gln Trp Tyr Ala Ala Asp Lys Phe Ser Thr Cys Gln Asp
   70                  75                  80                  85
   atc gaa ttg acg gtt gac gac caa cca gtc aca ccg tta cca atc gcg     2443
   Ile Glu Leu Thr Val Asp Asp Gln Pro Val Thr Pro Leu Pro Ile Ala
                   90                  95                  100
   cca ctc aat aac aaa tac acg gat cac gaa atc gcc acg aaa gtc tct     2491
   Pro Leu Asn Asn Lys Tyr Thr Asp His Glu Ile Ala Thr Lys Val Ser
               105                 110                 115
   ctg gct tac cac ttc gtt acc acg acc gtt cct agt acc atc gtc aca     2539
   Leu Ala Tyr His Phe Val Thr Thr Thr Val Pro Ser Thr Ile Val Thr
           120                 125                 130
   gtg act tat acg gtg aca gca gac ggt cag atc aat atc gcc acc cat     2587
   Val Thr Tyr Thr Val Thr Ala Asp Gly Gln Ile Asn Ile Ala Thr His
       135                 140                 145
   tat agc ggt cag tct gat ttg cca gag cta ccc gca ttt ggt ctg cgg     2635
   Tyr Ser Gly Gln Ser Asp Leu Pro Glu Leu Pro Ala Phe Gly Leu Arg
   150                 155                 160                 165
   ttt atc atg cca act acc gcg acc ggc ttc gac tat acc ggt ttg tcc     2683
   Phe Ile Met Pro Thr Thr Ala Thr Gly Phe Asp Tyr Thr Gly Leu Ser
                   170                 175                 180
   ggt gag act tat cct gac cgg ctg gct ggc gca aca cac ggg caa ttc     2731
   Gly Glu Thr Tyr Pro Asp Arg Leu Ala Gly Ala Thr His Gly Gln Phe
               185                 190                 195
   cac gtt gac agt ctg cca gtc aca cca tac ttg gtc cca caa gaa tgc     2779
   His Val Asp Ser Leu Pro Val Thr Pro Tyr Leu Val Pro Gln Glu Cys
           200                 205                 210
   ggc atg cac atg caa act gaa caa gtg aca gta acg cga tca aca aca     2827
   Gly Met His Met Gln Thr Glu Gln Val Thr Val Thr Arg Ser Thr Thr
       215                 220                 225
   caa aat aac gct gac cac gac aac aca cca ttc agt tta aca ttt agc     2875
   Gln Asn Asn Ala Asp His Asp Asn Thr Pro Phe Ser Leu Thr Phe Ser
   230                 235                 240                 245
   caa acc gat gca cca ttc gcc ttc agc tgc ctt ccc tat acc gct gct     2923
   Gln Thr Asp Ala Pro Phe Ala Phe Ser Cys Leu Pro Tyr Thr Ala Ala
                   250                 255                 260
   gaa cta gaa aac gca aca cac atg gaa gaa tta cca tta gca cgg cga     2971
   Glu Leu Glu Asn Ala Thr His Met Glu Glu Leu Pro Leu Ala Arg Arg
               265                 270                 275
   acg gtc tta tca atc tac ggt gcc gtt cgt ggg gtc ggt ggc att gac     3019
   Thr Val Leu Ser Ile Tyr Gly Ala Val Arg Gly Val Gly Gly Ile Asp
           280                 285                 290
   agt tgg gga acg gac gta gaa gcc cca tat cat atc ctc gct aat caa     3067
   Ser Trp Gly Thr Asp Val Glu Ala Pro Tyr His Ile Leu Ala Asn Gln
       295                 300                 305
   gac att gac ttc agc ttt aac att cat ttc taa aaattaattt gatttcaaaa   3120
   Asp Ile Asp Phe Ser Phe Asn Ile His Phe
   310                 315
   gaatcgctcc ggcagttatt tgccagagcg ttcttttaat atgttttatg gctgagcttt   3180
   agtcctttga agtga                                                    3195
<210> 15
   <211> 319
   <212> PRT
   <213> Leuconostoc lactis
<400> 15
   Met Ala Tyr Thr Asn Asn Gln Leu His Val Ile Tyr Gly Asp Gly Ser
   1               5                   10                  15
   Leu Gly Leu Gln Gly Ala Asn Phe His Tyr Leu Phe Ser Tyr Glu Arg
               20                  25                  30
   Gly Gly Leu Glu Ser Leu Val Val Asn Asp Lys Glu Trp Leu Tyr Arg
           35                  40                  45
   Thr Pro Thr Pro Met Phe Trp Arg Ala Thr Thr Asp Asn Asp His Gly
       50                  55                  60
   Ser Gly Phe Ser Val Lys Ser Ala Gln Trp Tyr Ala Ala Asp Lys Phe
   65                  70                  75                  80
   Ser Thr Cys Gln Asp Ile Glu Leu Thr Val Asp Asp Gln Pro Val Thr
                   85                  90                  95
   Pro Leu Pro Ile Ala Pro Leu Asn Asn Lys Tyr Thr Asp His Glu Ile
               100                 105                 110
   Ala Thr Lys Val Ser Leu Ala Tyr His Phe Val Thr Thr Thr Val Pro
           115                 120                 125
   Ser Thr Ile Val Thr Val Thr Tyr Thr Val Thr Ala Asp Gly Gln Ile
       130                 135                 140
   Asn Ile Ala Thr His Tyr Ser Gly Gln Ser Asp Leu Pro Glu Leu Pro
   145                 150                 155                 160
   Ala Phe Gly Leu Arg Phe Ile Met Pro Thr Thr Ala Thr Gly Phe Asp
                   165                 170                 175
   Tyr Thr Gly Leu Ser Gly Glu Thr Tyr Pro Asp Arg Leu Ala Gly Ala
               180                 185                 190
   Thr His Gly Gln Phe His Val Asp Ser Leu Pro Val Thr Pro Tyr Leu
           195                 200                 205
   Val Pro Gln Glu Cys Gly Met His Met Gln Thr Glu Gln Val Thr Val
       210                 215                 220
   Thr Arg Ser Thr Thr Gln Asn Asn Ala Asp His Asp Asn Thr Pro Phe
   225                 230                 235                 240
   Ser Leu Thr Phe Ser Gln Thr Asp Ala Pro Phe Ala Phe Ser Cys Leu
                   245                 250                 255
   Pro Tyr Thr Ala Ala Glu Leu Glu Asn Ala Thr His Met Glu Glu Leu
   Pro Leu Ala Arg Arg Thr Val Leu Ser Ile Tyr Gly Ala Val Arg Gly
   275 280 285
   Val Gly Gly Ile Asp Ser Trp Gly Thr Asp Val Glu Ala Pro Tyr His
   290 295 300
   Ile Leu Ala Asn Gln Asp Ile Asp Phe Ser Phe Asn Ile His Phe
   305 310 315